# EUROPÄISCHE PATENTSCHRIFT

(11) **EP 0 836 485 B1**
(45) Veröffentlichungstag und Bekanntmachung des Hinweises auf die Patenterteilung: **24.07.2002**
(21) Anmeldenummer: 96922859.2
(22) Anmeldetag: 20.06.1996
(51) Int. Cl.: A61K 49/00

(54) **KASKADEN-POLYMER-KOMPLEXE, VERFAHREN ZU IHRER HERSTELLUNG UND DIESE ENTHALTENDE PHARMAZEUTISCHE MITTEL**
CASCADE POLYMER COMPLEXES
COMPLEXES POLYMERES EN CASCADE, LEUR PROCEDE DE PRODUCTION ET PRODUITS PHARMACEUTIQUES LES CONTENANT

(30) Priorität: 04.07.1995 DE 19525924
(43) Veröffentlichungstag der Anmeldung: 22.04.1998
(73) Patentinhaber: SCHERING AKTIENGESELLSCHAFT, 13353 Berlin (DE)
(72) Erfinder: SCHMITT-WILLICH, Heribert, D-12161 Berlin (DE); PLATZEK, Johannes, D-12621 Berlin (DE); RADÜCHEL, Bernd, D-13465 Berlin (DE); MÜHLER, Andreas, D-15366 Neuenhagen (DE); FRENZEL, Thomas, D-12247 Berlin (DE)
(86) Internationale Anmeldenummer: EP9602671
(87) Internationale Veröffentlichungsnummer: WO9702051

(56) Entgegenhaltungen:
- EP-A- 0 430 863
- WO-A-95/24225
- WO-A-95/28966
- DE-A- 4 344 460
- DE-A- 4 425 857
- US-A- 5 527 524
- MAGNETIC RESONANCE IN MEDICINE, Bd. 31, Nr. 1, 1.Januar 1994, Seiten 1-8, XP000423671 WIENER E C ET AL: "DENDRIMER-BASED METAL CHELATES: A NEW CLASS OF MAGNETIC RESONANCE IMAGING CONTRAST AGENTS"

## Beschreibung

Die Erfindung betrifft den in den Patentansprüchen gekennzeichneten Gegenstand, das heißt neue Kaskaden-Polymer-Komplexe, diese Verbindungen enthaltende Mittel, die Verwendung der Komplexe zur Herstellung eines Mittels zur Anwendung in der Diagnostik und Therapie sowie Verfahren zur Herstellung dieser Verbindungen und Mittel.

Die zur Zeit klinisch eingesetzten Kontrastmittel für die modernen bildgebenden Verfahren Kernspintomographie (MRI) und Computertomographie (CT) [Magnevist ®, Pro Hance ®, Ultravist® und Omniscan ®] verteilen sich im gesamten extrazellulären Raum des Körpers (Intravasalraum und Interstitium). Dieser Verteilungsraum umfaßt etwa 20 % des Körpervolumens.

Extrazelluläre MRI-Kontrastmittel sind klinisch zuerst erfolgreich bei der Diagnostik von zerebralen und spinalen Krankheitsprozessen eingesetzt worden, da sich hier eine ganz besondere Situation hinsichtlich des regionalen Verteilungsraumes ergibt. Im Gehirn und im Rückenmark können extrazelluläre Kontrastmittel im gesunden Gewebe aufgrund der Blut-Hirn-Schranke nicht den Intravasalraum verlassen. Bei krankhaften Prozessen mit Störung der Blut-Hirn-Schranke (z.B. maligne Tumoren, Entzündungen, demyelinisierende Erkrankungen etc.) entstehen innerhalb des Hirns dann Regionen mit erhöhter Blutgefäß-Durchlässigkeit (Permeabilität) für diese extrazellulären Kontrastmittel (Schmiedl et al., MRI of blood-brain barrier permeability in astrocytic gliomas: application of small and large molecular weight contrast media, Magn. Reson. Med. 22: 288, 1991). Durch das Ausnutzen dieser Störung der Gefäßpermeabilität kann erkranktes Gewebe mit hohem Kontrast gegenüber dem gesunden Gewebe erkannt werden.

Außerhalb des Gehirns und des Rückenmarkes gibt es allerdings eine solche Permeabilitätsbarriere für die oben genannten Kontrastmittel nicht (Canty et al., First-pass entry of nonionic contrast agent into the myocardial extravascular space. Effects on radiographic estimate of transit time and blood volume. Circulation 84: 2071, 1991). Damit ist die Anreicherung des Kontrastmittels nicht mehr abhängig von der Gefäßpermeabilität, sondern nur noch von der Größe des extrazellulären Raumes im entsprechenden Gewebe. Eine Abgrenzung der Gefäße gegenüber dem umliegenden interstitiellen Raum bei Anwendung dieser Kontrastmittel ist nicht möglich.

Besonders für die Darstellung von Gefäßen wäre ein Kontrastmittel wünschenswert, das sich ausschließlich im vasalen Raum (Gefäßraum) verteilt. Ein solches blood-pool-agent soll es ermöglichen, mit Hilfe der Kernspintomographie gut durchblutetes von schlecht durchblutetem Gewebe abzugrenzen und somit eine Ischämie zu diagnostizieren. Auch infarziertes Gewebe ließe sich aufgrund seiner Anämie vom umliegenden gesunden oder ischämischen Gewebe abgrenzen, wenn ein vasales Kontrastmittel angewandt wird. Dies ist von besonderer Bedeutung, wenn es z.B. darum geht, einen Herzinfarkt von einer Ischämie zu unterscheiden.

Bisher müssen sich die meisten der Patienten, bei denen Verdacht auf eine kardiovaskuläre Erkrankung besteht (diese Erkrankung ist die häufigste Todesursache in den westlichen Industrieländern), invasiven diagnostischen Untersuchungen unterziehen. In der Angiographie wird zur Zeit vor allem die Röntgen-Diagnostik mit Hilfe von jodhaltigen Kontrastmitteln angewandt. Diese Untersuchungen sind mit verschiedenen Nachteilen behaftet: sie sind mit dem Risiko der Strahlenbelastung verbunden, sowie mit Unannehmlichkeiten und Belastungen, die vor allem daher kommen, daß die jodhaltigen Kontrastmittel, verglichen mit NMR-Kontrastmitteln, in sehr viel höherer Konzentration angewandt werden müssen.

Es besteht daher ein Bedarf an NMR-Kontrastmitteln, die den vasalen Raum markieren können (blood-pool-agent). Diese Verbindungen sollen sich durch eine gute Verträglichkeit und durch eine hohe Wirksamkeit (hohe Steigerung der Signalintensität bei MRI) auszeichnen.

Der Ansatz, zumindest einen Teil dieser Probleme durch Verwendung von Komplexbildnem, die ari Makro- oder Biomoleküle gebunden sind, zu lösen, war bisher nur sehr begrenzt erfolgreich.

So ist beispielsweise die Anzahl paramagnetischer Zentren in den Komplexen, die in den Europäischen Patentanmeldungen Nr. 0 088 695 und Nr. 0 150 844 beschrieben sind, für eine zufriedenstellende Bildgebung nicht ausreichend.

Erhöht man die Anzahl der benötigten Metallionen durch mehrfache Einführung komplexierender Einheiten in ein makromolekulares Biomolekül, so ist das mit einer nicht tolerierbaren Beeinträchtigung der Affinität und/oder Spezifizität dieses Biomoleküls verbunden [J. Nucl. Med. 24, 1158 (1983)].

Makromoleküle können generell als Kontrastmittel für die Angiographie geeignet sein. Albumin-GdDTPA (Radiology 1987; 162: 205) z.B. zeigt jedoch 24 Stunden nach intravenöser Injektion bei der Ratte eine Anreicherung im Lebergewebe, die fast 30 % der Dosis ausmacht. Außerdem werden in 24 Stunden nur 20 % der Dosis eliminiert.

Das Makromolekül Polylysin-GdDTPA (Europäische Patentanmeldung, Publikations-Nr. 0 233 619) erwies sich ebenfalls geeignet als blood-pool-agent. Diese Verbindung besteht jedoch herstellungsbedingt aus einem Gemisch von Molekülen verschiedener Größe. Bei Ausscheidungsversuchen bei der Ratte konnte gezeigt werden, daß dieses Makromolekül unverändert durch glomeruläre Filtration über die Niere ausgeschieden wird. Synthesebedingt kann Polylysin-GdDTPA aber auch Makromoleküle enthalten, die so groß sind, daß sie bei der glomerulären F Itration die Kapillaren der Niere nicht passieren können und somit im Körper zurückbleiben.

Auch makromolekulare Kontrastmittel auf der Basis von Kohlenhydraten, z.B. Dextran, sind beschrieben worden (Europäische Patentanmeldung, Publikations-Nr. 0 326 226). Der Nachteil dieser Verbindungen liegt darin, daß diese in der Regel nur ca. 5 % des signalverstärkenden paramagnetischen Kations tragen.

Die in der Europäischen Patentanmeldung Nr. 0 430 863 sowie die in der deutschen Offenlegungsschrift DE 43 44 460 beschriebenen Polymere stellen bereits einen Schritt auf dem Wege zu blood-pool-agents dar, da sie nicht mehr die für die vorher erwähnten Polymere charakteristische Heterogenität bezüglich Größe und Molmasse aufweisen. Sie lassen jedoch ähnlich wie die von Wiener et al. in einer Veröffentlichung in "Magnetic Resonance in Medicine" vom Januar 1994 (Seite 1 bis 8) offenbarten Verbindungen immer noch Wünsche im Hinblick auf vollständige Ausscheidung, Verträglichkeit und/oder Wirksamkeit offen.

Es bestand daher die Aufgabe, neue diagnostische Mittel vor allem zur Erkennung und Lokalisierung von Gefäßkrankheiten, die die genannten Nachteile nicht besitzen, zur Verfügung zu stellen. Diese Aufgabe wird durch die vorliegende Erfindung gelöst.

Es wurde gefunden, daß sich Komplexe, die aus stickstoffhaltigen, mit komplexbildenden Liganden versehenen Kaskaden-Polymeren, mindestens 16 Ionen eines Elements der Ordnungszahlen 20-29, 39, 42, 44 oder 57-83 sowie gegebenenfalls Kationen anorganischer und/oder organischer Basen, Aminosäuren oder Aminosäureamide bestehen und gegebenenfalls acylierte Aminogruppen enthalten, überraschenderweise hervorragend zur Herstellung von NMR- und Röntgen-Diagnostika ohne die genannten Nachteile aufzuweisen, eignen.

Die erfindungsgemäßen komplexbildenden Kaskaden-Polymere lassen sich durch die allgemeine Formel I beschreiben

A-{X-[Y-(Z-〈W-K_{w}〉_{z})_{y}]ₓ}ₐ (I),

worin
- A: für einen stickstoffhaltigen Kaskadenkern der Basismultiplizität a,
- X und Y: unabhängig voneinander für eine direkte Bindung oder eine Kaskadenreproduktionseinheit der Reproduktionsmultiplizität x bzw. y,
- Z and W: unabhängig voneinander für eine Kaskadenreproduktionseinheit der Reproduktionsmultiplizität z bzw. w,
- K: für den Rest eines Komplexbildners der allgemeinen Formel I A oder IB,
- a: für die Ziffern 2 bis 12,
- x, y, z und w: unabhängig voneinander für die Ziffern 1 bis 4 stehen,
mit der Maßgabe, daß mindestens zwei Reproduktionseinheiten unterschiedlich sind und daß für das Produkt der Multiplizitäten gilt
16 ≤ a · x · y · z · w ≤ 64.

Als Kaskadenkern A sind geeignet:
Stickstoffatom, 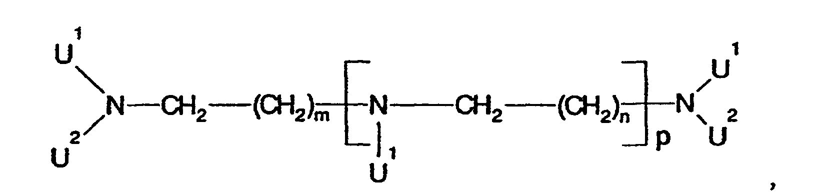 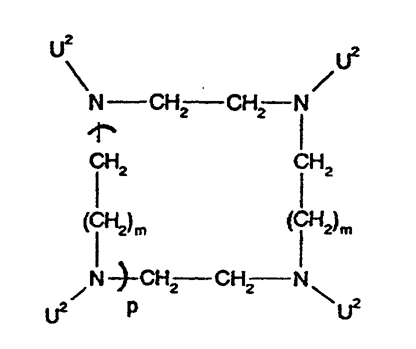 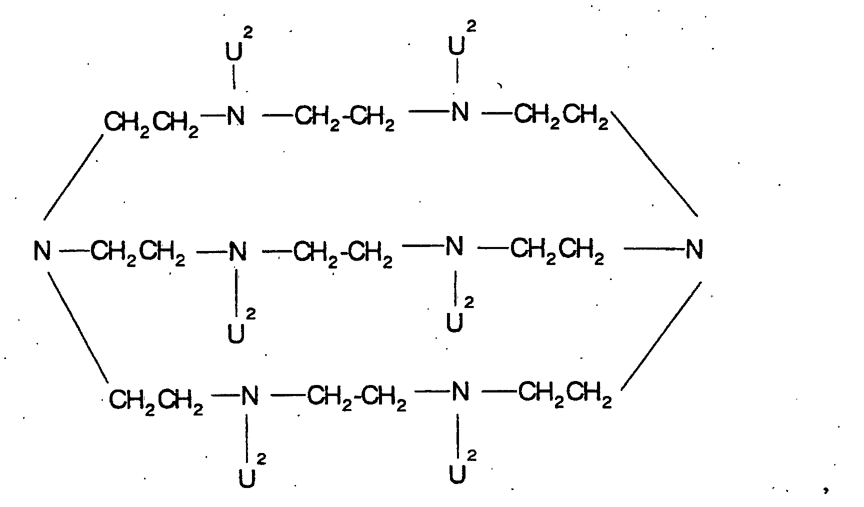 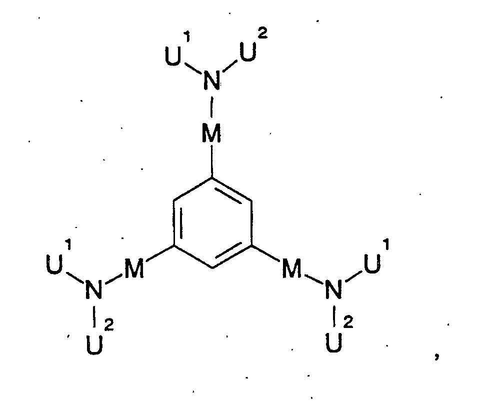 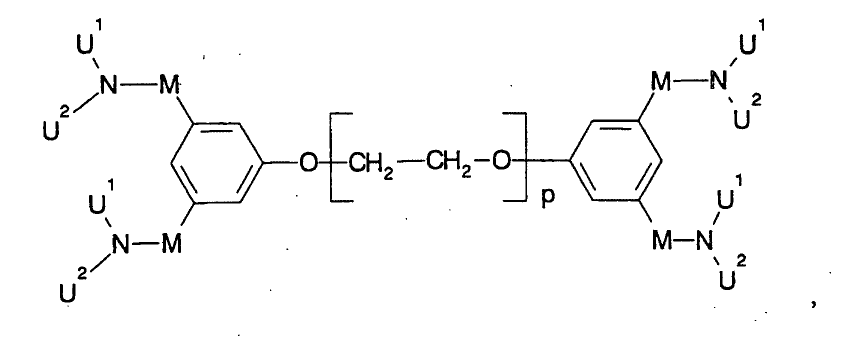 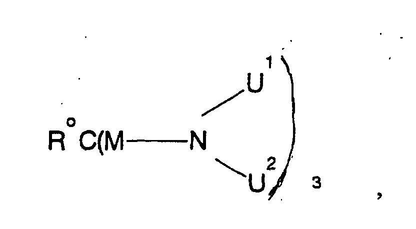 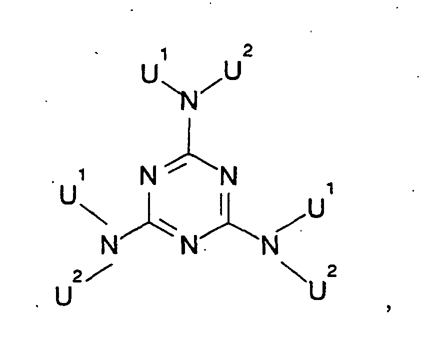 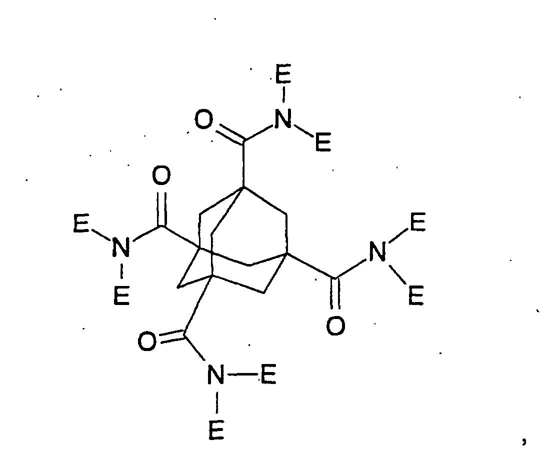 worin
- m: und n für die Ziffern 1 bis 10,
- p: für die Ziffern 0 bis 10,
- U¹: für Q¹ oder E,
- U²: für Q² oder E mit

- E: in der Bedeutung der Gruppe 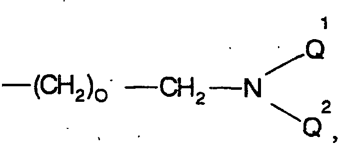 wobei
o für die Ziffern 1 bis 6,
Q¹ für ein Wasserstoffatom oder Q² und
Q² für eine direkte Bindung
- M: für eine C₁-C₁₀-Alkylenkette, die gegebenenfalls durch 1 bis 3 Sauerstoffatome unterbrochen ist und/oder gegebenenfalls mit 1 bis 2 Oxogruppen substituiert ist,
- R^{o}: für einen verzweigten oder unverzweigten C₁-C₁₀-Alkylrest, eine Nitro-, Amino-, Carbonsäuregruppe oder für 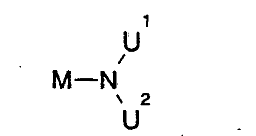
stehen,
wobei die Anzahl Q² der Basismultiplizität a entspricht.

Den einfachsten Fall eines Kaskadenkerns stellt das Stickstoffatom dar, dessen drei Bindungen (Basismultiplizität a = 3) in einer ersten "inneren Schicht" (Generation 1) von drei Reproduktionseinheiten X bzw. Y (wenn X für eine direkte Bindung steht) bzw. Z (wenn X und Y jeweils für eine direkte Bindung stehen) besetzt sind; anders formuliert: die drei Wasserstoffatome des zugrundeliegenden Kaskadenstarters Ammoniak A(H)ₐ = NH₃ sind durch drei Reproduktionseinheiten X bzw. Y bzw. Z substituiert worden. Die im Kaskadenkern A enthaltene Anzahl Q² gibt dabei die Basismulitplizität a wieder.

Die Reproduktionseinheiten X, Y, Z und W enthalten -NQ¹Q²-Gruppen, worin Q¹ ein Wasserstoffatom oder Q² und Q² eine direkte Bindung bedeuten. Die in der jeweiligen Reproduktionseinheit (z.B. X) enthaltene Anzahl Q² entspricht der Reproduktionsmultiplizität dieser Einheit (z.B. x im Falle von X). Das Produkt aller Multiplizitäten a·x·y·z·w gibt die Anzahl der im Kaskadenpolymeren gebundenen Komplexbildner-Reste K an. Die erfindungsgemäßen Polymere enthalten mindestens 16 und höchstens 64 Reste K im Molekül, die jeweils ein bis maximal drei (im Falle von zweiwertigen Ionen), vorzugsweise ein Ion, eines Elements der oben genannten Ordnungszahlen binden können.

Die letzte Generation, d.h. die an die Komplexbildner-Reste K gebundene Reproduktionseinheit W ist über NH-Gruppen (-NQ¹Q² mit Q1 in der Bedeutung eines Wasserstoffatoms und Q² = direkte Bindung) an K gebunden, während die vorangehenden Reproduktionseinheiten sowohl über NHQ²-Gruppen (z.B. durch Acylierungsreaktionen) als auch über NQ²Q²-Gruppen (z.B. durch Alkylierungsreaktionen) miteinander verknüpft sein können.

Die erfindungsgemäßen Kaskaden-Polymer-Komplexe weisen maximal 10 Generationen auf (d.h. es können auch mehr als jeweils nur eine der Reproduktionseinheiten X, Y und Z im Molekül vorhanden sein), vorzugsweise jedoch 2 bis 4 Generationen, wobei mindestens zwei der Reproduktionseinheiten im Molekül unterschiedlich sind.

Als bevorzugte Kaskadenkerne A seien diejenigen angeführt, die unter die oben genannten allgemeinen Formeln fallen, wenn
- m: für die Ziffern 1-3, besonders bevorzugt für die Ziffer 1,
- n: für die Ziffern 1-3, besonders bevorzugt für die Ziffer 1,
- p: für die Ziffern 0-3, besonders bevorzugt für die Ziffer 1,
- o: für die Ziffer 1,
- M: für eine -CH₂-, -CO- oder -CH₂CO-Gruppe und
- R^{o}: für eine -CH₂NU¹U²-, CH₃- oder NO₂-Gruppe
steht.

Als weitere bevorzugte Kaskadenstarter A(H)ₐ seien z.B. aufgeführt:
(In der Klammer wird die Basismultiplizität a angegeben für den Fall der zum Aufbau der nächsten Generation dienenden nachfolgenden Mono- bzw. Disubstitution)

| | |
|---|---|
| Tris(aminoethyl)amin | (a = 6 bzw. 3); |
| Tris(aminopropyl)amin | (a = 6 bzw. 3); |
| Diethylentriamin | (a = 5 bzw. 3); |
| Triethylentetramin | (a = 6 bzw. 4); |
| Tetraethylenpentamin | (a = 7 bzw. 5); |
| 1,3,5-Tris(aminomethyl)benzol | (a = 6 bzw. 3); |
| Trimesinsäuretriamid | (a = 6 bzw. 3); |
| 1,4,7-Triazacyclononan | (a = 3); |
| 1,4,7,10-Tetraazacyclododecan | (a = 4); |
| 1,4,7,10,13-Pentaazacyclopentadecan | (a = 5); |
| 1,4,8,11-Tetraazacyclotetradecan | (a = 4); |
| 1,4,7,10,13,16-Hexaazacyclooctadecan | (a = 6); |
| 1,4,7,10,13,16,19,22,25,28-Decaazacyclotriacontan | (a = 10); |
| Tetrakis(aminomethyl)methan | (a = 8 bzw. 4); |
| 1,1,1-Tris(aminomethyl)ethan | (a = 6 bzw. 3); |
| Tris(aminopropyl)-nitromethan | (a = 6 bzw. 3); |
| 2,4,6-Triamino-1,3,5-triazin | (a = 6 bzw. 3); |
| 1,3,5,7-Adamantantetracarbonsäureamid | (a = 8 bzw. 4); |
| 3,3',5,5'-Diphenylether-tetracarbonsäureamid | (a = 8 bzw. 4); |
| 1,2-Bis[Phenoxyethan]-3',3",5',5"-tetracarbonsäureamid | (a = 8 bzw. 4); |
| 1,4,7,10,13,16,21,24-Octaazabicyclo[8.8.8.]hexacosan | (a = 6). |

Es sei darauf hingewiesen, daß die Definition als Kaskadenkern A und damit die Trennung von Kaskadenkern und erster Reproduktionseinheit rein formal und damit unabhängig von dem tatsächlichen synthetischen Aufbau der gewünschten Kaskaden-Polymer-Komplexe gewählt werden kann. So kann man z.B. das in Beispiel 4 verwendete Tris(aminoethyl)-amin sowohl selbst als Kaskadenkern A (vergleiche die erste für A angegebene allgemeine Formel mit m = n = p = 1, U¹ = E mit o in der Bedeutung der Ziffer 1 und U¹ = U² = Q²) aber auch als Stickstoffatom (= Kaskadenkern A), das als erste Generation drei Reproduktionseinheiten 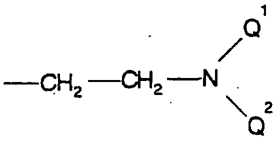 (vergleiche die Definition von E) aufweist, ansehen.

Die Kaskadenreproduktionseinheiten X, Y, Z und W werden unabhängig voneinander durch
E, 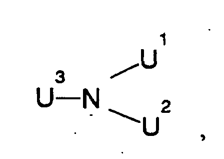 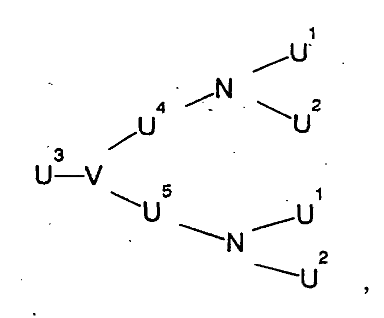 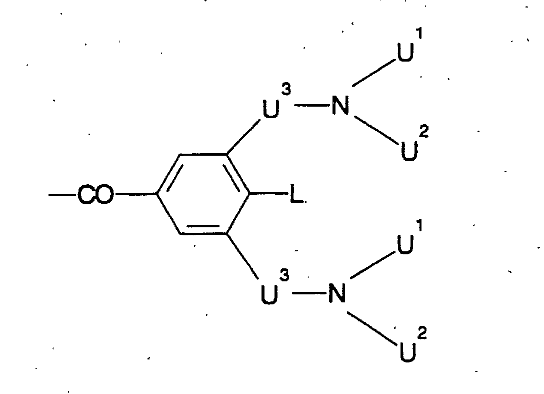 bestimmt,
worin
- U¹: für Q¹ oder E,
- U²: für Q² oder E mit
E in der Bedeutung der Gruppe 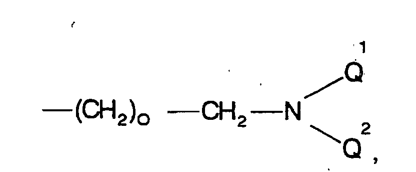 wobei
o für die Ziffern 1 bis 6,
Q¹ für ein Wasserstoffatom oder Q²,
Q² für eine direkte Bindung,
- U³: für eine C₁-C₂₀-Alkylenkette, die gegebenenfalls durch 1 bis 10 Sauerstoffatome und/oder 1 bis 2-N(CO)_{q}-R²-, 1 bis 2 Phenylen- und/oder 1 bis 2 Phenylenoxyreste unterbrochen ist und/oder gegebenenfalls durch 1 bis 2 Oxo-, Thioxo-, Carboxy-, C₁-C₅-Alkylcarboxy-, C₁-C₅-Alkoxy-, Hydroxy-, C₁-C₅-alkylgruppen substituiert ist, wobei
q für die Ziffern 0 oder 1 und
R² für ein Wasserstoffatom, einen Methyl- oder einen Ethylrest, der gegebenenfalls mit 1-2 Hydroxy- oder 1 Carboxygruppe(n) substituiert ist,
L für ein Wasserstoffatom oder die Gruppe 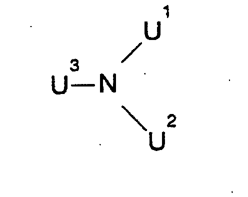
- V: für die Methingruppe 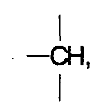 wenn gleichzeitig U⁴ eine direkte Bindung oder die Gruppe M bedeutet und U⁵ eine der Bedeutungen von U³ besitzt oder
- V: für die Gruppe 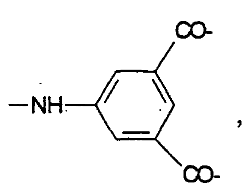 wenn gleichzeitig U⁴ und U⁵ identisch sind und die direkte Bindung oder die Gruppe M bedeuten,
stehen.

Bevorzugte Kaskadenreproduktionseinheiten X, Y, Z und W sind diejenigen, bei denen in den oben genannten allgemeinen Formeln der Rest U³ für
-CO-, -COCH₂OCH₂CO-, -COCH₂-, -CH₂CH₂-, -CONHC₆H₄-, -COCH₂CH₂CO-, -COCH₂-CH₂CH₂CO-, -COCH₂CH₂CH₂CH₂CO-,
der Rest U⁴ für eine direkte Bindung, für -CH₂CO-,
der Rest U⁵ für eine direkte Bindung, für -(CH₂)₄-, -CH₂CO-, -CH(COOH)-, CH₂OCH₂CH₂-, -CH₂C₆H₄-, CH₂-C₆H₄OCH₂CH₂-,
der Rest E für eine Gruppe 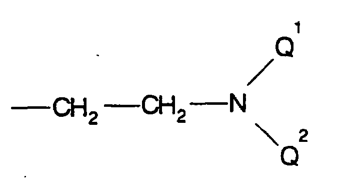 steht.

Als beispielhaft genannte Kaskadenreproduktionseinheiten X, Y, Z und W seien angeführt:
-CH₂CH₂NH-; -CH₂CH₂N< ; -COCH(NH-)(CH₂)₄NH-; -COCH(N< )(CH₂)₄N< ; -COCH₂OCH₂CON(CH₂CH₂NH-)₂; -COCH₂OCH₂CON(CH₂CH₂N< )₂; -COCH₂N(CH₂CH₂NH-)₂; -COCH₂N(CH₂CH₂N< )₂; -COCH₂NH-; -COCH₂N< ; -COCH₂CH₂CON(CH₂CH₂NH-)₂; -COCH₂CH₂CON(CH₂CH₂N< )₂ ; -COCH₂OCH₂CONH-C₆H₄-CH[CH₂CON(CH₂CH₂NH-)₂]₂; -COCH₂OCH₂CONH-C₆H₄-CH[CH₂CON(CH₂CH₂N< )₂]₂ ; -COCH₂CH₂CO-NH-C₆H₄-CH[CH₂CON(CH₂CH₂NH-)₂]₂ ; -COCH₂CH₂CO-NH-C₆H₄-CH[CH₂CON(CH₂CH₂N<)₂]₂ ; -CONH-C₆H₄-CH[CH₂CON(CH₂CH₂NH-)₂]₂ ; -CONH-C₆H₄-CH[CH₂CON(CH₂CH₂N<)₂]₂ ; -COCH(NH-)CH(COOH)NH-; -COCH(N< )CH(COOH)N< ;

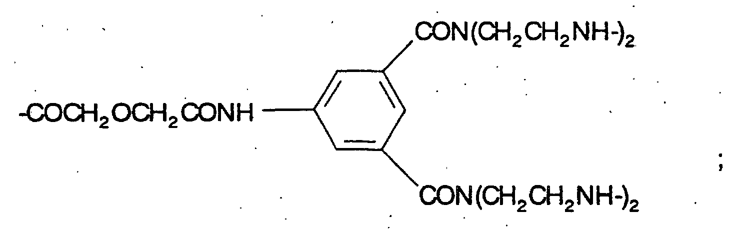

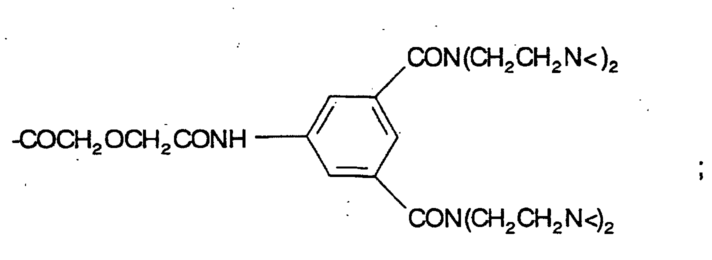

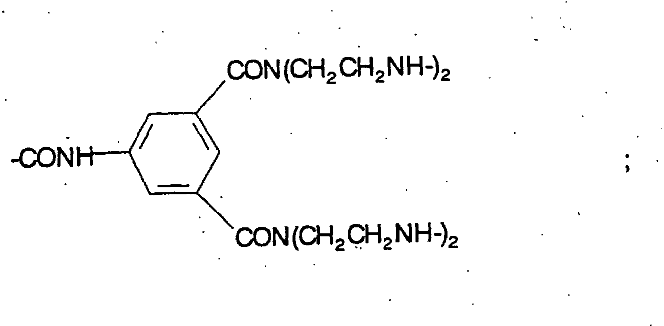

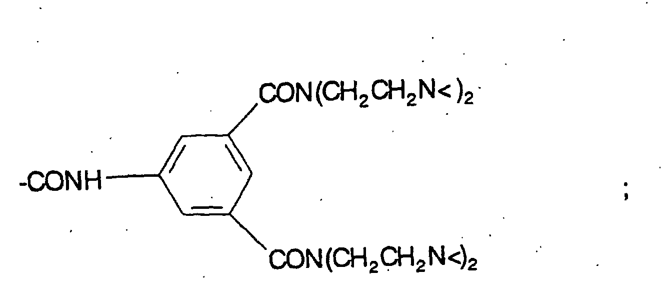

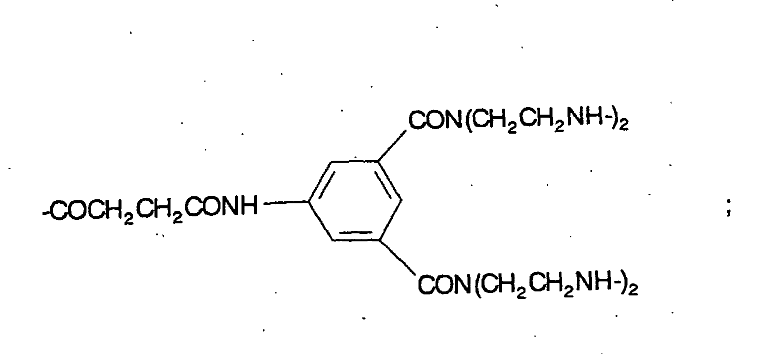

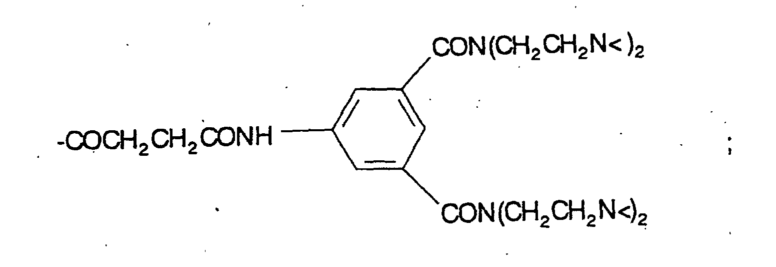

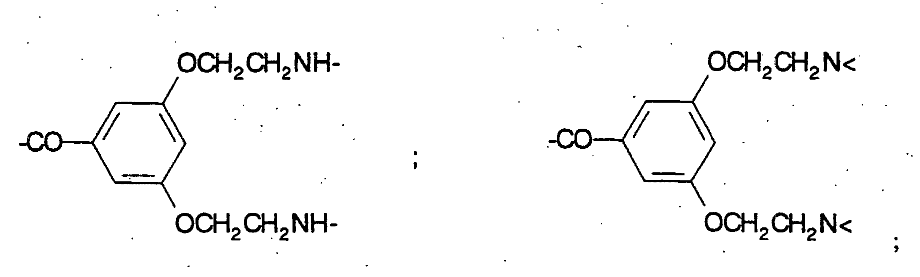

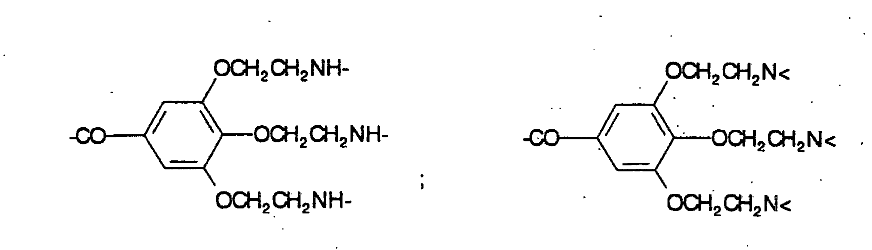

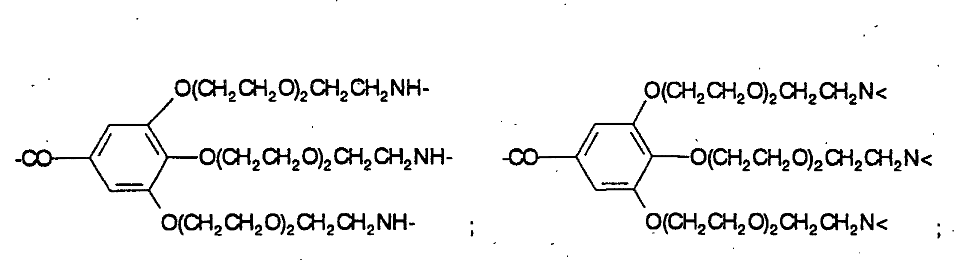

Die Komplexbildner-Reste K werden durch die allgemeinen Formeln IA und IB beschrieben: 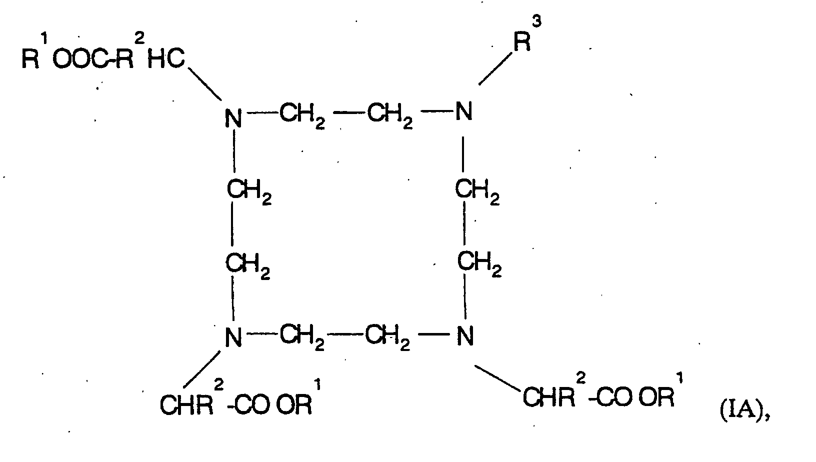 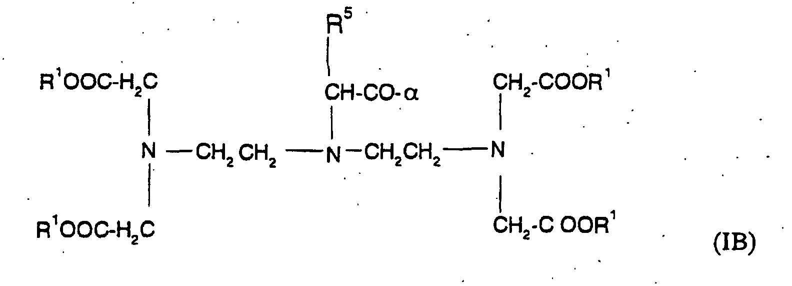 steht, worin
- R¹: unabhängig voneinander für ein Wasserstoffatom oder ein Metallionenäquivalent der Ordnungszahlen 20-29, 39, 42-44 oder 57-83,
- R²: für ein Wasserstoffatom, einen Methyl- oder einen Ethylrest, der gegebenenfalls mit 1-2 Hydroxy- oder 1 Carboxygruppe(n) substituiert ist,
- R³: für eine 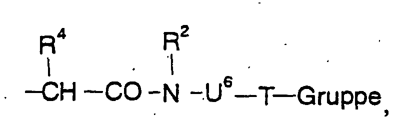
- R⁴: für eine geradkettige, verzweigte, gesättigte oder ungesättigte C₁-C₃₀-Alkylkette, die gegebenenfalls durch 1-10 Sauerstoffatome, 1 Phenylen-, 1 Phenylenoxygruppen unterbrochen und/oder gegebenenfalls durch 1-5 Hydroxy-, 1-3 Carboxy-, 1 Phenylgruppe(n) substituiert ist,
- R⁵: für ein Wasserstoffatom oder für R⁴,
- U⁶: für eine gegebenenfalls 1-5 Imino-, 1-3 Phenylen-, 1-3 Phenylenoxy-, 1-3 Phenylenimino-, 1-5 Amid-,1-2 Hydrazid-, 1-5 Carbonyl-, 1-5 Ethylenoxy-, 1 Harnstoff-, 1-Thioharnstoff-, 1-2 Carboxyalkyl imino-, 1-2 Estergruppen, 1-10 Sauerstoff-, 1-5 Schwefel- und/oder 1-5 Stickstoff-Atom(e) enthaltende und/oder gegebenenfalls durch 1-5 Hydroxy-, 1-2 Mercapto-, 1-5 Oxo-, 1-5 Thioxo-, 1-3 Carboxy-, 1-Carboxyalkyl-, 1-5 Ester- und/oder 1-3 Aminogruppe(n) substituierte geradkettige, verzweigte, gesättigte oder ungesättigte C₁-C₂₀-Alkylengruppe, wobei die gegebenenfalls enthaltenden Phenylengruppen durch 1-2 Carboxy-, 1-2 Sulfon- oder 1-2 Hydroxygruppen substituiert sein können,
- T: für eine -CO-α, -NHCO-α- oder -NHCS-α-Gruppe und
- α: für die Bindungsstelle an die terminalen Stickstoffatome der letzten Generation, der Reproduktionseinheit W
stehen.

Als bevorzugte Komplexbildner-Reste K seien diejenigen genannt, bei denen in der oben angegebenen Formel IA die für U⁶ stehende C₁-C₂₀-, bevorzugt C₁-C₁₂-, Alkylenkette die Gruppen
-CH₂-, -CH₂NHCO-, -NHCOCH₂O-, -NHCOCH₂OC₆H₄-, -N(CH₂CO₂H)-, -NHCOCH₂C₆H₄-, -NHCSNHC₆H₄-, -CH₂OC₆H₄-, -CH₂CH₂O-, enthält und/oder durch die Gruppen -COOH, -CH₂COOH substituiert ist.

Als Beispiele für U⁶ seien folgende Gruppen angeführt:
-CH₂-, -CH₂CH₂-, -CH₂CH₂CH₂-, -C₆H₄-, -C₆H₁₀-, -CH₂C₆H₅-, -CH₂NHCOCH₂CH(CH₂CO₂H)-C₆H₄-, -CH₂NHCOCH₂OCH₂-, -CH₂NHCOCH₂C₆H₄-, 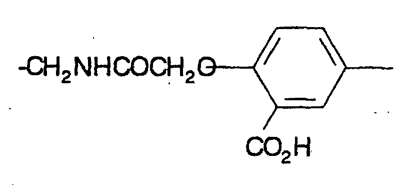 -CH₂NHCSNH-C₆H₄-CH(CH₂COOH)CH₂-, -CH₂OC₆H₄-N(CH₂COOH)CH₂-, -CH₂NHCOCH₂O(CH₂CH₂O)₄-C₆H₄-, -CH₂O-C₆H₄-, -CH₂CH₂-O-CH₂CH₂-, -CH₂CH₂-O-CH₂CH₂-O-CH₂CH₂-,

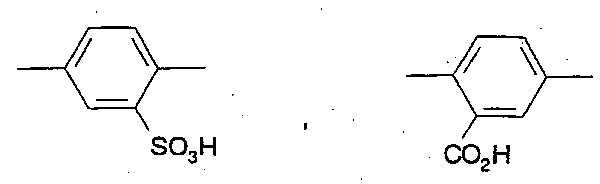

Als Beispiele für R⁴ seien folgende Gruppen angegeben:
-CH₃, -C₆H₅, -CH₂-COOH, -CH₂-C₆H₅, -CH₂-O-(CH₂CH₂-O-)₆CH₃, -CH₂-OH

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21-29, 42, 44 und 58-70. Geeignete Ionen sind beispielsweise das Chrom(III)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)-, Mangan(II)- und Eisen(III)-ion.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21-29, 39, 42, 44, 57-83 enthalten, geeignet sind; dies sind beispielsweise das Lanthan(III)-ion und die oben genannten Ionen der Lanthanidenreihe.

Die erfindungsgemäßen Kaskaden-Polymer-Komplexe enthalten mindestens 16 Ionen eines Elements der oben genannten Ordnungszahl.

Die restlichen aciden Wasserstoffatome, das heißt diejenigen, die nicht durch das Zentralion substituiert worden sind, können gegebenenfalls ganz oder teilweise durch Kationen von anorganischen und/oder organischen Basen, Aminosäuren oder Aminosäureamiden ersetzt sein.

Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Die erfindungsgemäßen Verbindungen, die ein Molekulargewicht von 10.000-80.000 D, vorzugsweise 15.000-40.000 D, besitzen, weisen die eingangs geschilderten gewünschten Eigenschaften auf. Sie enthalten die für ihre Verwendung benötigte große Anzahl von Metallionen im Komplex stabil gebunden.

Sie reichern sich in Gebieten mit erhöhter Gefäßpermeabilität, wie z.B. in Tumoren, an, erlauben Aussagen über die Perfusion von Geweben, geben die Möglichkeit, das Blutvolumen in Geweben zu bestimmen, die Relaxationszeiten bzw. Densitäten des Blutes selektiv zu verkürzen, und die Permeabilität der Blutgefäße bildlich darzustellen. Solche physiologischen Informationen sind nicht durch den Einsatz von extrazellulären Kontrastmitteln, wie z.B. Gd-DTPA [Magnevist®], zu erhalten. Aus diesen Gesichtspunkten ergeben sich auch die Einsatzgebiete bei den modernen bildgebenden Verfahren Kernspintomographie und Computertomographie: spezifischere Diagnose von malignen Tumoren, frühe Therapiekontrolle bei zytostatischer, antiphlogistischer oder vasodilatativer Therapie, frühe Erkennung von minderperfundierten Gebieten (z.B. im Myokard), Angio-graphie bei Gefäßerkrankungen, und Erkennung und Diagnose von (sterilen oder infektiösen) Entzündungen.

Die erfindungsgemäßen Kaskaden-Polymer-Komplexe eignen sich auch hervorragend für die (interstitielle und i.v.) Lymphographie.

Als weitere Vorteile gegenüber extrazellulären Kontrastmitteln, wie z.B. Gd-DTPA [Magnevist®], muß die höhere Effektivität als Kontrastmittel für die Kernspintomographie (höhere Relaxivität) hervorgehoben werden, was zu einer deutlichen Reduktion der diagnostisch notwendigen Dosis führt. Gleichzeitig können die erfindungsgemäßen Kontrastmittel als Lösungen isoosmolar zum Blut formuliert werden und verringern dadurch die osmotische Belastung des Körpers, was sich in einer verringerten Toxizität der Substanz (höhere toxische Schwelle) niederschlägt. Geringere Dosen und höhere toxische Schwelle führen zu einer signifikanten Erhöhung der Sicherheit von Kontrastmittelanwendungen bei modernen bildgebenden Verfahren.

Im Vergleich zu den makromolekularen Kontrastmitteln auf der Basis von Kohlenhydraten, z.B. Dextran (Europäische Patentanmeldung, Publikations-Nr. 0 326 226), die - wie erwähnt - in der Regel nur ca. 5 % des signalverstärkenden paramagnetischen Kations tragen, weisen die erfindungsgemäßen Polymer-Komplexe einen Gehalt von in der Regel ca. 20 % des paramagnetischen Kations auf. Somit bewirken die erfindungsgemäßen Makromoleküle pro Molekül eine sehr viel höhere Signalverstärkung, was gleichzeitig dazu führt, daß die zur Kernspintomographie notwendige Dosis gegenüber der makromolekularer Kontrastmittel auf Kohlenhydratbasis erheblich kleiner ist.

Mit den erfindungsgemäßen Polymer-Komplexen ist es gelungen, Makromoleküle so zu konstruieren und herzustellen, daß diese ein einheitlich definiertes Molekulargewicht haben. Es ist somit überraschenderweise möglich, die Größe der Makromoleküle so zu steuern, daß diese groß genug sind, um den vasalen Raum nur langsam verlassen zu können, aber gleichzeitig klein genug, die Kapillaren der Niere, welche 300-800 Å groß sind, noch passieren zu können.
Im Vergleich zu den anderen erwähnten Polymer-Verbindungen des Stands der Technik zeichnen sich die erfindungsgemäßen Kaskaden-Polymer-Komplexe durch verbessertes Ausscheidungsverhalten, höhere Wirksamkeit, größere Stabilität und/oder bessere Verträglichkeit aus.

Ein weiterer Vorteil der vorliegenden Erfindung liegt darin, daß nunmehr Komplexe mit hydrophilen oder lipophilen, makrocyclischen oder offenkettigen, niedermolekularen oder hochmolekularen Liganden zugänglich geworden sind. Dadurch ist die Möglichkeit gegeben, Verträglichkeit und Pharmakokinetik dieser Polymer-Komplexe durch chemische Substitution zu steuern.

Die Herstellung der erfindungsgemäßen Kaskaden-Polymer-Komplexe erfolgt dadurch, daß man Verbindungen der allgemeinen Formel I'

A-{X-[Y-(Z-〈W-β_{w}〉_{z})_{y}]ₓ}ₐ (I'),

worin
- A: für einen stickstoffhaltigen Kaskadenkern der Basismultiplizität a,
- X und Y: unabhängig voneinander für eine direkte Bindung oder eine Kaskadenreproduktionseinheit der Reproduktionsmultiplizität x bzw. y,
- Z und W: unabhängig voneinander für eine Kaskadenreproduktionseinheit der Reproduktionsmultiplizität z bzw. w,
- a: für die Ziffern 2 bis 12,
- x, y, z und w: unabhängig voneinander für die Ziffern 1 bis 4 und
- β: für die Bindungsstelle der terminalen NH-Gruppen der letzten Generation, der Reproduktionseinheit W stehen
mit der Maßgabe, daß mindestens zwei Reproduktionseinheiten unterschiedlich sind und daß für das Produkt der Multiplizitäten gilt
16 ≤ a · x · y · z · w ≤ 64,
mit einem Komplex oder Komplexbildner K' der allgemeinen Formel I'A oder I'B 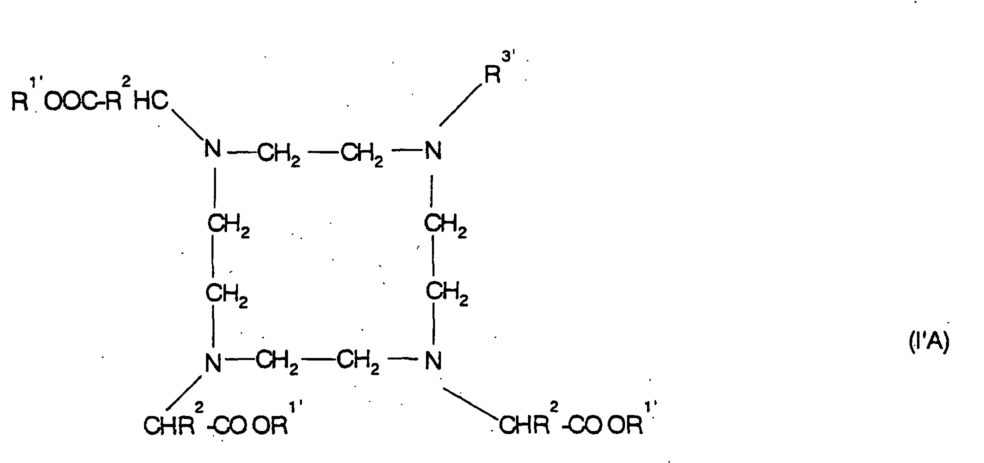 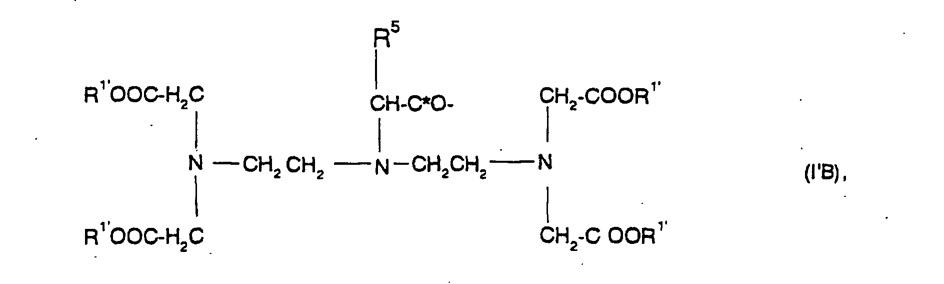 wobei
- R1': unabhängig voneinander für ein Wasserstoffatom, ein Metallionenäquivalent der Ordnungszahlen 20-29, 39, 42-44 oder 57-83 oder eine Säureschutzgruppe,
- R²: für ein Wasserstoffatom, einen Methyl- oder einen Ethylrest, der gegebenenfalls mit 1-2 Hydroxy- oder 1 Carboxygruppe(n) substituiert ist,
- R^{3'}: für eine 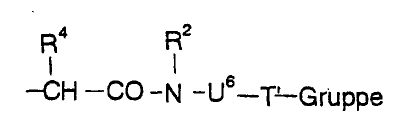
- R⁴: für eine geradkettige, verzweigte, gesättigte oder ungesättigte C₁-C₃₀-Alkylkette, die gegebenenfalls durch 1-10 Sauerstoffatome, 1 Phenylen-, 1 Phenylenoxygruppen unterbrochen und/oder gegebenenfalls durch 1-5 Hydroxy-, 1-3 Carboxy-, 1 Phenylgruppe(n) substituiert ist,
- R⁵: für ein Wasserstoffatom oder für R⁴,
- U⁶: für eine gegebenenfalls 1-5 Imino-, 1-3 Phenylen-, 1-3 Phenylenoxy-, 1-3 Phenylenimino-, 1-5 Amid-, 1-2 Hydrazid-, 1-5 Carbonyl-, 1-5 Ethylenoxy-, 1 Harnstoff-, 1-Thioharnstoff-, 1-2 Carboxyalkylimino-, 1-2 Estergruppen, 1-10 Sauerstoff-, 1-5 Schwefel- und/oder 1-5 Stickstoff-Atom(e) enthaltende und/oder gegebenenfalls durch 1-5 Hydroxy-, 1-2 Mercapto-, 1-5 Oxo-, 1-5 Thioxo-, 1-3 Carboxy-, 1-5 Carboxyalkyl-, 1-5 Ester- und/oder 1-3 Aminogruppe(n) substituierte geradkettige, verzweigte, gesättigte oder ungesättigte C₁-C₂₀-Alkylengruppe, wobei die gegebenenfalls enthaltenden Phenylengruppen durch 1-2 Carboxy-, 1-2 Sulfon- oder 1-2 Hydroxygruppen substituiert sein können,
- T': für eine -C*O-, -COOH-, -N=C=O- oder -N=C=S-Gruppe und
- C*O: für eine aktivierte Carboxylgruppe
stehen
mit der Maßgabe, daß - sofern K' für einen Komplex steht - mindestens zwei (bei zweiwertigen Metallen) bzw. drei (bei dreiwertigen Metallen) der Substituenten R¹ für ein Metallionenäquivalent der oben genannten Elemente stehen und daß gewünschtenfalls weitere Carboxylgruppen in Form ihrer Salze mit anorganischen und/oder organischen Basen, Aminosäuren oder Aminosäureamiden vorliegen,
umsetzt, gegebenenfalls vorhandene Schutzgruppen abspaltet, die so erhaltenen Kaskaden-Polymere - sofern K' für einen Komplexbildner steht - in an sich bekannter Weise mit mindestens einem Metalloxid oder Metallsalz eines Elements der Ordnungszahlen 20-29, 39, 42, 44 oder 57-83 umsetzt und gegebenenfalls anschließend in den so erhaltenen Kaskaden-Polymer-Komplexen noch vorhandene acide Wasserstoffatome ganz oder teilweise durch Kationen von anorganischen und/oder organischen Basen, Aminosäuren oder Aminosäureamiden substituiert und gegebenenfalls noch vorhandene freie terminale Aminogruppen gewünschtenfalls-vor oder nach der Metallkomplexierung-acyliert.

Einen weiteren Aspekt der vorliegenden Erfindung stellen die neuen Verbindungen der allgemeinen Formel I'A dar 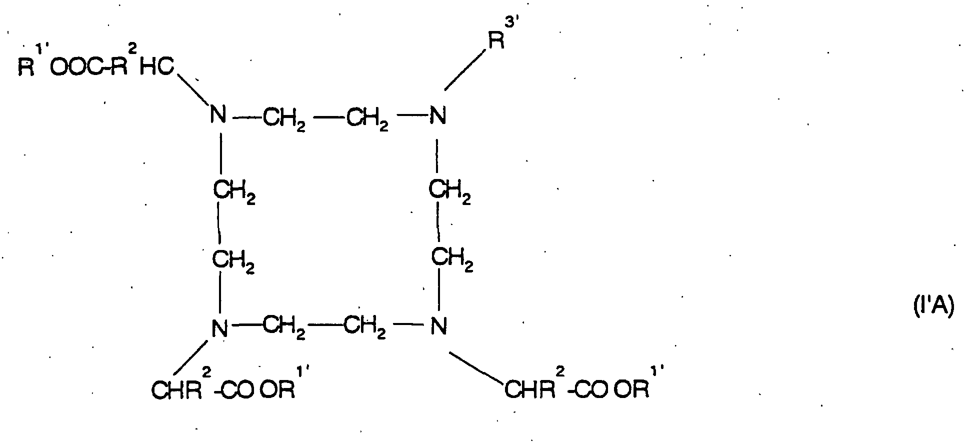 wobei
- R1': unabhängig voneinander für ein Wasserstoffatom, ein Metallionenäquivalent der Ordnungszahlen 20-29, 39, 42-44 oder 57-83 oder eine Säureschutzgruppe,
- R²: für ein Wasserstoffatom, einen Methyl- oder einen Ethylrest, der gegebenenfalls mit 1-2 Hydroxy- oder 1 Carboxygruppe(n) substituiert ist,
- R^{3'}: für eine 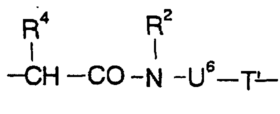 Gruppe
- R⁴: für eine geradkettige, verzweigte, gesättigte oder ungesättigte C₁-C₃₀-Alkylkette, die gegebenenfalls durch 1 10 Sauerstoffatome, 1 Phenylen-, 1 Phenylenoxygruppen unterbrochen und/oder gegebenenfalls durch 1-5 Hoydroxy-, 1-3 Carboxy-, 1 Phenylgruppe(n) substituiert ist,
- U⁶: für eine gegebenenfalls 1-5 Imino-, 1-3 Phenylen-, 1-3 Phenylenoxy-, 1-3 Phenylenimino-, 1-5 Amid-, 1-2 Hydrazid-, 1-5 Carbonyl-, 1-5 Ethylenoxy-, 1 Harnstoff-, 1-Thioharnstoff-, 1-2 Carboxyalkylimino-, 1-2 Estergruppen, 1-10 Sauerstoff-, 1-5 Schwefel- und/oder 1-5 Stickstoff-Atom(e) enthaltende und/oder gegebenenfalls durch 1-5 Hydroxy-, 1-2 Mercapto-, 1-5 Oxo-, 1-5 Thioxo-, 1-3 Carboxy-, 1-5 Carboxyalkyl-, 1-5 Ester- und/oder 1-3 Aminogruppe(n) substituierte geradkettige, verzweigte, gesättigte oder ungesättigte C₁-C₂₀-Alkylengruppe, wobei die gegebenenfalls enthaltenden Phenylengruppen durch 1-2 Carboxy-, 1-2 Sulfon- oder 1-2 Hydroxygruppen substituiert sein können,
- T': für eine -C*O-, -COOH-, -N=C=O- oder -N=C=S-Gruppe und
- C*O: für eine aktivierte Carboxylgruppe
stehen.

Sie dienen als wichtige Zwischenprodukte für die Herstellung der Kaskaden-Polymer-Komplexe der allgemeinen Formel I.

Als Beispiel für eine aktivierte Carbonylgruppe C*O in den Komplexen bzw. Komplexbildnern K' seien Anhydrid, p-Nitrophenylester, N-Hydroxysuccinimidester, Pentafluorphenylester und Säurechlorid genannt.

Die zur Einführung der Komplexbildner-Einheiten vorgenommene Addition oder Acylierung wird mit Substraten durchgeführt, die den gewünschten Substituenten K (eventuell gebunden an eine Fluchtgruppe) enthalten, oder aus denen der gewünschte Substituent durch die Reaktion generiert wird.

Als Beispiele für Additionsreaktionen seien die Umsetzung von Isocyanaten und Isothiocyanaten genannt, wobei die Umsetzung von Isocyanaten bevorzugt in aprotischen Solventien wie z.B. THF, Dioxan, DMF, DMSO, Methylenchlorid bei Temperaturen zwischen 0 und 100 °C, bevorzugt zwischen 0 und 50 °C, gegebenenfalls unter Zusatz einer organischen Base wie Triethylamin, Pyridin, Lutidin, N-Ethyldiisopropylamin, N-Methylmorpholin, durchgeführt wird. Die Umsetzung mit Isothiocyanaten wird in der Regel in Lösungsmitteln wie z.B. Wasser oder niederen Alkoholen wie z.B. Methanol, Ethanol, Isopropanol oder deren Mischungen, DMF oder Mischungen aus DMF und Wasser bei Temperaturen zwischen 0 und 100 °C, bevorzugt zwischen 0 und 50 °C, gegebenenfalls unter Zusatz einer organischen oder anorganischen Base wie z.B. Triethylamin, Pyridin, Lutidin, N-Ethyldiisopropylamin, N-Methylmorpholin oder Erdalkali-, Alkalihydroxiden wie z.B. Lithium-, Natrium-, Kalium-, Calciumhydroxid oder deren Carbonate wie z.B. Magnesiumcarbonat, durchgeführt.

Als Beispiele für Acylierungsreaktionen seien die Umsetzung von freien Carbonsäuren nach den dem Fachmann bekannten Methoden [z.B. J.P. Greenstein, M. Winitz, Chemistry of the Amino Acids, John Wiley & Sons, N.Y. (1961), S. 943-945] genannt. Als vorteilhaft hat sich jedoch erwiesen, die Carbonsäuregruppe vor der Acylierungsreaktion in eine aktivierte Form wie z.B. Anhydrid, Aktivester oder Säurechlorid zu überführen [z.B. E. Gross, J. Meienhofer, The Peptides, Academic Press, N.Y. (1979), Vol. 1, S. 65-314; N.F. Albertson, Org. React. 12, 157 (1962)].

Im Falle der Umsetzung mit Aktivester sei auf die dem Fachmann bekannte Literatur [z.B. Houben-Weyl, Methoden der organischen Chemie, Georg Thieme Verlag, Stuttgart, Band E 5 (1985), 633] verwiesen. Sie kann unter den oben für die Anhydridreaktion angegebenen Bedingungen durchgeführt werden. Es können aber auch aprotische Lösungsmittel wie z.B. Methylenchlorid, Chloroform verwendet werden.

Im Falle der Säurechlorid-Umsetzungen werden nur aprotische Lösungsmittel wie z.B. Methylenchlorid, Chloroform, Toluol oder THF bei Temperaturen zwischen -20 bis 50 °C, bevorzugt zwischen 0 bis 30 °C, verwendet. Des weiteren sei auf die dem Fachmann bekannte Literatur [z.B. Houben-Weyl, Methoden der Organischen Chemie, Georg-Thieme-Verlag, Stuttgart, (1974), Band 15/2, S. 355-364] verwiesen.

Falls R1' für eine Säureschutzgruppe steht, kommen niedere Alkyl-, Aryl- und Aralkylgruppen, beispielsweise die Methyl-, Ethyl-, Propyl-, Butyl-, Phenyl-, Benzyl-, Diphenylmethyl-, Triphenylmethyl-, bis-(p-Nitrophenyl)-methylgruppe, sowie Trialkylsilylgruppen in Frage.

Die gegebenenfalls gewünschte Abspaltung der Schutzgruppen erfolgt nach den dem Fachmann bekannten Verfahren, beispielsweise durch Hydrolyse, Hydrogenolyse, alkalische Verseifung der Ester mit Alkali in wäßrig-alkoholischer Lösung bei Temperaturen von 0 °C bis 50 °C oder im Fall von tert.-Butylestern mit Hilfe von Trifluoressigsäure.

Gegebenenfalls unvollständig mit Ligand oder Komplex acylierte terminale Äminogruppen können, wenn gewünscht, in Amide oder Halbamide überführt werden. Beispielhaft genannt sei die Umsetzung mit Acetanhydrid, Bernsteinsäureanhydrid oder Diglykolsäureanhydrid.

Die Einführung der gewünschten Metallionen erfolgt in der Weise, wie sie z.B. in der Deutschen Offenlegungsschrift 34 01 052 offenbart worden ist, indem man das Metalloxid oder ein Metallsalz (beispielsweise das Nitrat, Acetat, Carbonat, Chlorid oder Sulfat) des Elements der Ordnungszahlen 20-29, 42, 44, 57-83 in Wasser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene acide Wasserstoffatome der Säuregruppen durch Kationen von anorganischen und/oder organischen Basen, Aminosäuren oder Aminosäureamiden substituiert.

Die Einführung der gewünschten Metallionen kann sowohl auf der Stufe der Komplexbildner I'A oder I'B, d.h. vor der Kopplung an die Kaskaden-Polymere, als auch nach Kopplung der unmetallierten Liganden I'A oder I'B erfolgen.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin oder von Amiden ursprünglich neutraler oder saurer Aminosäuren, wie zum Beispiel Hippursäure, Glycinacetamid.

Zur Herstellung der neutralen Komplexverbindungen kann man beispielsweise den sauren Komplexsalzen in wäßriger Lösung oder Suspension so viel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man den komplexbildenden Liganden in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Die Reinigung der so erhaltenen Kaskaden-Polymer-Komplexe erfolgt, gegebenenfalls nach Einstellung des pH-Wertes durch Zusatz einer Säure oder Base auf pH6 bis 8, bevorzugt ca. 7, vorzugsweise durch Ultrafiltration mit Membranen geeigneter Posengröße (z.B. Amicon®XM30, Amicon®YM10, Amicon®YM3) oder Gelfiltration an z.B. geeigneten Sephadex®-Gelen.

Im Falle von neutralen Komplexverbindungen ist es häufig von Vorteil, die polymeren Komplexe über einen Anionenaustauscher, beispielsweise IRA 67 (OH⁻-Form) und gegebenenfalls zusätzlich über einen Kationenaustauscher, beispielsweise IRC 50 (H⁺-Form) zur Abtrennung ionischer Komponenten zu geben.

Die Herstellung der für die Kopplung an die Komplexbildner K' (bzw. auch die entsprechenden metallhaltigen Komplexe) benötigten terminale Aminogruppen tragenden Kaskaden-Polymere geht im allgemeinen aus von käuflichen bzw. nach oder analog literaturbekannten Methoden herstellbaren stickstoffhaltigen Kaskadenstartern A(H)ₐ. Die Einführung der Generationen X, Y, Z und W erfolgt nach literaturbekannten Methoden [z.B. J. March, Advanced Organic Chemistry, 3^{rd} ed.; John Wiley & Sons, (1985), 364-381] durch Acylierungs- bzw. Alkylierungsreaktionen mit die gewünschten Strukturen aufweisenden geschützten Aminen, die zur Bindung an den Kaskadenkern befähigte funktionelle Gruppen wie z.B. Carbonsäuren, Isocyanate, Isothiocyanate oder aktivierte Carbonsäuren (wie z.B. Anhydride, Aktivester, Säurechloride) bzw. Halogenide (wie z.B. Chloride, Bromide, Iodide), Aziridin, Mesylate, Tosylate oder andere dem Fachmann bekannte Fluchtgruppen enthalten.

Es sei jedoch hier nochmals betont, daß die Unterscheidung zwischen Kaskadenkern A und Reproduktionseinheiten rein formal ist. Es kann synthetisch von Vorteil sein, daß man nicht den formalen Kaskadenstarter A(H)ₐ verwendet, sondern die per Definition zum Kaskadenkern gehörigen Stickstoffatome erst zusammen mit der ersten Generation einführt. So ist es z.B. zur Synthese der in Beispiel 1b) beschriebenen Verbindung vorteilhafter, nicht den formalen Kaskadenkern Trimesinsäuretriamid mit z.B. Benzyloxycarbonylaziridin (sechsfach) zu alkylieren, sondern Trimesinsäuretrichlorid mit Bis[2-(benzyloxycarbonylamino)-ethyl]-amin (dreifach) umzusetzen.

Als Aminschutzgruppen seien die dem Fachmann geläufigen Benzyloxycarbonyl-, tertiär-Butoxycarbonyl-, Trifluoracetyl-, Fluorenylmethoxycarbonyl-, Benzyl- und Formylgruppe [Th. W. Greene, P.G.M Wuts, Protective Groups in Organic Syntheses, 2nd ed, John Wiley and Sons (1991), S. 309-385] genannt. Nach Abspaltung dieser Schutzgruppen, die ebenfalls nach literaturbekannten Methoden erfolgt, kann die nächste gewünschte Generation in das Molekül eingeführt werden. Neben diesem aus jeweils zwei Reaktionsstufen (Alkylierung bzw. Acylierung und Schutzgruppenabspaltung) bestehenden Aufbau einer Generation ist auch mit ebenfalls nur zwei Reaktionsstufen die gleichzeitige Einführung von zwei, z.B. X-[Y]x, oder mehrerer Generationen, z.B. X-[Y-(Z)_{y}]ₓ, möglich. Der Aufbau dieser Mehrgenerationen-Einheiten erfolgt durch Alkylierung bzw. Acylierung von die Strukturen der gewünschten Reproduktionseinheiten aufweisenden ungeschützten Aminen ("Reproduktionsamin") mit einem zweiten Reproduktionsamin, dessen Amingruppen in geschützter Form vorliegen.

Die als Kaskadenstarter benötigten Verbindungen der allgemeinen Formel A(H)ₐ sind käuflich zu erwerben oder nach bzw. analog literaturbekannten Methoden [z.B. Houben-Weyl, Methoden der Org. Chemie, Georg-Thieme-Verlag, Stutgart (1957), Bd. 11/1; M. Micheloni et al., Inorg. Chem. (1985), 24, 3702; T.J. Atkins et al., Org. Synth., Vol. 58 (1978), 86-98; The Chemistry of Heterocyclic Compounds: J.S. Bradshaw et al., Aza-Crown-Macrocycles, John Wiley & Sons, N.Y. (1993)] herstellbar. Beispielhaft angeführt seien:
Tris(aminoethyl)amin [z.B. Fluka Chemie AG, Schweiz; Aldrich-Chemie, Deutschland];
Tris(aminopropyl)amin [z.B. C. Woerner et al., Angew. Chem. Int. Ed. Engl. (1993), 32, 1306];
Diethylentriamin [z.B. Fluka; Aldrich];
Triethylentetramin [z.B. Fluka; Aldrich];
Tetraethylenpentamin [z.B. Fluka; Aldrich];
1,3,5-Tris(aminomethyl)benzol [z.B. T.M. Garrett et al., J. Am. Chem. Soc. (1991), 113, 2965];
Trimesinsäuretriamid [z.B. H. Kurihara; Jpn. Kokai Tokkyo Koho JP 04077481;
CA 117, 162453];
1,4,7-Triazacyclononan [z.B. Fluka; Aldrich];
1,4,7,10,13-Pentaazacyclopentadecan [z.B. K.W. Aston, Eur. Pat. Appl. 0.524 161,
CA 120, 44580];
1,4,7,10-Tetraazacyclododecan [z.B. Aldrich]
1,4,8,11-Tetraazacyclotetradecan [z.B. Fluka; Aldrich];
1,4,7,10,13,16,19,22,25,28-Decaazacyclotriacontan [z.B. A. Andres et al., J. Chem. Soc. Dalton Trans. (1993), 3507];
1,1,1-Tris(aminomethyl)ethan [z.B. R.J. Geue et al., Aust. J. Chem. (1983), 36, 927];
Tris(aminopropyl)-nitromethan [z.B. G.R. Newkome et al., Angew. Chem. 103, 1205 (1991) analog zu R.C. Larock, Comprehensive Organic Transformations, VCH Publishers, N.Y. (1989), 419-420]
1,3,5,7-Adamantantetracarbonsäureamid [z.B. H. Stetter et al., Tetr. Lett. 1967, 1841];
1,2-Bis[Phenoxyethan]-3',3",5',5"-tetracarbonsäureamid [z.B. J.P. Collman et al.; J. Am. Chem. Soc. (1988), 110, 3477-86 analog zur Vorschrift für das Beispiel 1b)];
1,4,7,10,13,16,21,24-Octaazabicyclo[8.8.8.]hexacosan [z.B. P.H. Smith et al., J. Org. Chem. (1993), 58, 7939].

Die Herstellung der für den Aufbau der Generationen benötigten die oben genannten funktionellen Gruppen enthaltenden Reproduktionsamine erfolgt nach bzw. analog den im experimentellen Teil beschriebenen Vorschriften bzw. nach literaturbekannten Verfahren.

Beispielhaft genannt seien:
N^{α},N^{ε}-Di-Benzyloxycarbonyl-Lysin-p-nitrophenylester [s. Vorschrift für Beispiel 1c)];
   HOOC-CH₂OCH₂CO-N(CH₂CH₂NH-CO-O-CH₂C₆H₅)₂ ; HOOC-CH₂N(CH₂CH₂NH-CO-O-CH₂C₆H₅)₂ ; HOOC-CH₂CH₂CO-N(CH₂CH₂NH-COCF₃)₂ [herzustellen nach Vorschrift für das Beispiel 3a), indem man anstelle von Bis(benzyloxycarbonylaminoethyl)amin von Bis(trifluoracetylaminoethyl)amin und anstelle von Diglycolsäureanhydrid von Bemsteinsäureanhydrid ausgeht]; HOOC-CH₂OCH₂CONH-C₆H₄-CH[CH₂CON(CH₂CH₂NH-CO-O-CH₂C₆H₅)₂]₂ [herzustellen analog der Vorschrift für Beispiel 3a); O=C=N-C₆H₄-CH[CH₂CON(CH₂CH₂NH-CO-O-CH₂C₆H₅)₂]₂

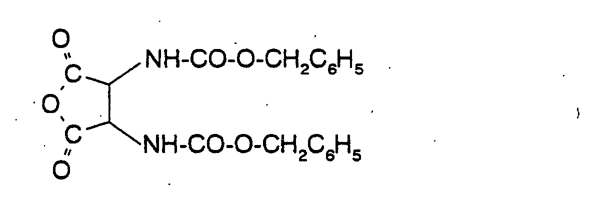

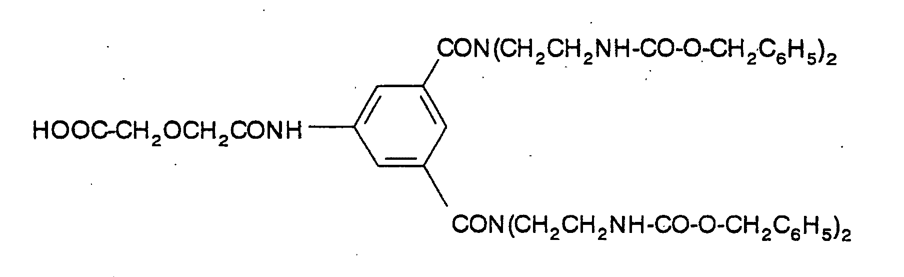

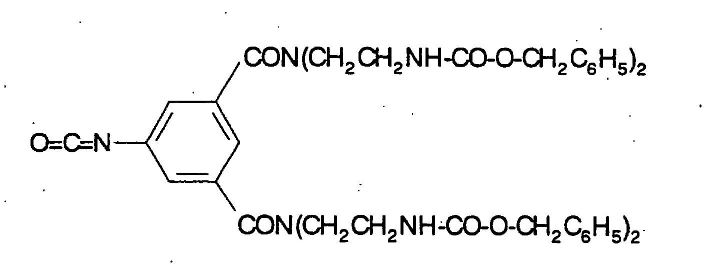

- N-Benzyloxycarbonyl-aziridin: herzustellen nach M. Zinic et al., J. Chem. Soc, Perkin Trans 1, 21-26 (1993)
- N-Benzyloxycarbonyl-glycin: käuflich bei z.B. Bachem California 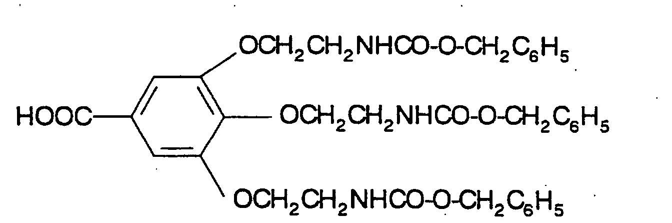 herzustellen nach C.J. Cavallito et al.,
J. Amer. Chem. Soc. 1943, 65, 2140, indem man anstelle von Benzylchlorid von N-CO-O-CH₂C₆H₅-(2-Bromethyl)amin [A.R. Jacobson et al.,
J. Med. Chem. (1991), 34, 2816] ausgeht.

Die Herstellung der Komplexe und Komplexbildner der allgemeinen Formel I'A und I'B erfolgt nach bzw. analog den im experimentellen Teil beschriebenen Vorschriften bzw. nach literaturbekannten Methoden (s. z.B. Europäische Patentanmeldungen Nr. 0 512 661, 0 430 863, 0 255 471 und 0 565 930.

So kann die Herstellung von Verbindungen der allgemeinen Formel I'A z.B. dadurch erfolgen, daß als Vorstufe der funktionellen Gruppe T' eine Gruppe T" dient, entweder in der Bedeutung einer geschützten Säurefunktion, die unabhängig von den Säureschutzgruppen R^{1'} nach den oben aufgeführten Verfahren in die freie Säurefunktion überführt werden kann, oder in der Bedeutung einer geschützten Aminfunktion, die nach literaturbekannten Verfahren deblockiert [Th.W. Greene, P.G.M. Wuts, Protective Groups in Organic Synthesis, 2nd edition, John Wiley & Sons (1991), S. 309-385] und anschließend in die Isocyanate bzw. Isothiocyanate überführt werden kann [Methoden der Org. Chemie (Houben-Weyl), E 4, S. 742-749, 837-843, Georg Thieme Verlag, Stuttgart, New York (1983)]. Solche Verbindungen sind nach bzw. analog den im experimentellen Teil beschriebenen Vorschriften durch Monoalkylierung von Cyclen mit geeigneten α-Halogen-carbonsäureamiden [in aprotischen Lösungsmitteln, wie z.B. Chloroform] herstellbar.

Die Herstellung von Verbindungen der allgemeinen Formel I'B kann beispielsweise dadurch erfolgen, daß als Vorstufe der aktivierten Carboxylgruppe-C*O- eine geschützte Säurefunktion dient, die unabhängig von den Säureschutzgruppen R^{1'} nach den oben aufgeführten Verfahren in die freie Säurefunktion überführt und nach den ebenfalls oben beschriebenen literaturbekannten Verfahren aktiviert werden kann. Solche Verbindungen sind nach bzw. analog den im experimentellen Teil beschriebenen Vorschriften herstellbar oder beispielsweise dadurch, daß ein Aminosäurederivat der allgemeinen Formel II 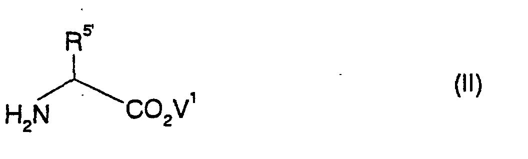 worin
- R^{5'}: die für R⁵ angegebene Bedeutung hat, wobei gegebenenfalls in R⁵ enthaltene Hydroxy- oder Carboxygruppen gegebenenfalls in geschützter Form vorliegen und
- V¹: eine geradkettige oder verzweigte C1-C6-Alkylgruppe, eine Benzyl-, Trimethylsilyl-Triisopropylsilyl-, 2,2,2,-Trifluorethoxy- oder 2,2,2,-Trichlorethoxygruppe, wobei V¹ verschieden von R^{1"} ist, mit einem Alkylierungsagenz der allgemeinen Formel III 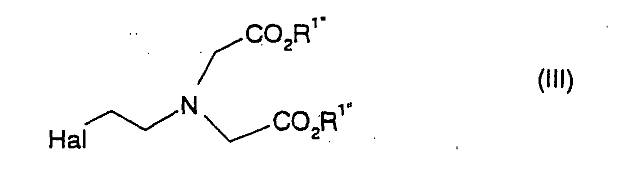
worin
R^{1"} für eine Schutzgruppe und
Hal für ein Halogenatom wie Cl, Br oder I, bevorzugt jedoch Cl, steht,
umgesetzt wird [siehe auch M.A. Williams, H. Rapoport, J. Org. Chem. 58, 1151(1993)].

Bevorzugte Aminosäurederivate sind die Ester von natürlich vorkommenden α-Aminosäuren.

Die Reaktion von Verbindung (II) mit Verbindung (III) erfolgt bevorzugt in einer gepufferten Alkylierungsreaktion, wobei als Puffer eine wäßrige Phosphat-Pufferlösung dient. Die Umsetzung erfolgt bei pH-Werten von 7-9, bevorzugt jedoch bei pH8. Die Pufferkonzentration kann zwischen 0,1-2,5 M liegen, bevorzugt wird jedoch eine 2 M-Phosphat-Pufferlösung verwendet. Die Temperatur der Alkylierung kann zwischen 0 und 50 °C liegen; die bevorzugte Temperatur ist Raumtemperatur.

Die Reaktion wird in einem polaren Lösungsmittel, wie z.B. Acetonitril, Tetrahydrofuran, 1,4-Dioxan oder 1,2-Dimethoxyethan durchgeführt. Bevorzugt wird Acetonitril verwendet.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern oder schwachen Komplexen (wie zum Beispiel Diethylentriaminpentaessigsäure oder die korrespondierenden Ca-Kaskaden-Polymer-Komplexe) oder - falls erforderlich - Elektrolyte wie zum Beispiel Natriumchlorid oder-falls erforderlich - Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) [zum Beispiel Methylcellulose, Lactose, Mannit] und/oder Tensid(en) [zum Beispiel Lecithine, Tween®, Myrj® ] und/oder Aromastoff(en) zur Geschmackskorrektur [zum Beispiel ätherischen Ölen] gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 1µMol- 1,3 Mol/l des Komplexsalzes und werden in der Regel in Mengen von 0,0001-5 mMol/kg dosiert. Sie sind zur enteralen und parenteralen Applikation bestimmt. Die erfindungsgemäßen Komplexverbindungen kommen zur Anwendung
1. für die NMR- und Röntgen-Diagnostik in Form ihrer Komplexe mit den Ionen der Elemente mit den Ordnungszahlen 21-29, 39, 42, 44 und 57-83;
2. für die Radiodiagnostik und Radiotherapie in Form ihrer Komplexe mit den Radioisotopen der Elemente mit den Ordnungszahlen 27, 29, 31, 32, 37-39, 43, 49, 62, 64, 70, 75 und 77.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit - Hilfe des Kernspintomographen erhaltene Bild in seiner Aussagekraft zu verbessern.
Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, das heißt NMR-Diagnostika müssen 100- bis 1000fach besser wasserlöslich sein als für die NMR-Spektroskopie. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen mittel für die Anwendung als NMR-Diagnostika in Mengen von 0,0001-5 mMol/kg, vorzugsweise 0,005- 0,5 mMol/kg, dosiert. Details der Anwendung werden zum Beispiel in H.-J. Weinmann et al., Am. J. of Roentgenology 142, 619 (1984) diskutiert.

Besonders niedrige Dosierungen (unter 1 mg/kg Körpergewicht) von organspezifischen NMR-Diagnostika sind zum Beispiel zum Nachweis von Tumoren und von Herzinfarkt einsetzbar.

Ferner können die erfindungsgemäßen Komplexverbindungen vorteilhaft als Suszeptibilitäts-Reagenzien und als shift-Reagenzien für die in-vivo-NMR-Spektroskopie verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer Anwendung und Dosierung werden z.B. in "Radiotracers for Medical Applications", CRC-Press, Boca Raton, Florida, beschrieben.

Eine weitere bildgebende Methode mit Radioisotopen ist die Positronen-Emissions-Tomographie, die positronenemittierende Isotope wie z.B. ⁴³Sc, ⁴⁴Sc, ⁵²Fe, ⁵⁵Co und ⁶⁸Ga verwendet (Heiss, W.D.; Phelps, M.E.; Positron Emission Tomography of Brain, Springer Verlag Berlin, Heidelberg, New York 1983).

Die erfindungsgemäßen Verbindungen sind überraschenderweise auch zur Differenzierung von malignen und benignen Tumoren in Bereichen ohne Blut-Hirn-Schranke geeignet.

Sie zeichnen sich auch dadurch aus, daß sie vollständig aus dem Körper eliminiert werden und somit gut verträglich sind.

Da sich die erfindungsgemäßen Substanzen in malignen Tumoren anreichern (keine Diffusion in gesunde Gewebe, aber hohe Durchlässigkeit von Tumorgefäßen), können sie auch die Strahlentherapie von malignen Tumoren unterstützen. Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Hierzu werden Wechselwirkungen der in den Komplexen enthaltenen Metalle (wie z.B. Eisen oder Gadolinium) mit ionisierenden Strahlungen (z.B. Röntgenstrahlen) oder mit Neutronenstrahlen ausgenutzt. Durch diesen Effekt wird die lokale Strahlendosis am Ort, wo sich der Metallkomplex befindet (z.B. in Tumoren) signifikant erhöht. Um die gleiche Strahlendosis im malignen Gewebe zu erzeugen, kann bei Anwendung solcher Metallkomplexe die Strahlenbelastung für gesunde Gewebe erheblich reduziert und damit belastende Nebenwirkungen für die Patienten vermieden werden. Die erfindungsgemäßen Metallkomplex-Konjugate eignen sich deshalb auch als radiosensibilisierende Substanz bei Strählentherapie von malignen Tumoren (z.B. Ausnutzen von Mössbauer-Effekten oder bei Neutroneneinfangtherapie). Geeignete β-emittierende Ionen sind zum Beispiel ⁴⁶Sc, ⁴⁷Sc, ⁴⁸Sc, ⁷²Ga, ⁷³Ga und ⁹⁰Y. Geeignete geringe Halbwertzeiten aufweisende α-emittierende Ionen sind zum Beispiel ²¹¹Bi, ²¹²Bi, ²¹³Bi und ²¹⁴Bi, wobei ²¹²Bi bevorzugt ist. Ein geeignetes Photonen- und Elektronenemittierendes Ion ist ¹⁵⁸Gd, das aus ¹⁵⁷Gd durch Neutroneneinfang erhalten werden kann.

Ist das erfindungsgemäße Mittel zur Anwendung in der von R.L. Mills et al. (Nature Vol. 336, (1988), S. 787] vorgeschlagenen Variante der Strahlentherapie bestimmt, so muß sich das Zentralion von einem Mößbauer-Isotop wie beispielsweise ⁵⁷Fe oder ¹⁵¹Eu ableiten.

Bei der in-vivo-Applikation der erfindungsgemäßen therapeutischen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der bildgebenden Methode.

Die erfindungsgemäßen therapeutischen Mittel werden parenteral, vorzugsweise i.v., appliziert.

Details der Anwendung von Radiotherapeutika werden z.B. in R.W. Kozak et al. TIBTEC, Oktober 1986, 262, diskutiert.

Die erfindungsgemäßen Mittel sind hervorragend als Röntgenkontrastmittel geeignet, insbesondere für die Computertomographie (CT), wobei besonders hervorzuheben ist, daß sich mit ihnen keine Anzeichen der von den jodhaltigen Kontrastmitteln bekannten anaphylaxieartigen Reaktionen in biochemisch-pharmakologischen Untersuchungen erkennen lassen. Besonders wertvoll sind sie wegen der günstigen Absorptionseigenschaften in Bereichen höherer Röhrenspannungen für digitale Substraktionstechniken.

Im allgemeinen werden die erfindungsgemäßen Mittel für die Anwendung als Röntgenkontrastmittel in Analogie zu zum Beispiel Meglumin-Diatrizoat in Mengen von 0,1-5 mMol/kg, vorzugsweise 0,25-1 mMol/kg, dosiert.

Details der Anwendung von Röntgenkontrastmitteln werden zum Beispiel in Barke, Röntgenkontrastmittel, G. Thieme, Leipzig (1970) und P. Thurn, E. Bücheler "Einführung in die Röntgendiagnostik", G. Thieme, Stuttgart, New York (1977) diskutiert.

Insgesamt ist es gelungen, neue Komplexbildner, Metallkomplexe und Metallkomplexsalze zu synthetisieren, die neue Möglichkeiten in der diagnostischen und therapeutischen Medizin erschließen.

Die nachfolgenden Beispiele dienen zur näheren Erläuterung des Erfindungsgegenstands:

### Beispiel 1

### a) Bis[2-(benzyloxycarbonylamino)-ethyl-amin

51,5 g (500 mmol) Diethylentriamin und 139 ml (1 mol) Triethylamin werden in Dichlormethan gelöst und bei -20°C mit 161 g Benzylcyanformiat (Fluka) in Dichlormethan versetzt und anschließend über Nacht bei Raumtemperatur gerührt. Nach Beendigung der Reaktion wird im Abzug eingedampft, der Rückstand in Diethylether aufgenommen, die organische Phase mit Natriumcarbonatlösung gewaschen und mit Natriumsulfat getrocknet. Das Filtrat wird mit Hexan versetzt, der Niederschlag abfiltriert und getrocknet. Ausbeute: 163,4 g (88 % d. Th.)

| Elementaranalyse: | | | |
|---|---|---|---|
| ber. | C 64,67 | H 6,78 | N 11,31 |
| gef. | C 64,58 | H 6,83 | N 11,28 |

### b) N,N,N',N',N",N"-Hexakis[2-(benzyloxycarbonylamino)-ethyl]-trimesinsäuretriamid

13,27 g (50 mmol) Trimesinsäure-trichlorid (Aldrich) und 34,7 ml (250 mmol) Triethylamin werden in Dimethylformamid (DMF) gelöst und bei 0°C mit 65,0 g (175 mmol) des in Beispiel 1a) beschriebenen Amins versetzt und anschließend über Nacht bei Raumtemperatur gerührt. Die Lösung wird im Vakuum eingedampft und der Rückstand mit Ethylacetat an Kieselgel chromatographiert.
Ausbeute: 39,4 g (62 % d. Th.)

| Elementaranalyse: | | | |
|---|---|---|---|
| ber. | C 65,24 | H 5,95 | N 9,92 |
| gef. | C 65,54 | H 5,95 | N 9,87 |

### c) N^{α}, N^{ε}-Bis(N,N'-dibenzyloxycarbonyl-lysyl)-lysin, geschütztes "Tri-Lysin"

3,6 g (20 mmol) Lysin-Hydrochlorid und 6,95 ml (50 mmol) Triethylamin werden in DMF gelöst, mit 26,8 g (50 mmol) N^{α}, N^{ε}-Dibenzyloxycarbonyl-Lysin-p-nitrophenylester (Bachem) versetzt und 2 Tage bei Raumtemperatur gerührt. Nach Beendigung der Reaktion wird im Vakuum eingedampft, der Rückstand in Ethylacetat aufgenommen und mit verdünnter Salzsäure ausgeschüttelt. Die organische Phase wird mit Natriumsulfat getrocknet, das Lösungsmittel eingedampft und der Rückstand mit Ethylacetat/Ethanol in einem Stufengradienten chromatographiert.
Ausbeute: 10,7 g (57 % d. Th.)

| Elementaranalyse: | | | |
|---|---|---|---|
| ber. | C 63,95 | H 6,65 | N 8,95 |
| gef. | C 63,63 | H 6,69 | N 8,93 |

### d) Voll geschütztes Benzyloxycarbonyl-24-Polyamin auf der Basis des N,N,N',N',N",N"-Hexakis[2-(trilysyl-amino)-ethyl]-trimesinsäuretriamids

1,27 g (1 mmol) des im Beispiel 1b) beschriebenen Hexa-Benzyloxycarbonylamins werden in Eisessig gelöst und unter Rühren mit 33 %igem Bromwasserstoff in Eisessig versetzt. Nach 60 Minuten wird mit Diethylether die begonnene Fällung vervollständigt, das entstandene Hexaaminhydrobromid mit Ether gewaschen, im Vakuum getrocknet und ohne weitere Reinigung in die weiter unten beschriebene Reaktion eingesetzt.
Ausbeute: 0,95 g (quantitativ)

7,0 g (7,5 mmol) des in Beispiel 1c) beschriebenen geschützten "Tri-Lysins", 1,2 g (7,5 mmol) 1-Hydroxybenzotriazol und 2,4 g (7,5 mmol) 2-(1H-Benzotriazol-1-yl)-1,1,3,3-tetramethyluronium tetrafluorborat (TBTU; Peboc Limited, UK) werden in DMF gelöst und 15 Minuten gerührt. Diese Lösung wird anschließend mit 5,16 ml (30 mmol)
N-Ethyldiisopropylamin und mit 0,95 g (1 mmol) des oben beschriebenen Hexaaminhydrobromids versetzt und über Nacht bei Raumtemperatur gerührt. Nach Beendigung der Reaktion wird im Vakuum eingedampft und der Rückstand mit Ethylacetat/Ethanol (2:1) an Kieselgel chromatographiert.
Ausbeute: 4,55 g (76 % d. Th.)

| Elementaranalyse: | | | |
|---|---|---|---|
| ber. | C 64,35 | H 6,71 | N 10,52 |
| gef. | C 64,08 | H 6,57 | N 10,29 |

### e) 2-Brompropionylglycin-benzylester

Zu 100 g (296,4 mmol) Glycinbenzylester-p-Toluolsulfonsäuresalz und 33,0 g (326,1 mmol) Triethylamin in 400 ml Methylenchlorid tropft man bei 0 °C 55,9 g (326,1 mmol) 2-Brompropionsäurechlorid zu. Man läßt die Temperatur nicht über 5 °C kommen. Nach beendeter Zugabe wird eine Stunde bei 0 °C gerührt, anschließend 2 Stunden bei Raumtemperatur. Man setzt 500 ml Eiswasser zu und stellt die Wasserphase mit 10 % aqu. Salzsäure auf pH 2. Die organische Phase wird abgetrennt, je einmal mit 300 ml 5 % aqu. Sodalösung und 400 ml Wasser gewaschen. Man trocknet die organische Phase über Magnesiumsulfat und dampft im Vakuum zur Trockne ein. Der Rückstand wird aus Diisopropylether umkristallisiert.
Ausbeute: 68,51 g (75 % d. Th.) eines farblosen kristallinen Pulvers
Schmelzpunkt: 69-70°C

| Elementaranalyse: | | | | |
|---|---|---|---|---|
| ber. | C 46,76 | H 7,19 | N 4,54 | Br 25,92 |
| gef. | C 46,91 | H 7,28 | N 4,45 | Br 25,81 |

### f) 1-[4-(Benzyloxycarbonyl)-1-methyl-2-oxo-3-azabutyl]-1,4,7,10-tetraazacyclododecan

Zu 55,8 g (324,4 mmol) 1,4,7,10-Tetraazacyclododecan, gelöst in 600 ml Chloroform, gibt man 50 g (162,2 mmol) der Titelverbindung aus Beispiel 1e) und rührt über Nacht bei Raumtemperatur. Man gibt 500 ml Wasser zu, trennt die organische Phase ab und wäscht sie noch jeweils 2 mal mit 400 ml Wasser. Man trocknet die organische Phase über Magnesiumsulfat und dampft im Vakuum zur Trockne ein. Der Rückstand wird an Kieselgel chromatographiert (Laufmittel: Chloroform/Methanol/aqu. 25 % Ammoniak = 10/5/1).
Ausbeute. 40,0 g [63 % d. Th.bezogen auf eingesetztes 1e)] eines leicht gelblichen zähen Öls.

| Elementaranalyse: | | | |
|---|---|---|---|
| ber. | C 61,36 | H 8,50 | N 17,89 |
| gef. | C 61,54 | H 8,68 | N 17,68 |

### g) 10-[4-(Benzyloxycarbonyl)-1-methyl-2-oxo-3-azabutyl]-1,4,7-tris(tert.-butoxycarbonylmethyl)-1,4,7,10-tetraazacyclododecan (Natriumbromid-Komplex)

Zu 20 g (51,08 mmol) der Titelverbindung aus Beispiel 1f) und 17,91 (169 mmol) Natriumcarbonat in 300 ml Acetonitril gibt man 33 g (169 mmol) Bromessigsäure-tert.-butylester zu und rührt 24 Stunden bei 60 °C. Man kühlt auf 0 °C ab, filtriert von den Salzen ab und dampft das Filtrat zur Trockne ein. Der Rückstand wird an Kieselgel chromatographiert (Laufmittel: Essigsäureethylester/Ethanol: 15/1). Die das Produkt enthaltenden Fraktionen werden eingedampft und der Rückstand aus Diisopropylether umkristallisiert.
Ausbeute: 34,62 g (81 % d. Th.) eines farblosen kristallinen Pulvers
Schmelzpunkt: 116-117 °C

| Elementaranalyse: | | | | | |
|---|---|---|---|---|---|
| ber. | C 54,54 | H 7,59 | N 8,37 | Na 2,74 | Br 9,56 |
| gef. | C 54,70 | H 7,65 | N 8,24 | Na 2,60 | Br 9,37 |

### h) 10-(4-Carboxy-1-methyl-2-oxo-3-azabutyl)-1,4,7-tris(tert.-butoxycarbonylmethyl)-1,4,7,10-tetraazacyclododecan(Natriumbromid-Komplex)

30 g (35,85 mmol) der Titelverbindung aus Beispiel 1g werden in 500 ml Isopropanol gelöst und 3 g Palladiumkatalysator (10 % Pd/C) hinzugegeben. Man hydriert über Nacht bei Raumtemperatur. Es wird vom Katalysator abfiltriert, das Filtrat im Vakuum zur Trockne eingedampft und aus Aceton umkristallisiert.
Ausbeute: 22,75 g (85 % d. Th.) eines farblosen kristallinen Pulvers
Schmelzpunkt: 225 °C (Zers.)

| Elementaranalyse: | | | | | |
|---|---|---|---|---|---|
| ber. | C 49,86 | H 7,69 | N 9,38 | Na 3,07 | Br 10,71 |
| gef. | C 49,75 | H 7,81 | N 9,25 | Na 2,94 | Br 10,58 |

*) DO3A= 1,4,7-Tris(carboxymethyl)-1,4,7,10-tetraazacyclododecan

### i) 24-mer N-(5-DO3A-yl-4-oxo-3-azahexanoyl)-Kaskadenpolyamid auf der Basis des N,N,N',N',N",N"-Hexakis[2-(trilysylamino)-ethyl]-trimesinsäuretriamids *)

6,0 g (1 mmol) des in Beispiel 1d) beschriebenen Poly-Benzyloxycarbonylamins werden in Eisessig gelöst und unter Rühren mit 33 %igem Bromwasserstoff in Eisessig versetzt. Nach 3 Stunden wird mit Diethylether die begonnene Fällung vervollständigt, das entstandene 24-Amin-hydrobromid mit Ether gewaschen und im Vakuum getrocknet.

35,84 g (48 mmol) der im vorstehenden Beispiel 1h) beschriebenen Säure werden in DMF gelöst, mit 7,35 g (48 mmol) 1-Hydroxybenzotriazol, mit 15,41 g (48 mmol) TBTU (Peboc Limited, UK) und mit 49,3 ml (288 mmol) N-Ethyldiisopropylamin versetzt und 20 Minuten bei Raumtemperatur gerührt. Diese Lösung wird anschließend mit dem oben beschriebenen (1 mmol) 24-Aminhydrobromid versetzt und 4 Tage bei Raumtemperatur gerührt. Die Lösung wird im Vakuum eingeengt, das verbleibende Öl im Eisbad gekühlt und mit Trifluoressigsäure versetzt, über Nacht bei Raumtemperatur gerührt und anschließend mit Diethylether gefällt. Der Niederschlag wird im Vakuum getrocknet, in Wasser aufgenommen, auf pH 7 eingestellt, über eine YM3 Amicon® -Ultrafiltrationsmembran von niedermolekularen Anteilen gereinigt und das Retentat schließlich membranfiltriert und gefriergetrocknet.
Ausbeute: 13,5 g (83 % d.Th.)
H₂O-Gehalt (Karl-Fischer): 6,2 %

| Elementaranalyse (bezogen auf wasserfreie Substanz): | | | | |
|---|---|---|---|---|
| ber. | C 45,82 | H 6,09 | N 15,07 | Na 10,79 |
| gef. | C 45,56 | H 6,15 | N 14,80 | Na 10,52 |

### k) 24-mer-Gd-Komplex des N-(5-DO3A-yl-4-oxo-3-azahexanoyl)-Kaskadenpolyamids auf der Basis des N,N,N',N',N",N"-Hexakis[2-(trilysylamino)-ethyl]-trimesinsäuretriamids

8,13 g (0,5 mmol) der im vorstehenden Beispiel 1i) beschriebenen Komplexbildnersäure werden in Wasser mit verd. Salzsäure auf pH 3 gestellt, mit 2,17 g (6 mmol) Gd₂O₃ versetzt, 30 Minuten bei 80 °C gerührt, nach dem Abkühlen auf pH 7 eingestellt und über eine YM3 AMICON®-Ultrafiltrationsmembran entsalzt. Das Retentat wird schließlich membranfiltriert und gefriergetrocknet.
Ausbeute: 8,89 g (92,1 % d. Th.)
H₂O-Gehalt (Karl-Fischer): 9,6 %
Gd-Bestimmung (AAS): 19,6 %

| Elementaranalyse (bezogen auf wasserfreie Substanz): | | | | |
|---|---|---|---|---|
| ber. | C 40,26 | H 5,35 | N 13,24 | Gd 21,62 |
| gef. | C 39,98 | H 5,51 | N 13,42 | Gd 21,37 |

### Beispiel 2

### a) 2-Brompropionyl-β-alanin-benzylester

Zu 100 g (285 mmol) β-Alaninbenzylester-p-Toluolsulfonsäuresalz und 31,67 g (313 mmol) Triethylamin in 400 ml Methylenchlorid tropft man bei 0 °C 53,65 g (313 mmol) 2-Brompropionsäurechlorid zu. Man läßt die Temperatur nicht über 5 °C kommen. Nach beendeter Zugabe wird 1 Stunde bei 0 °C gerührt, anschließend 2 Stunden bei Raumtemperatur. Man setzt 500 ml Eiswasser zu und stellt die Wasserphase mit 10 % aqu. Salzsäure auf pH 2. Die organische Phase wird abgetrennt, je einmal mit 300 ml 5 % aqu. Salzsäure, 300 ml 5 % aqu. Sodalösung und 400 ml Wasser gewaschen. Man trocknet die organische Phase über Magnesiumsulfat und dampft im Vakuum zur Trockne ein. Der Rückstand wird aus
Diisopropylether umkristallisiert.
Ausbeute: 71,36 g (78 % d. Th.) eines farblosen kristallinen Pulvers

| Elementaranalyse: | | | | |
|---|---|---|---|---|
| ber. | C 48,46 | H 7,51 | N 4,35 | Br 24,80 |
| gef. | C 48,29 | H 7,65 | N 4,25 | Br 24,61 |

### b) 1-[5-(Benzyloxycarbonyl)-1-methyl-2-oxo-3-azapentyl]-1,4,7,10-tetraazacyclododecan

Zu 53,32 g (310 mmol) 1,4,7,10 Tetraazacyclododecan gelöst, in 600 ml Chloroform, gibt man 50 g (155,2 mmol) der Titelverbindung aus Beispiel 2a) und rührt über Nacht bei Raumtemperatur. Man gibt 500 ml Wasser zu, trennt die organische Phase ab und wäscht sie noch jeweils 2 mal mit 400 ml Wasser. Man trocknet die organische Phase über Magnesiumsulfat und dampft im Vakuum zur Trockne ein. Der Rückstand wird an Kieselgel chromatographiert (Laufmittel: Chloroform/Methanol/aqu. 25 % Ammoniak: 10/5/1). Ausbeute: 38,39 g [61 % d. Th.bezogen auf eingesetztes 2a)] eines leicht gelblichen zähen Öls.

| Elementaranalyse: | | | |
|---|---|---|---|
| ber. | C 62,20 | H 8,70 | N 17,27 |
| gef. | C 62,05 | H 8,81 | N 17,15 |

### c) 10-[5-(Benzyloxycarbonyl)-1-methyl-2-oxo-3-azapentyl]-1,4,7-tris(tert.-butoxycarbonylmethyl)-1,4,7,10-tetraazacyclododecan (Natriumbromid-Komplex)

Zu 20 g (49,32 mmol) der Titelverbindung aus Beispiel 2b) und 17,28 g (163 mmol) Natriumcarbonat in 300 ml Acetonitril gibt man 31,8 g (163 mmol) Bromessigsäure-tert.butylester zu und rührt 24 Stunden bei 60 °C. Man kühlt auf 0 °C ab, filtriert von den Salzen ab und dampft das Filtrat zur Trockne ein. Der Rückstand wird an Kieselgel chromatographiert (Laufmittel: Essigsäureethylester/Ethanol = 10/1). Die das Produkt enthaltenden Fraktionen werden eingedampft und der Rückstand aus Diisopropylether umkristallisiert.
Ausbeute: 31,89 g (76 % d. Th.) eines farblosen, kristallinen Pulvers

| Elementaranalyse: | | | | | |
|---|---|---|---|---|---|
| ber. | C 55,05 | H 7,70 | N 8,23 | Na 2,69 | Br 9,40 |
| gef. | C 55,17 | H 7,85 | N 8,10 | Na 2,51 | Br 9,30 |

### d) 10-[5-(carboxy)-1-methyl-2-oxo-3-azapentyl]-1,4,7-tris(tert.-butoxycarbonylmethyl)-1,4,7,10-tetraazacyclododecan (Natriumbromid-Komplex)

30 g (35,26 mmol) der Titelverbindung aus Beispiel 2c) werden in 500 ml Isopropanol gelöst und 3 g Palladiumkatalysator (10 % Pd/C) hinzugegeben. Man hydriert über Nacht bei Raumtemperatur. Es wird vom Katalysator abfiltriert, das Filtrat im Vakuum zur Trockne eingedampft und aus Aceton umkristallisiert.
Ausbeute: 24,41 g (91 % d. Th.) eines farblosen, kristallinen Pulvers

| Elementaranalyse: | | | | | |
|---|---|---|---|---|---|
| ber. | C 50,52 | H 7,82 | N 9,21 | Na 3,01 | Br 10,52 |
| gef. | C 50,41 | H 7,95 | N 9,10 | Na 2,91 | Br 10,37 |

### e) 24-mer N-(6-DO3A-yl-5-oxo-4-azaheptanoyl)-Kaskadenpolyamid auf der Basis des N,N,N',N',N",N"-Hexakis[2-(trilysylamino)-ethyl]-trimesinsäuretriamids

6,0 g (1 mmol) des in Beispiel 1d) beschriebenen Poly-Benzyloxycarbonylamins werden in Eisessig gelöst und unter Rühren mit 33 %igem Bromwasserstoff in Eisessig versetzt. Nach 3 Stunden wird mit Diethylether die begonnene Fällung vervollständigt, das entstandene 24-Amin-hydrobromid mit Ether gewaschen und im Vakuum getrocknet.
36,52 g (48 mmol) der im vorstehenden Beispiel 2d) beschriebenen Säure werden in DMF gelöst, mit 7,35 g (48 mmol) 1-Hydroxybenzotriazol, mit 15,41 g (48 mmol) TBTU (Peboc Limited, UK) und mit 49,3 ml (288 mmol) N-Ethyldiisopropylamin versetzt und 20 Minuten bei Raumtemperatur gerührt. Diese Lösung wird anschließend mit den oben beschriebenen (1 mmol) 24-Aminhydrobromid versetzt und 4 Tage bei Raumtemperatur gerührt. Die Lösung wird im Vakuum eingeengt, das verbleibende Öl im Eisbad gekühlt und mit Trifluoressigsäure versetzt, über Nacht bei Raumtemperatur gerührt und anschließend mit Diethylether gefällt. Der Niederschlag wird im Vakuum getrocknet, in Wasser aufgenommen, auf pH 7 eingestellt, über eine YM3 Amicon® -Ultrafiltrationsmembran von niedermolekularen Anteilen gereinigt und das Retentat schließlich membranfiltriert und gefriergetrocknet.
Ausbeute: 14,4 g (85 % d.Th.)
H₂O-Gehalt (Karl-Fischer): 8,7 %

| Elementaranalyse (bezogen auf wasserfreie Substanz): | | | | |
|---|---|---|---|---|
| ber. | C 46,82 | H 5,98 | N 14,79 | Na 10,59 |
| gef. | C 47,04 | H 6,23 | N 14,96 | Na 10,26 |

### f) 24-mer-Gd-Komplex des N-(6-DO3A-yl-5-oxo-4-azaheptanoyl)-Kaskadenpolyamids auf der Basis des N,N,N',N',N",N"-Hexakis[2-(trilysylamino)-ethyl-trimesinsäuretriamids

8,5 g (0,5 mmol) der im vorstehenden Beispiel 2e) beschriebenen Komplexbildnersäure werden in Wasser mit verd. Salzsäure auf pH 3 gestellt, mit 2,17 g (6 mmol) Gd₂O₃ versetzt, 30 Minuten bei 80 °C gerührt, nach dem Abkühlen auf pH 7 eingestellt und über eine YM3 AMICON®-Ultrafiltrationsmembran entsalzt. Das Retentat wird schließlich membranfiltriert und gefriergetrocknet.
Ausbeute: 8,50 g (88 % d. Th.)
H₂O-Gehalt (Karl-Fischer): 7,9 %
Gd-Bestimmung (AAS): 19,4 %

| Elementaranalyse (bezogen auf wasserfreie Substanz): | | | | |
|---|---|---|---|---|
| ber. | C 41,12 | H 5,52 | N 12,99 | Gd 21,21 |
| gef. | C 40,86 | H 5,34 | N 13,25 | Gd 20,95 |

### Beispiel 3

### a) N,N'-Bis(benzyloxycarbonyl)-3-[carboxymethoxyacetyl]-3-azapentan-1,5-diamin

37,14 g (100 mmol) des in Beispiel 1a) beschriebenen Bis(benzyloxycarbonyl-aminoethyl) amins werden in DMF gelöst, im Eisbad mit 17,4 g (150 mmol) Diglykolsäureanhydrid (Janssen Chimica) und 21 ml (150 mmol) Triethylamin versetzt und anschließend über Nacht bei Raumtemperatur gerührt. Die Lösung wird im Vakuum eingedampft, der Rückstand in Ethylacetat aufgenommen und mit verdünnter Salzsäure ausgeschüttelt. Die organische Phase wird mit Natriumsulfat getrocknet und nach Filtration vom Trocknungsmittel durch Zugabe von Hexan kristallisiert.
Ausbeute: 41,4 g (85 % d. Th.)

| Elementaranalyse: | | | |
|---|---|---|---|
| ber. | C 59,13 | H 6,00 | N 8,62 |
| gef. | C 58,99 | H 5,93 | N 8,70 |

### b) N,N',N",N"'-Tetrakis{8-(Benzyloxycarbonylamino)-6-[2-(benzyloxycarbonylaminoethyl]-5-oxo-3-oxaoctanoyl}cyclen

345 mg (2 mmol) 1,4,7,10-Tetraazacyclododecan (Cyclen; Fluka) werden mit Toluol azeotrop entwässert. Zu der abgekühlten Lösung von Cyclen in Toluol wird bei Raumtemperatur eine Lösung von 4,88 g (10 mmol) N,N'-Bis(benzyloxycarbonyl)-3-[carboxymethoxyacetyl]-3-azapentan-1,5-diamin [Beispiel 3a)] in Tetrahydrofuran (THF) sowie 2,47 g (10 mmol) 2-Ethoxy-1-ethoxycarbonyl-1,2-dihydrochinolin (EEDQ; Fluka) zugegeben und über Nacht gerührt. Nach Beendigung der Reaktion wird das Produkt durch Zugabe von Hexan ausgefällt, vom Lösungsmittel abdekantiert und noch einmal aus THF/Hexan und anschließend aus THF/Toluol umgefällt. Man erhält nach Trocknung im Vakuum 2,78 g (68 % d. Th.) eines blaßgelben Feststoffs.

| Elementaranalyse: | | | |
|---|---|---|---|
| ber. | C 60,93 | H 6,29 | N 10,93 |
| gef. | C 60,68 | H 6,40 | N 10,97 |

### c) Vollgeschütztes Benzyloxycarbonyl-32-Polyamin auf der Basis des aus N,N',N",N'''-Tetrakis{8-Benzyloxycarbonylamino)-6-[2-(benzyloxycarbonylamino)-ethyl]-5-oxo-3-oxaoctanoyl}cyclen mit N^{α},N^{ε}-bis(lysyl)-Lysin("Tri-Lysin") kondensierten 32-Amins

2,05 g (1 mmol) des im Beispiel 3b) beschriebenen Okta-Benzyloxycarbonylamins werden in Eisessig gelöst und unter Rühren mit 33 igem Bromwasserstoff in Eisessig versetzt. Nach 90 Minuten wird mit Diethylether die begonnende Fällung vervollständigt, das entstandene Okta-amin-hydrobromid mit Ether gewaschen, im Vakuum getrocknet und ohne weitere Reinigung in die weiter unten beschriebene Reaktion eingesetzt.
Ausbeute: 1,6 g (quantitativ)

9,4 g (10 mmol) des in Beispiel 1c) beschriebenen geschützten "Tri-Lysins", 1,5 g (10 mmol) 1-Hydroxybenzotriazol und 3,2 g (10 mmol) 2-(1H-Benzotriazol-1-yl)-1,1,3,3-tetramethyluronium tetrafluorborat (TBTU; Peboc Limited, UK) werden in DMF gelöst und 15 Minuten gerührt. Diese Lösung wird anschließend mit 5,16 ml (30 mmol) N-Ethyldiisopropylamin und mit 1,6 g (1 mmol) des oben beschriebenen Oktaaminhydrobromids versetzt und über Nacht bei Raumtemperatur gerührt. Nach Beendigung der Reaktion wird im Vakuum eingedampft und der Rückstand mit Dichlormethan/Methanol (10:1) an Kieselgel chromatographiert.
Ausbeute: 6,0 g (72 % d. Th.)

| Elementaranalyse: | | | |
|---|---|---|---|
| ber. | C 63,32 | H 6,76 | N 10,74 |
| gef. | C 62,98 | H 6,91 | N 10,43 |

### d) 32-mer N-(5-DO3A-yl-4-oxo-3-azahexanoyl)-Kaskadenpolyamid auf der Basis des im vorstehenden Beispiel 3c) beschriebenen 32-mer Amins

8,35 g (1 mmol) des in Beispiel 3c) beschriebenen 32-mer-Benzyloxycarbonylamins werden in Eisessig gelöst und unter Rühren mit 33 %igem Bromwasserstoff in Eisessig versetzt. Nach 3 Stunden wird mit Diethylether die begonnene Fällung vervollständigt, das entstandene 32-Amin-hydrobromid mit Ether gewaschen und im Vakuum getrocknet.

47,8 g (64 mmol) der im Beispiel 1h) beschriebenen Säure werden in DMF gelöst, mit 9,8 g (64 mmol) 1-Hydroxybenzotriazol, mit 20,5 g (64 mmol) TBTU (Peboc Limited, UK) und mit 65,7 ml (384 mmol) N-Ethyldiisopropylamin versetzt und 20 Minuten bei Raumtemperatur gerührt. Diese Lösung wird anschließend mit den oben beschriebenen (1 mmol) 32-Aminhydrobromid versetzt und 4 Tage bei Raumtemperatur gerührt. Die Lösung wird im Vakuum eingeengt, das verbleibende Öl im Eisbad gekühlt und mit Trifluoressigsäure versetzt, über Nacht bei Raumtemperatur gerührt und anschließend mit Diethylether gefällt. Der Niederschlag wird im Vakuum getrocknet, in Wasser aufgenommen, auf pH 7 eingestellt, über eine YM3 Amicon® -Ultrafiltrationsmembran von niedermolekularen Anteilen gereinigt und das Retentat schließlich membranfiltriert und gefriergetrocknet. Ausbeute: 17,2 g (76,4 % d.Th.)
H₂O-Gehalt (Karl-Fischer): 7,6 %

| Elementaranalyse (bezogen auf wasserfreie Substanz): | | | | |
|---|---|---|---|---|
| ber. | C 45,73 | H 6,12 | N 15,08 | Na 10,61 |
| gef. | C 45,89 | H 6,30 | N 14,84 | Na 10,31 |

### e) 32-mer-Gd-Komplex des N-(5-DO3A-yl-4-oxo-3-azahexanoyl)-Kaskadenpolyamids auf der Basis in Beispiel 3c) beschriebenen 32-mer Amins

10,4 g (0,5 mmol) der im vorstehenden Beispiel 3d) beschriebenen Komplexbildnersäure werden in Wasser mit verd. Salzsäure auf pH 3 gestellt, mit 2,89 g (8 mmol) Gd₂O₃ versetzt, 30 Minuten bei 80 °C gerührt, nach dem Abkühlen auf pH 7 eingestellt und über eine YM3 AMICON®-Ultrafiltrationsmembran entsalzt. Das Retentat wird schließlich membranfiltriert und gefriergetrocknet.
Ausbeute: 12,1 g (91,1 % d. Th.)
H₂O-Gehalt (Karl-Fischer): 11,0 %
Gd-Bestimmung (AAS): 18,6 %

| Elementaranalyse (bezogen auf wasserfreie Substanz): | | | | |
|---|---|---|---|---|
| ber. | C 40,26 | H 5,39 | N 13,28 | Gd 21,30 |
| gef. | C 40,10 | H 5,21 | N 13,04 | Gd 21,03 |

In analoger Weise erhält man mit Yb₂(CO₃)₃ den Ytterbium-Komplex:

| Elementaranalyse (bezogen auf wasserfreie Substanz): | | | | |
|---|---|---|---|---|
| ber. | C 39,42 | H 5,28 | N 13,00 | Yb 22,94 |
| gef. | C 39,29 | H 5,40 | N 12,81 | Yb 22,65 |

### Beispiel 4

### a) Hexaethylenglycolmonomethylether-p-toluolsulfonsäureester

Zu 20 g (67,49 mmol) Hexaethylenglycolmonomethylether und 7,59 g (75 mmol) Triethylamin in 200 ml Chloroform gibt man bei 0 °C portionsweise 14,3 g (75 mmol) p-Toluolsulfonsäurechlorid zu und rührt anschließend 4 Stunden bei dieser Temperatur. Man dampft im Vakuum zur Trockne ein und chromatographiert den Rückstand an Kieselgel (Laufmittel: Chloroform/Methanol= 5/1).
Ausbeute: 27,67 g (91 % d. Th.) eines schuppenartigen, glasigen Feststoffes

| Elementaranalyse: | | | |
|---|---|---|---|
| ber. | C 53,32 | H 7,61 | S 7,12 |
| gef. | C 53,15 | H 7,70 | S 7,03 |

### b) 1-Benzyloxy-5-(benzyloxycarbonyl)-2-chloro-3-oxo-4-azapentan

Zu 100 g (296,4 mmol) Glycinbenzylester-p-Toluolsulfonsäuresalz und 33,0 g (326,1 mmol) Triethylamin in 400 ml Methylenchlorid tropft man bei 0 °C 76 g (326,1 mmol) 2-Chlor-3-(benzyloxy)-propionsäurechlorid (hergestellt nach Inorg. Chem. Vol. 31; 2422, 1992) zu und rührt 2 Stunden bei dieser Temperatur. Man setzt 500 ml Eiswasser zu und stellt die Wasserphase mit 10 % aqu. Salzsäure auf pH 2. Die organische Phase wird abgetrennt, je einmal mit 300 ml 5 % aqu. Salzsäure, 300 ml 5 % aqu. Sodalösung und 400 ml Wasser gewaschen. Man trocknet die organische Phase über Magnesiumsulfat und dampft im Vakuum zur Trockne ein. Der Rückstand wird an Kieselgel chromatographiert (Laufmittel: Methylenchlorid/Hexan/Aceton= 15/5/1).
Ausbeute: 75,07 g (70 % d. Th.) eines schwach gelbgefärbten zähen Öls

| Elementaranalyse: | | | | |
|---|---|---|---|---|
| ber. | C 63,07 | H 5,57 | N 3,87 | Cl 9,80 |
| gef. | C 63,17 | H 5,65 | N 3,75 | Cl 9,63 |

### c) 1-[4-(Benzyloxycarbonyl)-1-(benzyloxymethyl)-2-oxo-3-azabutyl]-1,4,7,10-tetraazacyclododecan

70 g (193,5 mmol) der Titelverbindung aus Beispiel 4b) und 11,1 g (64,5 mmol) 1,4,7,10-Tetraazacyclododecan werden in 70 ml Dimethylformamid gelöst und 2 Tage bei 50 °C gerührt. Man dampft im Vakuum zur Trockne ein, nimmt den Rückstand in 700 ml Wasser auf und extrahiert 2 mal mit je 250 ml Chloroform. Die organische Phase wird über Magnesiumsulfat getrocknet und im Vakuum zur Trockne eingedampft. Der Rückstand wird an Kieselgel chromatographiert (Laufmittel: Chloroform/Methanol/25 % aqu. Ammoniak = 10/5/1).
Ausbeute: 13,16 g (41 % d. Th.bezogen auf Cyclen) eines zähen, farblosen Öls

| Elementaranalyse: | | | |
|---|---|---|---|
| ber. | C 65,17 | H 7,90 | N 14,07 |
| gef. | C 65,24 | H 7,77 | N 14,18 |

### d) 10-[4-(Benzyloxycarbonyl)-1-(benzyloxymethyl)-2-oxo-3-azabutyl]-1,4,7-tris(tert.butoxycarbonylmethyl)-1,4,7,10-tetraazacyclododecan (Natriumbromid-Komplex)

Zu 13 g (26,12 mmol) der Titelverbindung aus Beispiel 4c) und 9,14 g (86,2 mmol) Natriumcarbonat in 200 ml Acetonitril gibt man 16,81 g (86,2 mmol) Bromessigsäure-tert.butylester zu und rührt 24 Stunden bei 60 °C. Man kühlt auf 0 °C ab, filtriert von den Salzen ab und dampft das Filtrat zur Trockne ein. Der Rückstand wird an Kieselgel chromatographiert (Laufmittel: Essigsäureethylester/Ethanol = 15/1).
Ausbeute: 19,46 g (79 % d. Th.) eines wachsartigen Feststoffes

| Elementaranalyse: | | | | | |
|---|---|---|---|---|---|
| ber. | C 57,32 | H 7,38 | N 7,43 | Na 2,43 | Br 8,47 |
| gef. | C 57,22 | H 7,51 | N 7,27 | Na 2,33 | Br 8,29 |

### e) 10-[4-carboxy-2-oxo-1-hydroxymethyl-3-azabutyl]-1,4,7-tris(tert.-butoxycarbonylmethyl)-1,4,7,10-tetraazacyclododecan (Natriumbromid-Komplex)

Zu 19 g (20,15 mmol) der Titelverbindung aus Beispiel 4d) in 300 ml Isopropanol gibt man 3 g Palladiumkatalysator (10 % Pd/C) und hydriert über Nacht bei Raumtemperatur. Man filtriert vom Katalysator ab, dampft das Filtrat im Vakuum zur Trockne ein und kristallisiert den Rückstand aus Aceton um.
Ausbeute: 13,06 g (85 % d. Th.) eines farblosen, kristallinen Pulvers

| Elementaranalyse: | | | | | |
|---|---|---|---|---|---|
| ber. | C 48,82 | H 7,53 | N 9,18 | Na 3,00 | Br 10,49 |
| gef. | C 48,71 | H 7,68 | N 9,03 | Na 2,81 | Br 10,23 |

### f) 10[4-(Benzyloxycarbonyl)-1-(hydroxymethyl)-2-oxo-3-azabutyl]-1,4,7-tris(tert.-butoxycarbonylmethyl)-1,4,7,10-tetraazacyclododecan

Zu 13 g (17,04 mmol) der Titelverbindung aus Beispiel 4e) und 6,11 g (18,75 mmol) wasserfreiem Cäsiumcarbonat in 70 ml Dimethylformamid gibt man 3,42 g (20 mmol) Benzylbromid und rührt über Nacht bei 50 °C. Man kühlt auf 0 °C ab und gibt 700 ml Wasser zu. Anschließend wird 2 mal mit je 300 ml Methylenchlorid extrahiert. Die vereinigten organischen Phasen werden 2 mal mit Wasser gewaschen, über Magnesiumsulfat getrocknet und im Vakuum zur Trockne eingedampft. Der Rückstand wird an Kieselgel chromatographiert (Laufmittel: Essigsäureethylester/Ethanol)
Ausbeute: 9,97g (78 % d. Th.) eines farblosen, zähen Öls

| Elementaranalyse: | | | |
|---|---|---|---|
| ber. | C 60,86 | H 8,47 | N 9,34 |
| gef. | C 60,95 | H 8,61 | N 9,21 |

### g) 10-[4-(Benzyloxycarbonyl)-1-(2,5,8,11,14,17,20-heptaoxa-heneicosanoyl)-2-oxo-3-azabutyl]-1,4,7-tris(tert.-butoxycarbonylmethyl)-1,4,7,10-tetraazacyclododecan

9,7 g (12,93 mmol) der Titelverbindung aus Beispiel 4f) werden in 50 ml THF gelöst und bei -10 °C 0,43 g (14,22 mmol) Natriumhydrid (80 % in Paraffin) zugegeben. Man rührt 30 Minuten bei 0 °C. Dann werden 11,65 g (25,86 mmol) der Titelverbindung aus Beispiel 4a) und 3,46 g (25,86 mmol) Lithiumjodid zugegeben. Man rührt 24 Stunden bei Raumtemperatur. Es werden vorsichtig 3 ml Wasser zugegeben und anschließend zur Trockne eingedampft. Der Rückstand wird an Kieselgel chromatographiert (Laufmittel:
Chloroform/Methanol= 10:1).
Ausbeute: 12,1 g (91 % d. Th.) eines glasigen Feststoffes

| Elementaranalyse: | | | |
|---|---|---|---|
| ber. | C 59,57 | H 8,72 | N 6,81 |
| gef. | C 59,65 | H 8,91 | N 6,62 |

### h) 10-[1-(2-8,11,14,17,20-Heptaoxa-heneicosanoyl)-2-oxo-3-aza-4-(carboxy)-butyl]-1,4,7-tris(tert.-butoxycarbonylmethyl)-1,4,7,10-tetraazacyclododecan

12 g (11,67 mmol) der Titelverbindung aus Beispiel 4g) werden in 300 ml Isopropanol gelöst und 2 g Palladiumkatalysator (10 % Pd/C) zugegeben. Man hydriert über Nacht bei Raumtemperatur. Es wird vom Katalysator abfiltriert und das Filtrat zur Trockne eingedampft. Der Rückstand wird aus Aceton/Diisopropylether umkristallisiert.
Ausbeute: 10,18 g (93 % d. Th.) eines wachsartigen Feststoffes

| Elementaranalyse: | | | |
|---|---|---|---|
| ber. | C 56,33 | H 8,92 | N 7,46 |
| gef. | C 56,20 | H 9,03 | N 7,35 |

### i) 24-mer Gd-Komplex des N-(5-DO3A-yl-4-oxo-3-aza-7,10,13,16,19,22,25-heptaoxahexacosanoyl)-Kaskadenpolyamids auf der Basis des N,N,N',N',N",N"-Hexakis[2-(trilysylamino)-ethyl]-trimesinsäuretriamids

6,0 g (1 mmol) des in Beispiel 1d) beschriebenen 24-mer-Benzyloxycarbonylamins werden in Eisessig gelöst und unter Rühren mit 33 %igem Bromwasserstoff in Eisessig versetzt. Nach 3 Stunden wird mit Diethylether die begonnene Fällung vervollständigt, das entstandene 24-Amin-hydrobromid mit Ether gewaschen und im Vakuum getrocknet. 45,03 g (48 mmol) der im vorstehenden Beispiel 4h) beschriebenen Säure werden in DMF gelöst, mit 7,35 g (48 mmol) 1-Hydroxybenzotriazol, mit 15,41 g (48 mmol) TBTU (Peboc Limited, UK) und mit 49,3 ml (288 mmol) N-Ethyldiisopropylamin versetzt und 20 Minuten bei Raumtemperatur gerührt. Diese Lösung wird anschließend mit den oben beschriebenen (1 mmol) 24-Aminhydrobromid versetzt und 4 Tage bei Raumtemperatur gerührt. Die Lösung wird im Vakuum eingeengt, das verbleibende Öl im Eisbad gekühlt und mit Trifluoressigsäure versetzt, über Nacht bei Raumtemperatur gerührt und anschließend mit Diethylether gefällt. Der Niederschlag wird im Vakuum getrocknet, in Wasser aufgenommen, mit verd. Salzsäure auf pH 3 gestellt, mit 8,70 g (24 mmol) Gd₂O₃ versetzt, 4 Stunden bei 80 °C gerührt, nach dem Abkühlen auf pH 7 eingestellt und über eine YM3 AMICON®-Ultrafiltrationsmembran von niedermolekularen Anteilen gereinigt und das Retentat schließlich membranfiltriert und gefriergetrocknet.
Ausbeute: 19,6 g (73,3 % d. Th.)
H₂O-Gehalt (Karl-Fischer): 8,3 %
Gd-Bestimmung (AAS): 14,0 %

| Elementaranalyse (bezogen auf wasserfreie Substanz): | | | | |
|---|---|---|---|---|
| ber. | C 43,94 | H 6,38 | N 9,43 | Gd 15,39 |
| gef. | C 44,27 | H 6,22 | N 9,29 | Gd 15,09 |

### Beispiel 5

### a) 1,7-Bis(trifluoracetyl)-1,4,7-triazaheptan

In eine Lösung aus 41,14 g (390 mmol) 1,4,7-Triazaheptan in 350 ml Tetrahydrofuran werden bei 80 °C und unter Stickstoff 113,3 g (790 mmol) Trifluoressigsäureethylester zugetropft. Man läßt über Nacht bei Raumtemperatur rühren, engt im Vakuum ein. Das verbleibende Öl wird aus Hexan kristallisiert.
Ausbeute: 115 g (99,9 % d. Th.)
Schmelzpunkt: 68-70 °C

| Elementaranalyse: | | | | |
|---|---|---|---|---|
| ber. | C 32,55 | H 3,76 | F 38,62 | N 14,24 |
| gef. | C 32,63 | H 3,75 | F 38,38 | N 14,19 |

### b) 1,7-Bis(trifluoracetyl)-4-benzyloxycarbonyl-1,4,7-triazaheptan

In 120 ml Dichlormethan werden 14,75 g (50 mmol) der unter Beispiel 5a) hergestellten Trifluoracetylverbindung sowie 8,3 ml (60 mmol) Triethylamin gelöst und auf 0 °C gekühlt. Unter Rühren werden nun 7,5 ml (53 mmol) Chlorameisensäurebenzylester (97 %), gelöst in 20 ml Dichlormethan, zugetropft. Man läßt über Nacht bei Raumtemperatur rühren, extrahiert die Salze mit destilliertem Wasser, trocknet die Dichlormethanlösung über Natriumsulfat, engt im Vakuum zur Trockne ein und kristallisiert den Rückstand aus Ether/Hexan.
Ausbeute: 18,40 g (85,7 % d.Th.)
Schmelzpunkt: 131-32 °C

| Elementaranalyse: | | | | |
|---|---|---|---|---|
| ber. | C 44,76 | H 3,99 | F 26,55 | N 9,79 |
| gef. | C 44,87 | H 4,03 | F 26,62 | N 9,61 |

### c) 3,9-Bis(tert.-butoxycarbonylmethyl)-6-benzyloxycarbonyl-3,6,9-triazaundecandicarbonsäure-di-tert.-butylester

In 30 ml Ethanol werden 4,29 g (10 mmol) des unter Beispiel 5b) hergestellten Trifluoracetylderivates gelöst und mit 800 mg (20 mmol) Natronlauge in 10 ml destilliertem Wasser versetzt. Man rührt 3 Stunden bei Raumtemperatur, engt bei 40 °C Badtemperatur im Vakuum zur Trockne ein, entfernt Wasserreste durch azeotrope Destillation mit Isopropanol und nimmt in 30 ml Dimethylformamid auf. Dann gibt man 6,9 g (50 mmol) Kaliumcarbonat sowie 9,7 g (50 mmol) Bromessigsäure-tert.-butylester dazu und alkyliert das 4-Benzyloxycarbonyl-1,4,7-triazaheptan bei Raumtemperatur über Nacht. Man zieht dann das Dimethylformamid im Ölpumpenvakuum ab, verteilt den Rückstand zwischen Wasser und Dichlormethan, trocknet die organische Lösung über Natriumsulfat, engt im Vakuum zur Trockne ein und reinigt den Rückstand durch Chromatographie an Kieselgel. Die Titelverbindung wird mit Essigester/Hexan eluiert. Sie wird als Schaum erhalten.
Ausbeute: 6,49 g (93,6 % d. Th.)

| Elementaranalyse: | | | |
|---|---|---|---|
| ber. | C 62,32 | H 8,57 | N 6,06 |
| gef. | C 62,41 | H 8,66 | N 6,01 |

### d) 3,9-Bis(tert.-butoxycarbonylmethyl)-3,6,9-triazaundecandicarbonsäure-di-tert.-butylester

In 100 ml Ethanol werden 3,5 g (5 mmol) der unter Beispiel 5c) hergestellten Verbindung gelöst, mit 200 mg Pearlman-Katalysator(Pd 20 % auf Aktivkohle) versetzt und bis zur Aufnahme der berechneten Menge Wasserstoff hydriert. Man saugt vom Katalysator ab und engt im Vakuum zur Trockne ein. Die Titelverbindung wird als weißer Schaum erhalten. Ausbeute: 2,80 g (99,9 % d.Th.)

| Elementaranalyse: | | | |
|---|---|---|---|
| ber. | C 60,08 | H 9,54 | N 7,51 |
| gef. | C 60,02 | H 9,62 | N 7,56 |

### e) 3,9-Bis(tert.-butoxycarbonylmethyl)-6-[1-(ethoxycarbonyl)-ethyl]-3,6,9-triazaundecandisäure-di-tert.-butylester

In 30 ml Dimethylformamid werden 5,60 g (10 mmol) der unter Beispiel 5d) hergestellten Aminoverbindung gelöst. Dann gibt man bei Raumtemperatur 1,66 g (12 mmol) Kaliumcarbonat sowie 2,17 g (12 mmol) 2-Brompropionsäureethylester dazu und rührt über Nacht. Man gießt dann auf Eiswasser, extrahiert mit Essigester, trocknet die organische Lösung über Natriumsulfat, engt im Vakuum zur Trockne ein und gewinnt die Titelverbindung durch Chromatographie an Kieselgel. Als Elutionsmittel dient ein Gemisch aus Essigester/Hexan.
Ausbeute: 4,18 g (63,4 % d.Th.)

| Elementaranalyse: | | | |
|---|---|---|---|
| ber. | C 60,07 | H 9,32 | N 6,37 |
| gef. | C 60,18 | H 9,40 | N 6,31 |

### f) 3,9-Bis(tert.-butoxycarbonylmethyl)-6-[1-(carboxy)-ethyl]-3,6,9-triazaundecandisäuredi-tert.-butylester

In 50 ml Ethanol werden 6,60 g (10 mmol) der unter Beispiel 5e) hergestellten Verbindung gelöst. Man gibt dann die Lösung von 400 mg (10 mmol) Natriumhydroxid in 5 ml destilliertem Wasser dazu und rührt 3 Stunden bei 50 °C. Nach dem Dünnschichtchromatogramm ist die Verseifung quantitativ . Man engt im Vakuum zur Trockne ein, entfernt Spuren von Wasser durch Kodestillation mit Ethanol und trocknet den Rückstand bei 40 °C im Vakuum. Die Titelverbindung wird als weißes Pulver erhalten. Der verbliebene weiße Rückstand wird in 80 ml feuchtem Ethanol (9:1) gelöst und unter Rühren mit der Lösung von 535 mg (10 mmol) Ammonchlorid in 10 ml destilliertem Wasser versetzt. Man engt im Vakuum zur Trockne ein, nimmt die löslichen Anteile in Butanol auf und engt nochmals im Vakuum zur Trockne ein. Der Rückstand wird mit Toluol extrahiert. Man engt die organische Lösung im Vakuum zur Trockne ein und erhält die Titelverbindung als Schaum.
Ausbeute: 5,35 g (84,7 % d. Th.)

| Elementaranalyse: | | | |
|---|---|---|---|
| ber. | C 58,93 | H 9,09 | N 6,65 |
| gef. | C 59,01 | H 9,16 | N 6,60 |

### g) 24-mer N-{N,N-Bis[2-(N,N-Bis(carboxymethyl))-aminoethyl]-alanyl}-Kaskadenpolyamid auf der Basis des N,N,N',N',N",N"-Hexakis[2-(trilysylamino)-ethyl]-trimesinsäuretriamids, Natriumsalz

6,0 g (1 mmol) des in Beispiel 1d) beschriebenen Poly-Benzyloxycarbonylamins werden in Eisessig gelöst und unter Rühren mit 33 %igem Bromwasserstoff in Eisessig versetzt. Nach 3 Stunden wird mit Diethylether die begonnene Fällung vervollständigt, das entstandene 24-Amin-hydrobromid mit Ether gewaschen und im Vakuum getrocknet.

30,33 g (48 mmol) der im vorstehenden Beispiel 5f) beschriebenen Säure werden in DMF gelöst, mit 7,35 g (48 mmol) 1-Hydroxybenzotriazol, mit 15,41 g (48 mmol) TBTU (Peboc Limited, UK) und mit 49,3 ml (288 mmol) N-Ethyldiisopropylamin versetzt und 20 Minuten bei Raumtemperatur gerührt. Diese Lösung wird anschließend mit den oben beschriebenen (1 mmol) 24-Aminhydrobromid versetzt und 4 Tage bei Raumtemperatur gerührt. Die Lösung wird im Vakuum eingeengt, das verbleibende Öl im Eisbad gekühlt und mit Trifluoressigsäure versetzt, über Nacht bei Raumtemperatur gerührt und anschließend mit Diethylether gefällt. Der Niederschlag wird im Vakuum getrocknet, in Wasser aufgenommen, auf pH 7 eingestellt, über eine YM3 Amicon®-Ultrafiltrationsmembran von niedermolekularen Anteilen gereinigt und das Retentat schließlich membranfiltriert und gefriergetrocknet.
Ausbeute: 11,0 g (86,3 % d.Th.)
H₂O-Gehalt (Karl-Fischer): 8,2 %

| Elementaranalyse (bezogen auf wasserfreie Substanz): | | | | |
|---|---|---|---|---|
| ber. | C 42,87 | H 5,41 | N 11,96 | Na 12,08 |
| gef. | C 42,78 | H 5,66 | N 12,11 | Na 11,89 |

### h) 24-mer-Gd-Komplex des N-{N,N-Bis[2-(N,N-Bis(carboxymethyl))-aminoethyl]-alanyl}-Kaskadenpolyamids auf der Basis des N,N,N',N',N",N"-Hexakis[2-(trilysylamino)-ethyl]-trimesinsäuretriamids, Natriumsalz

8,13 g (0,5 mmol) der im vorstehenden Beispiel 5g) beschriebenen Komplexbildnersäure werden in Wasser mit verd. Salzsäure auf pH 3 gestellt, mit 2,17 g (6 mmol) Gd₂O₃ versetzt, 30 Minuten bei 80 °C gerührt, nach dem Abkühlen auf pH 7 eingestellt und über eine YM3 AMICON®-Ultrafiltrationsmembran entsalzt. Das Retentat wird schließlich membranfiltriert und gefriergetrocknet.
Ausbeute: 8,0 g (90,5 % d. Th.)
H₂O-Gehalt (Karl-Fischer): 7,5 %
Gd-Bestimmung (AAS): 21,0 %

| Elementaranalyse (bezogen auf wasserfreie Substanz): | | | | | |
|---|---|---|---|---|---|
| ber. | C 35,93 | H 4,38 | N 10,03 | Gd 23,09 | Na 3,38 |
| gef. | C 35,71 | H 4,65 | N 9,88 | Gd 22,84 | Na 3,50 |

### Beispiel 6

### a) 3,9-Bis(tert.-butoxycarbonylmethyl)-6-benzyloxycarbonylmethyl-3,6,9-triazaundecandisäure-di-tert.-butylester

In 30 ml Dimethylformamid werden 5,60 g (10 mmol) der unter Beispiel 5d) hergestellten Aminoverbindung gelöst. Dann gibt man bei Raumtemperatur 1,66 g (12 mmol) Kaliumcarbonat sowie 2,58 g (12 mmol) Bromessigsäurebenzylester dazu und rührt über Nacht. Man gießt dann auf Eiswasser, extrahiert mit Essigester, trocknet die organische Lösung über Natriumsulfat, engt im Vakuum zur Trockne ein und gewinnt die Titelverbindung durch Chromatographie an Kieselgel. Als Elutionsmittel dient ein Gemisch aus Essigester/Hexan.
Ausbeute: 6,32 g (89,3 % d. Th.)

| Elementaranalyse: | | | |
|---|---|---|---|
| ber. | C 64,65 | H 9,00 | N 5,95 |
| gef. | C 64,62 | H 9,07 | N 5,90 |

### b) 3,9-Bis(tert.-butoxycarbonylmethyl)-6-carboxymethyl-3,6,9-triazaundecandisäure-ditert.-butylester

In 100 ml Ethanol werden 7,08 g (10 mmol) des unter 6a) hergestellten Benzylesters gelöst und mit 0,4 g Pearlman-Katalysator (Pd 20 %, C) versetzt. Man hydriert bis zur Aufnahme von 224 ml Wasserstoff, saugt vom Katalysator ab, wäscht gut mit Ethanol nach und engt die Lösung im Vakuum zur Trockne ein. Das Produkt wird als Schaum erhalten, der aus Ether/Hexan kristallisiert.
Ausbeute: 6,87 g (97,3 % d. Th.)
Schmelzpunkt: 73-75 °C

| Elementaranalyse: | | | |
|---|---|---|---|
| ber. | C 57,85 | H 9,00 | N 5,95 |
| gef. | C 57,91 | H 9,11 | N 6,01 |

### c) 32-mer N-{N,N-Bis[2-(N,N-Bis(carboxymethyl))-aminoethyl]-glycyl}-Kaskadenpolyamid auf der Basis des im Beispiel 3c) beschriebenen 32-mer Amins, Natriumsalz

8,35 g (1 mmol) des in Beispiel 3c) beschriebenen 32-mer-Benzyloxycarbonylamins werden in Eisessig gelöst und unter Rühren mit 33 %igem Bromwasserstoff in Eisessig versetzt. Nach 3 Stunden wird mit Diethylether die begonnene Fällung vervollständigt, das entstandene 32-Amin-hydrobromid mit Ether gewaschen und im Vakuum getrocknet.

39,5 g (64 mmol) der im vorstehenden Beispiel 6b) beschriebenen Säure werden in DMF gelöst, mit 9,8 g (64 mmol) 1-Hydroxybenzotriazol, mit 20,5 g (64 mmol) TBTU (Peboc Limited, UK) und mit 65,7 ml (384 mmol) N-Ethyldiisopropylamin versetzt und 20 Minuten bei Raumtemperatur gerührt. Diese Lösung wird anschließend mit den oben beschriebenen (1 mmol) 32-Aminhydrobromid versetzt und 4 Tage bei Raumtemperatur gerührt. Die Lösung wird im Vakuum eingeengt, das verbleibende Öl im Eisbad gekühlt und mit Trifluoressigsäure versetzt, über Nacht bei Raumtemperatur gerührt und anschließend mit Diethylether gefällt. Der Niederschlag wird im Vakuum getrocknet, in Wasser aufgenommen, auf pH 7 eingestellt, über eine YM3 Amicon®-Ultrafiltrationsmembran von niedermolekularen Anteilen gereinigt und das Retentat schließlich membranfiltriert und gefriergetrocknet.
Ausbeute: 15,7 g (78,6 % d.Th.)
H₂O-Gehalt (Karl-Fischer): 9,0 %

| Elementaranalyse (bezogen auf wasserfreie Substanz): | | | | |
|---|---|---|---|---|
| ber. | C 41,77 | H 5,24 | N 12,33 | Na 12,14 |
| gef. | C 41,49 | H 5,36 | N 12,49 | Na 11,93 |

### d) 32-mer-Gd-Komplex des N-{N,N-Bis[2-(N,N-Bis(carboxymethyl))-aminoethyl]-glycyl}-Kaskadenpolyamids auf der Basis in Beispiel 3c) beschriebenen 32-mer Amins, Natriumsalz

10,0 g (0,5 mmol) der im vorstehenden Beispiel 6c) beschriebenen Komplexbildnersäure werden in Wasser mit verd. Salzsäure auf pH 3 gestellt, mit 2,89 g (8 mmol) Gd₂O₃ versetzt, 30 Minuten bei 80 °C gerührt, nach dem Abkühlen auf pH 7 eingestellt und über eine YM3 AMICON®-Ultrafiltrationsmembran entsalzt. Das Retentat wird schließlich membranfiltriert und gefriergetrocknet.
Ausbeute: 10,9 g (90,9 % d. Th.)
H₂O-Gehalt (Karl-Fischer): 9,5 %
Gd-Bestimmung (AAS): 20,9 %

| Elementaranalyse (bezogen auf wasserfreie Substanz): | | | | | |
|---|---|---|---|---|---|
| ber. | C 34,98 | H 4,24 | N 10,33 | Gd 23,19 | Na 3,39 |
| gef. | C 35,20 | H 4,08 | N 10,46 | Gd 22,89 | Na 3,60 |

### Beispiel für einen in-vivo Vergleich mit einem extrazellulären Kontrastmittel

Die Eignung der im Beispiel 1k) beschriebenen Verbindung als blood-pool-agent wird im folgenden Versuch gezeigt.

Als Versuchstiere dienen fünf 300-350 g schwere männliche (Schering-SPF-)Ratten. Vor dem Versuch wird das Abdomen eröffnet, der Darm verlagert und dann durch das hintere Bauchfell hindurch mit einer chirurgischen Nadel die Nierengefäße (arteriell+venös) beider Seiten abgebunden. Anschließend wird die Bauchhöhle wieder verschlossen. Danach werden je Tier 0.3 ml (jeweils 50 mmol/L) der folgenden Kontrastmittel-Lösung intravenös appliziert: Gemisch aus je 1 Teil der Verbindung aus Beispiel 1k), im folgenden Verbindung 1 genannt, und dem Dysprosium-Komplex des 10-(1-Hydroxymethyl-2,3-dihydroxypropyl)-1,4,7-tris(carboxymethyl)-1,4,7,10-tetraazacyclododecans, hergestellt analog der Vorschrift in der Europ. Patentanmeldung EP 448 191, im folgenden Verbindung 2 genannt. Über einen Katheter in der Arteria carotis communis werden Blutproben zu folgenden Zeitpunkten entnommen: 15, 30, 45, 60, 90 sec, 3, 5, 10, 15 min p.i. In den gewonnenen Blutproben werden jeweils parallel die Konzentrationen an Gadolinium (Gd) und Dysprosium (Dy) mittels Atomemissionsspektrometrie (ICP-AES) gemessen. Der im Blutraum verbliebene Anteil der injizierten Kontrastmittel Verbindung 1 (Gd) und Verbindung 2 (Dy, Vergleichssubstanz) kann durch die unterschiedliche Markierung im gleichen Tier verglichen werden. Da eine renale Ausscheidung nicht möglich ist, kann der Abfall der Blutkonzentration nur auf eine Verteilung in den Bluträumen und auf die Diffusion in das interstitielle Gewebe zurückzuführen sein.

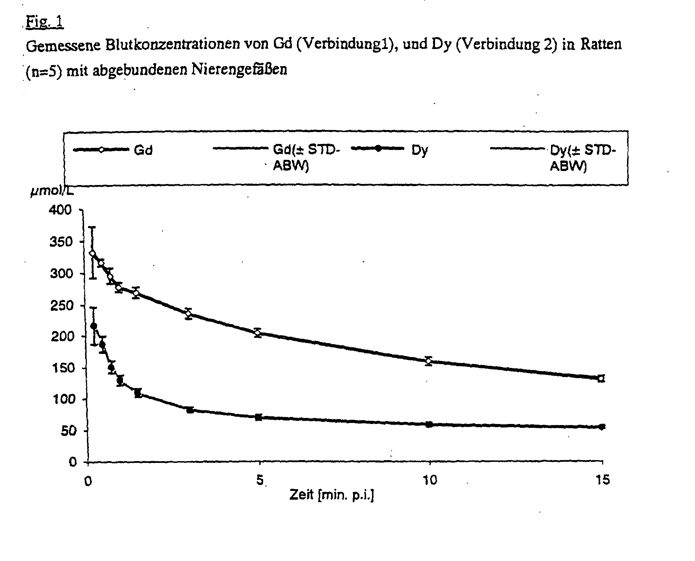

Ergebnisse: Die Diffusion von Verbindung 1 in das Interstitium ist im Vergleich zu einem extrazellulären Kontrastmittel Verbindung 2 deutlich verlangsamt (siehe Figur 1).

Das extrazelluläre Kontrastmittel (Verbindung 2) diffundiert so schnell in die interstitiellen Räume des Körpers, daß bereits nach 3-5 Minuten p.i. ein Equilibrium erreicht wird (angezeigt durch konstanten Blutspiegel). Im Gegensatz dazu werden beim Kaskadenpolymer (Verbindung 1) nicht nur stets höhere Blutkonzentrationen gemessen (Hinweis auf kleineres Verteilungsvolumen), sondern es wird auch über den gesamten Untersuchungszeitraum von 15 Minuten noch kein Equilibrium erreicht (Hinweis auf nur sehr langsam verlaufende Diffusion ins interstitielle Gewebe). Das bedeutet, daß sich Verbindung 1 als Blutpool-Kontrastmittel verhält.

### Beispiel für eine MR Angiographie am Kaninchen

Die unter Beispiel 1k genannte Verbindung wurde an Kaninchen (CH.R. Kisslegg, ≈ 4 kg Körpergewicht) in einem MR Angiographie-Experiment untersucht (Ganzkörper MRT System Siemens Vision. 1.5 Tesla, FISP 3D; TR: 400 ms; TE 15 ms; flip angle: 45°; coronal).

In der pre contrast Aufnahme (s. Foto) sind nur ein bis zwei größere Gefäße sichtbar (z.B. Aorta abdominalis) bei relativ schwachem Kontrast (Signalintensität SI dieser Gefäße zum background). Nach i.v. Applikation von 50 µmol Gd/kg Körpergewicht der im Beispiel 1k beschriebenen Verbindung sieht man eine deutliche Zunahme des Kontrasts (SI der Gefäße/SI des backgrounds) und eine Vielzahl kleiner und kleinster Blutgefäße (z.B. A. und V. femoralis, A. und V. mesenterica caudalis, A. und V. renalis, A. und V. subrenalis etc), die vor Kontrastmittelgabe nicht detektierbar waren.

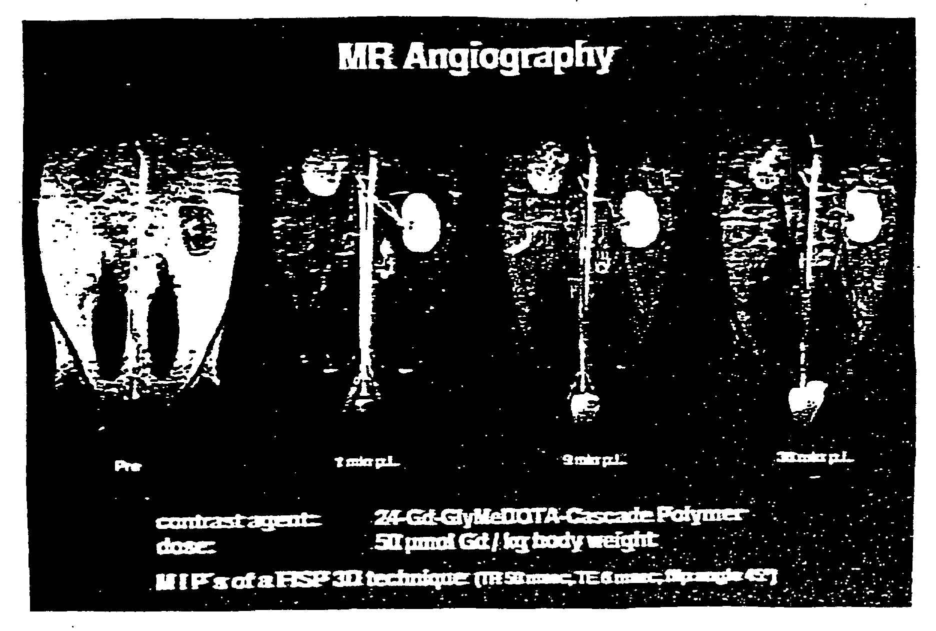

### Beispiel für eine Lymphknotenanreicherung am Meerschweinchen

Die unter Beispiel 1k genannte erfindungsgemäße Verbindung wurde 30 min bis 24 h nach subkutaner Gabe (10 µmol Gadolinium/kg Körpergewicht, Hinterpfote s.c.) an stimulierten Meerschweinchen (komplettes Freund-Adjuvant; jeweils 0,1 ml i.m. in den rechten und linken Ober- und Unterschenkel; 2 Wochen vor Gabe der Prüfsubstanzen) hinsichtlich ihrer Lymphknotenanreicherung in drei aufeinanderfolgenden Lymphknotenstationen (popliteal, inguinal, iliakal) untersucht. Hierbei wurden die nachfolgend aufgelisteten Ergebnisse (Ermittlung der Gadolinium-Konzentration mittels ICP-AES) erhalten:

| **Zeitpunkt der Lymphknotenentnahme** | **Gadolinium-Konzentration in drei aufeinanderfolgenden Lymphknotenstationen [µmol/l] [% Dosis/g Gewebe]** | | | |
|---|---|---|---|---|
| | **Popliteal** | **Inguinal** | **Iliakal** | **Verhältnis** |
| 30 min p.i. | 921 µmol/l 20,1 % | 387 µmol/l 8,5 % | 215 µmol/l 4,7 % | 10 : 4,2 : 2,3 |
| 90 min p.i. | 659 µmol/l 14,4 % | 120 µmol/l 2,6 % | 68 µmol/l 1,5 % | 10 : 1,8 : 1,0 |
| 4 h p.i. | 176 µmol/l 3,9 % | 79 µmol/l 1,7 % | 47 µmol/l 1,0 % | 10 : 4,5 : 2,7 |
| 24 h p.i. | 62 µmol/l 1,4 % | 13 µmol/l 0.3 % | 28 µmol/l 0,6 % | 10 : 2,1 : 4,5 |

## Patentansprüche

1. Kaskaden-Polymer-Kornplexe enthaltend
a) komplexbildende Liganden der allgemeinen Formel I
A-{X-[Y-(Z-〈W-K_{w}〉_{z})_{y}]ₓ}ₐ (I),
worin
A für einen stickstoffhaltigen Kaskadenkern der Basismultiplizität a,
X und Y unabhängig voneinander für eine direkte Bindung oder eine Kaskadenreproduktionseinheit der Reproduktionsmultiplizität x bzw. y,
Z und W unabhängig voneinander für eine Kaskadenreproduktionseinheit der Reproduktionsmultiplizität z bzw. w,
K für den Rest eines Komplexbildners,
a für die Ziffern 2 bis 12,
x, y, z und w unabhängig voneinander für die Ziffern 1 bis 4 stehen,
mit der Maßgabe, daß mindestens zwei Reproduktionseinheiten unterschiedlich sind und daß für das Produkt der Multiplizitäten gilt
16 ≤ a · x · y · z · w ≤ 64,
b) mindestens 16 Ionen eines Elements der Ordnungszahlen 20 bis 29, 39, 42, 44 oder 57-83,
c) gegebenenfalls Kationen anorganischer und/oder organischer Basen, Aminosäuren oder Aminosäureamide sowie
d) gegebenenfalls acylierte terminale Aminogruppen
**gekennzeichnet durch** folgende Merkmale:
der an die terminalen Stickstoffatome der letzten Generation der Reproduktionseinheit W gebundene Komplexbildner-Rest K steht für einen Rest der allgemeinen Formeln IA oder IB 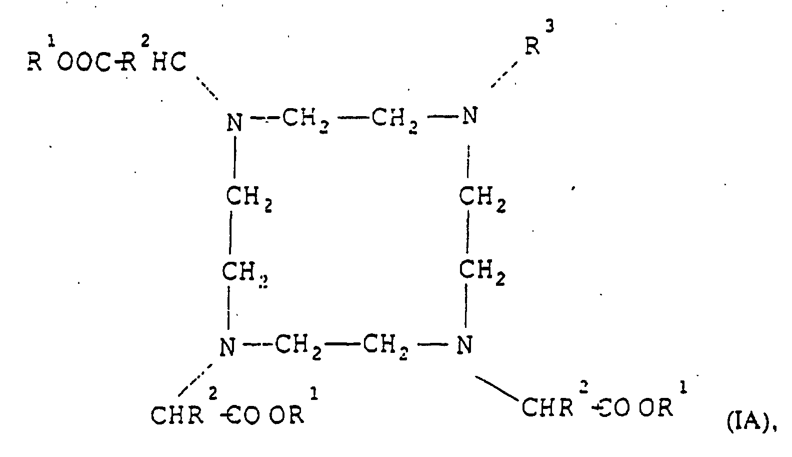 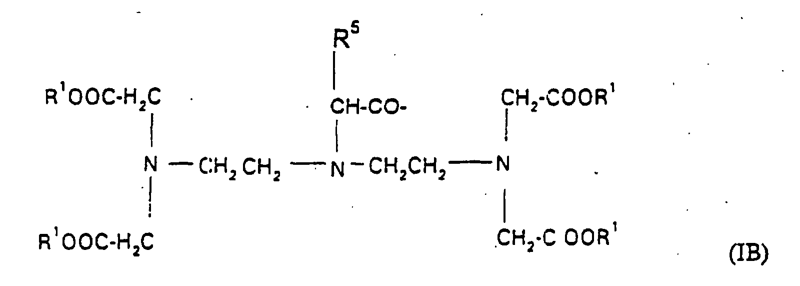 worin
R¹ unabhängig voneinander für ein Wasserstoffatom oder ein Metallionenäquivalent der Ordnungszahlen 20-29, 39, 42-44 oder 57-83,
R² für ein Wasserstoffatom, einen Methyl- oder einen Ethylrest, der gegebenenfalls mit 1-2 Hydroxy- oder 1 Carboxygruppe(n) substituiert ist,
R³ für eine 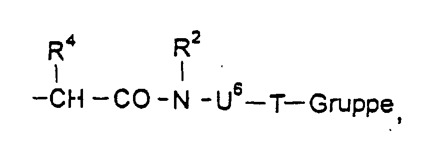
R⁴ für eine geradkettige, verzweigte, gesättigte oder ungesättigte C₁-C₃₀-Alkylkette, die gegebenenfalls **durch** 1-10 Sauerstoffatome, 1 Phenylen-, 1 Phenylenoxygruppen unterbrochen und/oder gegebenenfalls **durch** 1-5 Hydroxy-, 1-3 Carboxy-, 1 Phenylgruppe(n) substituiert ist,
R⁵ für ein Wasserstoffatom oder für R⁴,
U⁶ für eine gegebenenfalls 1-5 Imino-, 1-3 Phenylen-, 1-3 Phenylenoxy-, 1-3 Phenylenimino-, 1-5 Amid-, 1-2 Hydrazid-, 1-5 Carbonyl-, 1-5 Ethylenoxy-, 1 Harnstoff-, 1-Thioharnstoff-, 1-2 Carboxyalkylimino-, 1-2 Estergruppen, 1-10 Sauerstoff-, 1-5 Schwefel- und/oder 1-5 Stickstoff-Atom(e) enthaltende und/oder gegebenenfalls **durch** 1-5 Hydroxy-, 1-2 Mercapto-, 1-5 Oxo-, 1-5 Thioxo-, 1-3 Carboxy-, 1-5 Carboxyalkyl-, 1-5 Ester- und/oder 1-3 Aminogruppe(n) substituierte geradkettige, verzweigte, gesättigte oder ungesättigte C₁-C₂₀-Alkylengruppe, wobei die gegebenenfalls enthaltenen Phenylengruppen **durch** 1-2 Carboxy-, 1-2 Sulfon- oder 1-2 Hydroxygruppen substituiert sein können,
T für eine -CO-α, -NHCO-α- oder -NHCS-α-Gruppe und
α für die Bindungsstelle an die terminalen Stickstoffatome der letzten Generation, der Reproduktionseinheit W
stehen.

2. Kaskaden-Polymer-Komplexe gemäß Anspruch 1, **dadurch gekennzeichnet, daß** A ein Stickstoffatom, 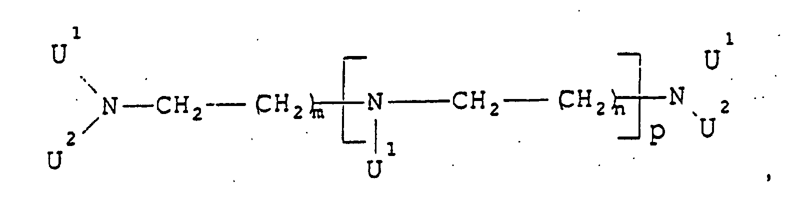 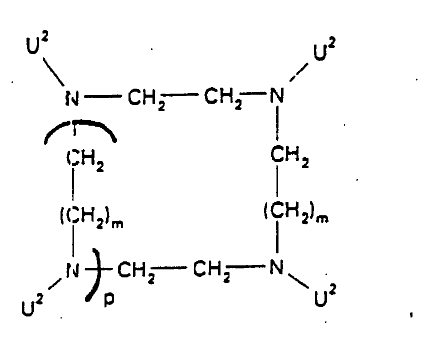 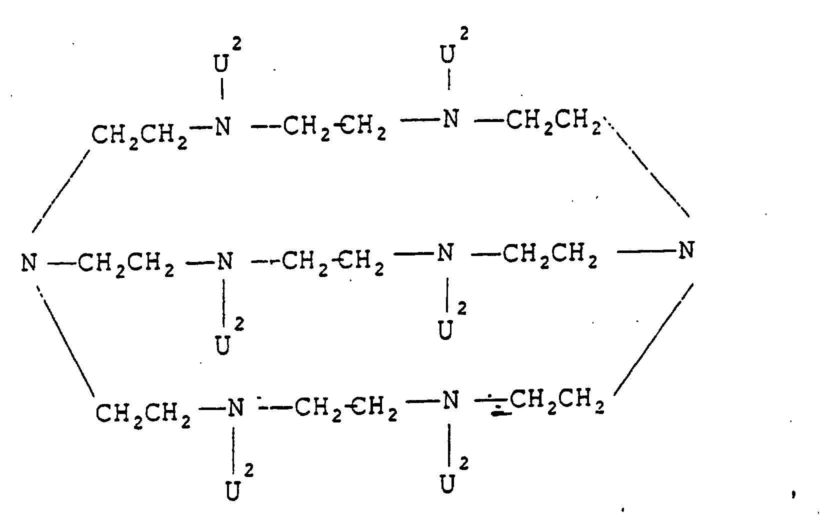 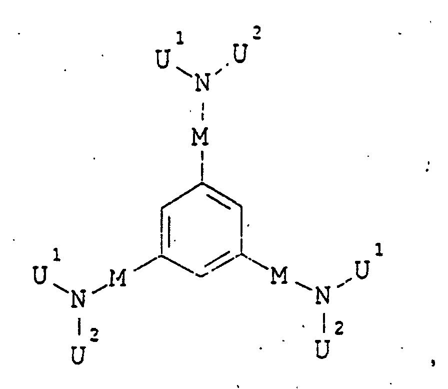 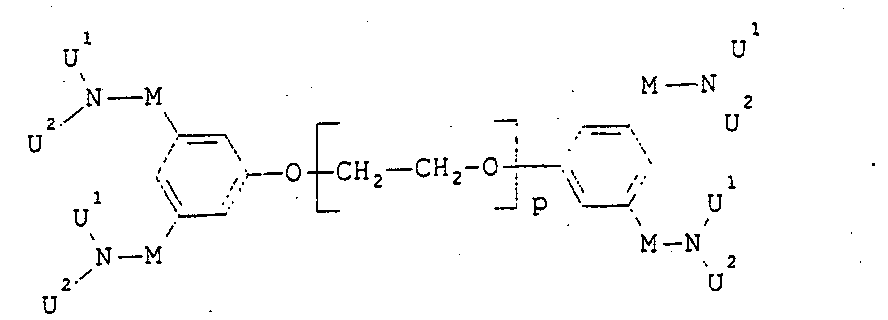 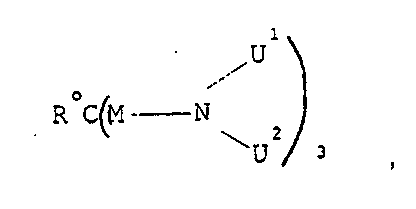 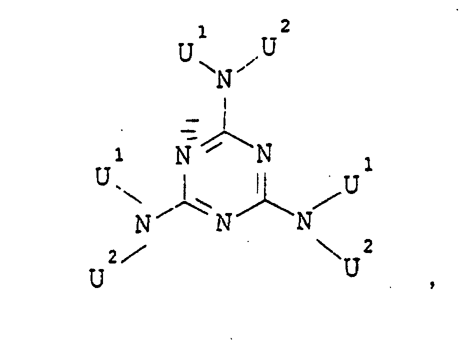 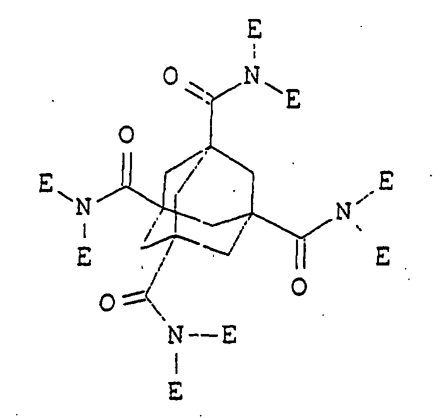 bedeutet, worin
m und n für die Ziffern 1 bis 10,
p für die Ziffern 0 bis 10,
U¹ für Q¹ oder E,
U² für Q² oder E mit
E in der Bedeutung der Gruppe 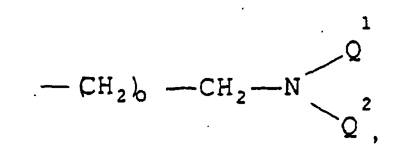 wobei
o für die Ziffern 1 bis 6,
Q¹ für ein Wasserstoffatom oder Q² und
Q² für eine direkte Bindung
M für eine C₁-C₁₀-Alkylenkette, die gegebenenfalls durch 1 bis 3 Sauerstoffatome unterbrochen ist und/oder gegebenenfalls mit 1 bis 2 Oxogruppen substituiert ist,
R^{o} für einen verzweigten oder unverzweigten C₁-C₁₀-Alkylrest, eine Nitro-, Amino-, Carbonsäuregruppe oder für 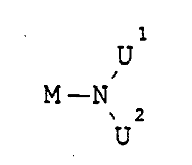 stehen,
wobei die Anzahl Q² der Basismultiplizität a entspricht.

3. Kaskaden-Polymer-Komplexe gemäß Anspruch 1, **dadurch gekennzeichnet, daß** die Kaskadenreproduktionseinheiten X, Y, Z und W unabhängig voneinander für
E, 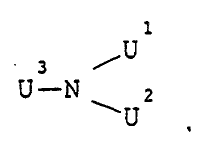 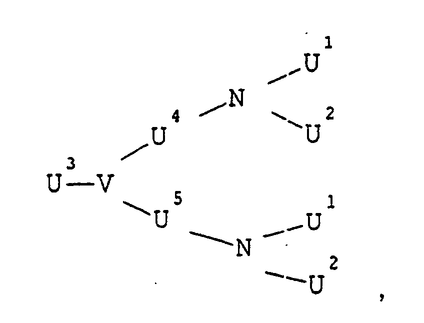 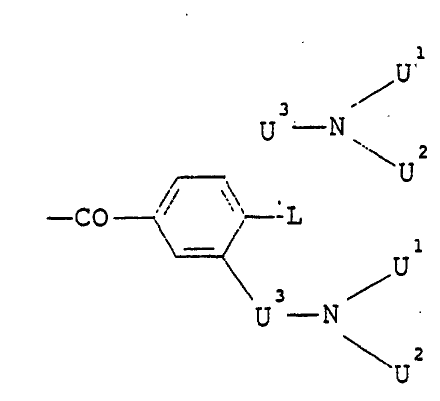 stehen,
worin
U¹ für Q¹ oder E,
U² für Q² oder E mit
E in der Bedeutung der Gruppe 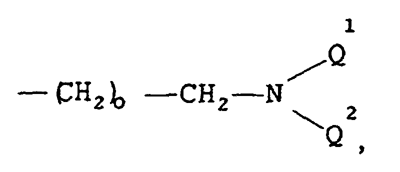 wobei
o für die Ziffern 1 bis 6,
Q¹ für ein Wasserstoffatom oder Q²,
Q² für eine direkte Bindung,
U³ für eine C₁-C₂₀-Alkylenkette, die gegebenenfalls durch 1 bis 10 Sauerstoffatome und/oder 1 bis 2 -N(CO)_{q}-R²-, 1 bis 2 Phenylenund/oder 1 bis 2 Phenylenoxyreste unterbrochen ist und/oder gegebenenfalls durch 1 bis 2 Oxo-, Thioxo-, Carboxy-, C₁-C₅-Alkylcarboxy-, C₁-C₅-Alkoxy-, Hydroxy-, C₁-C₅-alkylgruppen substituiert ist, wobei
q für die Ziffern 0 oder 1 und
R² für ein Wasserstoffatom, einen Methyl- oder einen Ethylrest, der gegebenenfalls mit 1-2 Hydroxy- oder 1 Carboxygruppe(n) sustituiert ist,
L für ein Wasserstoffatom oder die Gruppe 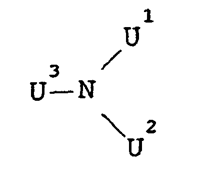
V für die Methingruppe 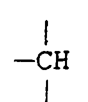 wenn gleichzeitig U⁴ eine direkte Bindung oder die Gruppe M bedeutet und U⁵ eine der Bedeutungen von U³ besitzt oder
V für die Gruppe 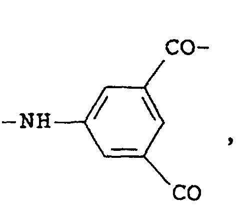 wenn gleichzeitig U⁴ und U⁵ identisch sind und die direkte Bindung oder die Gruppe M bedeuten,
wobei M für eine C₁-C₁₀-Alkylenkette, die gegebenenfalls durch 1 bis 3 Sauerstoffatome unterbrochen ist und/oder gegebenenfalls mit 1 bis 2 Oxogruppen substituiert ist, steht, stehen.

4. Kaskaden-Polymer-Komplexe gemäß Anspruch 1, **dadurch gekennzeichnet, daß** die für U⁶ stehende C₁-C₂₀-Alkylenkette die Gruppen
-CH₂-, -CH₂NHCO-, -NHCOCH₂O-, -NHCOCH₂OC₆H₄-, -N(CH₂CO₂H)-, -NHCOCH₂C₆H₄-, -NHCSNHC₆H₄-, -CH₂OC₆H₄-, -CH₂CH₂O-, enthält und/oder durch die Gruppen -COOH, -CH₂COOH
substituiert ist.

5. Kaskaden-Polymer-Komplexe gemäß Anspruch 1, **dadurch gekennzeichnet, daß** U⁶ für eine
-CH₂-, -CH₂CH₂-, -CH₂CH₂CH₂-, -C₆H₄-, -C₆H₁₀-, -CH₂C₆H₅-, -CH₂NHCOCH₂CH(CH₂CO₂H)-C₆H₄-, -CH₂NHCOCH₂OCH₂-, -CH₂NHCOCH₂C₆H₄-,
gruppe steht.

6. Kaskaden-Polymer-Komplexe gemäß Anspruch 3, **dadurch gekennzeichnet, daß** der in den Kaskadenreproduktionseinheiten X, Y, Z und W enthaltene Rest U³ für
-CO-, -COCH₂OCH₂CO-, -COCH₂-, -CH₂CH₂-, -CONHC₆H₄-, -COCH₂CH₂CO-, -COCH₂-CH₂CH₂CO-, -COCH₂CH₂CH₂CH₂CO-,
der Rest U⁴ für eine direkte Bindung, für -CH₂CO-,
der Rest U⁵ für eine direkte Bindung, für -(CH₂)₄-, -CH₂CO-, -CH(COOH)-, CH₂OCH₂CH₂-, -CH₂C₆H₄-, CH₂-C₆H₄OCH₂CH₂-,
der Rest E für eine Gruppe 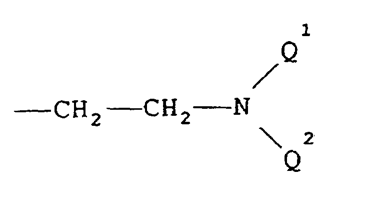 steht.

7. Kaskaden-Polymer-Komplexe gemäß Anspruch 3, **dadurch gekennzeichnet, daß** die Kaskadenreproduktionseinheiten X, Y, Z und W unabhängig voneinander für
-CH₂CH₂NH-; -CH₂CH₂N< ; -COCH(NH-)(CH₂)₄NH-; -COCH(N < )(CH₂)₄N< ; -COCH₂OCH₂CON(CH₂CH₂NH-)₂; -COCH₂OCH₂CON(CH₂CH₂N< )₂ ; -COCH₂N(CH₂CH₂NH-)₂; -COCH₂N(CH₂CH₂N< )₂; -COCH₂NH-; -COCH₂N< ; -COCH₂CH₂CON(CH₂CH₂NH-)₂; -COCH₂CH₂CON(CH₂CH₂N< )₂ ; -COCH₂OCH₂CONH-C₆H₄-CH[CH₂CON(CH₂CH₂NH-)₂]₂; -COCH₂OCH₂CONH-C₆H₄-CH[CH₂CON(CH₂CH₂N < )₂]₂ ; -COCH₂CH₂CO-NH-C₆H₄-CH[CH₂CON(CH₂CH₂NH-)₂]₂ ; -COCH₂CH₂CO-NH-C₆H₄-CH[CH₂CON(CH₂CH₂N< )₂]₂ ; -CONH-C₆H₄-CH[CH₂CON(CH₂CH₂NH-)₂]₂ ; -CONH-C₆H₄-CH[CH₂CON(CH₂CH₂N<)₂]₂ ; -COCH(NH-)CH(COOH)NH-; -COCH(N< )CH(COOH)N< ; 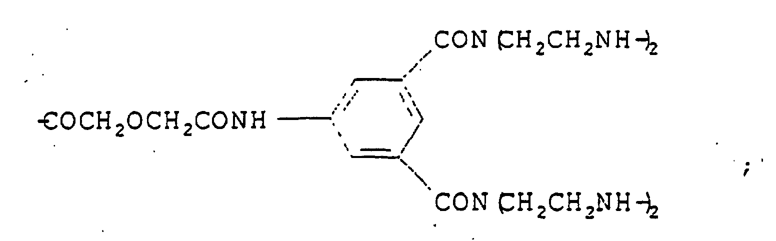 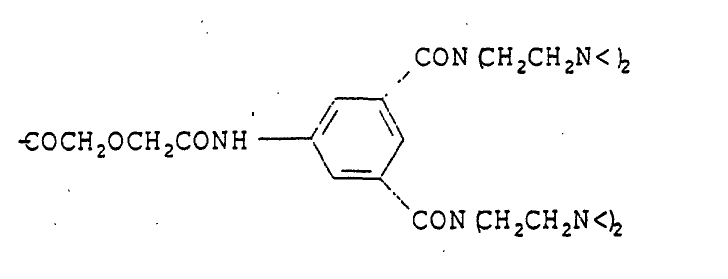 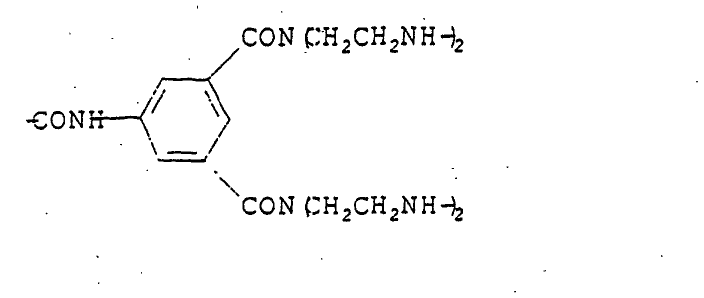 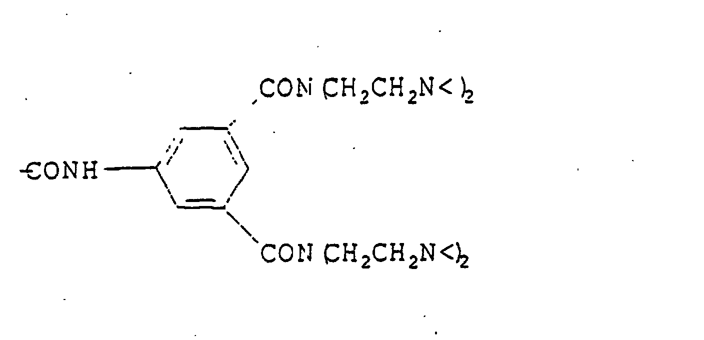 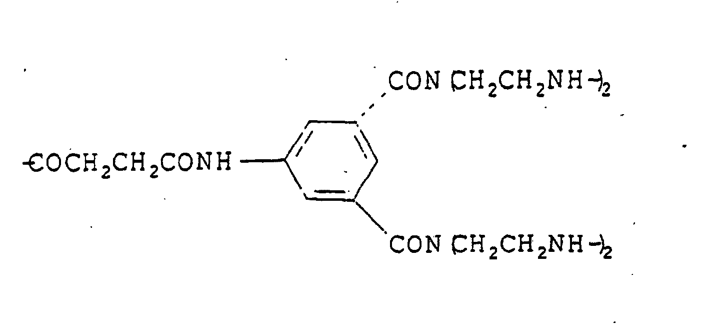 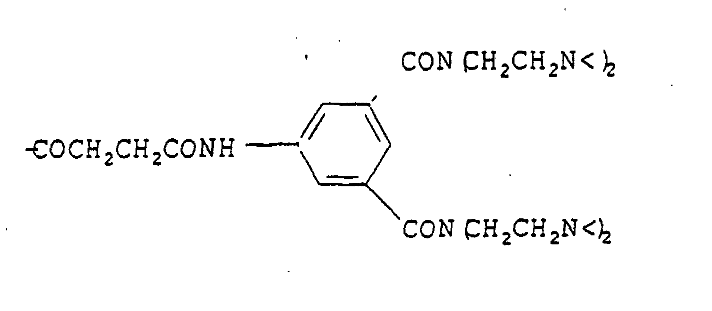 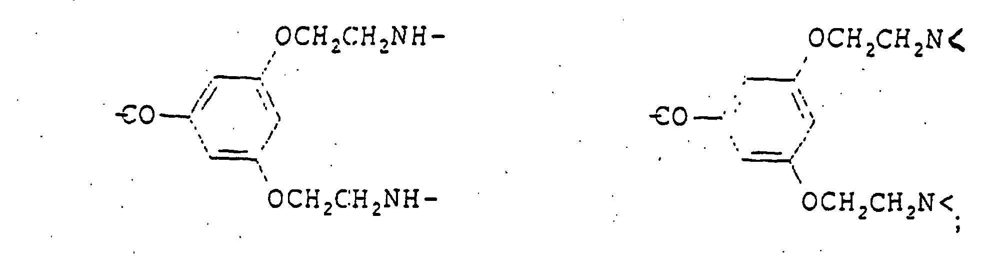 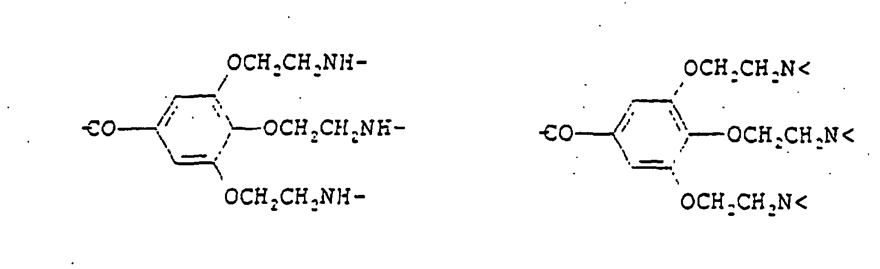 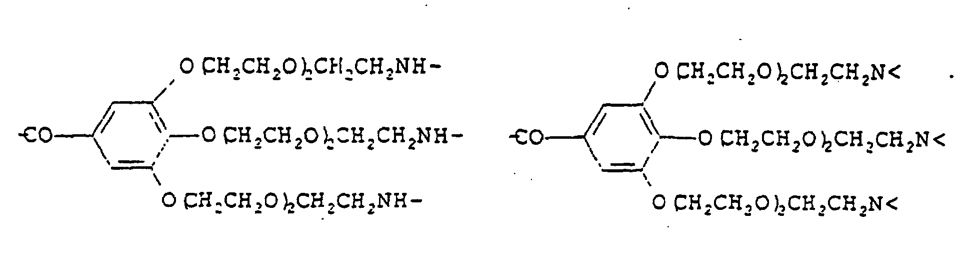 stehen.

8. Kaskaden-Polymer-Komplexe gemäß Anspruch 2, **dadurch gekennzeichnet, daß**
m für die Ziffern 1-3,
n für die Ziffern 1-3,
o für die Ziffer 1,
p für die Ziffern 0-3,
M für eine -CH₂-, -CO- oder -CH₂CO-Gruppe und
R^{o} für eine -CH₂NU¹U², CH₃- oder NO₂-Gruppe
stehen.

9. Pharmazeutische Mittel enthaltend mindestens einen Kaskaden-Polymer-Komplex nach Anspruch 1, gegebenenfalls mit den in der Galenik üblichen Zusätzen.

10. Verwendung von mindestens einem Polymer-Komplex nach Anspruch 1 für die Herstellung von Mitteln für die NMR- oder Röntgendiagnostik (konventionelle Methoden und Computertomographie).

11. Verwendung der Kaskaden-Polymer-Komplexe nach Anspruch 1 für die Herstellung von Mitteln zur Differenzierung von benignen und malignen Tumoren in Körperregionen ohne Blut-Hirn-Schranke.

12. Verfahren zur Herstellung von Kaskaden-Polymer-Komplexen gemäß den Ansprüchen 1 bis 8, **dadurch gekennzeichnet, daß** man Verbindungen der allgemeinen Formel I'
A-{X-[Y-(Z-〈W-β_{w}〉_{z})_{y}]ₓ}ₐ (I'),
worin
A für einen stickstoffhaltigen Kaskadenkern der Basismultiplizität a,
X und Y unabhängig voneinander für eine direkte Bindung oder eine Kaskadenreproduktionseinheit der Reproduktionsmultiplizität x bzw. y,
Z und W unabhängig voneinander für eine Kaskadenreproduktionseinheit der Reproduktionsmultiplizität z bzw. w,
a für die Ziffern 2 bis 12,
x, y, z und w unabhängig voneinander für die Ziffern 1 bis 4 und
β für die Bindungsstelle der terminalen NH-Gruppen der letzten Generation, der Reproduktionseinheit W stehen
mit der Maßgabe, daß mindestens zwei Reproduktionseinheiten unterschiedlich sind und daß für das Produkt der Multiplizitäten gilt
16 ≤ a · x · y · z · w ≤ 64, mit einem Komplex oder Komplexbildner K' der allgemeinen Formel I'A oder I'B 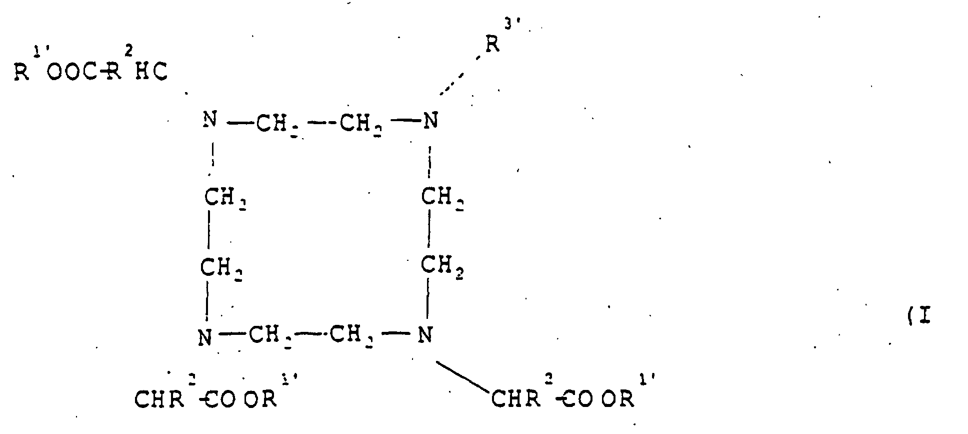 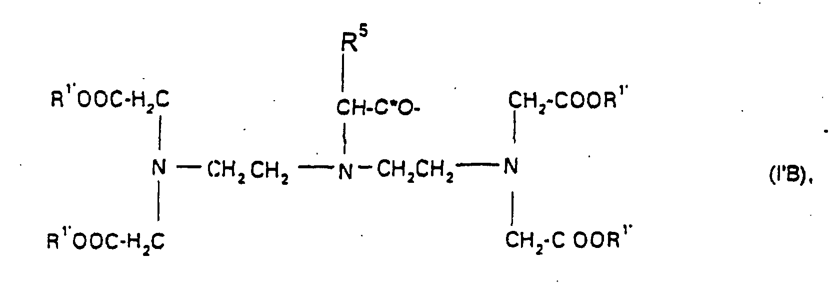 wobei
R1' unabhängig voneinander für ein Wasserstoffatom, ein Metallionenäquivalent der Ordnungszahlen 20-29, 39, 42-44 oder 57-83 oder eine Säureschutzgruppe,
R² für ein Wasserstoffatom, einen Methyl- oder einen Ethylrest, der gegebenenfalls mit 1-2 Hydroxy- oder 1 Carboxygruppe(n) substituiert ist,
R^{3'} für eine 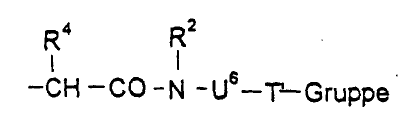
R⁴ für eine geradkettige, verzweigte, gesättigte oder ungesättigte C₁-C₃₀-Alkylkette, die gegebenenfalls durch 1-10 Sauerstoffatome, 1 Phenylen-, 1 Phenylenoxygruppen unterbrochen und/oder gegebenenfalls durch 1-5 Hydroxy-, 1-3 Carboxy-, 1 Phenylgruppe(n) substituiert ist,
R⁵ für ein Wasserstoffatom oder für R⁴,
U⁶ für eine gegebenenfalls 1-5 Imino-, 1-3 Phenylen-, 1-3 Phenylenoxy-, 1-3 Phenylenimino-, 1-5 Amid-, 1-2 Hydrazid-, 1-5 Carbonyl-, 1-5 Ethylenoxy-, 1 Harnstoff-, 1-Thioharnstoff-, 1-2 Carboxyalkylimino-, 1-2 Estergruppen, 1-10 Sauerstoff-, 1-5 Schwefel- und/oder 1-5 Stickstoff-Atom(e) enthaltende und/oder gegebenenfalls durch 1-5 Hydroxy-, 1-2 Mercapto-, 1-5 Oxo-, 1-5 Thioxo-, 1-3 Carboxy-, 1-5 Carboxyalkyl-, 1-5 Ester- und/oder 1-3 Aminogruppe(n) substituierte geradkettige, verzweigte, gesättigte oder ungesättigte C₁-C₂₀-Alkylengruppe, wobei die gegebenenfalls enthaltenen Phenylengruppen durch 1-2 Carboxy-, 1-2 Sulfon- oder 1-2 Hydroxygruppen substituiert sein können,
T' für eine-C*O-, -COOH-, -N=C=O- oder -N=C=S-Gruppe und
C*O für eine aktivierte Carboxylgruppe
stehen
mit der Maßgabe, daß - sofern K' für einen Komplex steht - mindestens zwei (bei zweiwertigen Metallen) bzw. drei (bei dreiwertigen Metallen) der Substituenten R¹ für ein Metallionenäquivalent der oben genannten Elemente stehen und daß gewünschtenfalls weitere Carboxylgruppen in Form ihrer Salze mit anorganischen und/oder organischen Basen, Aminosäuren oder Aminosäureamiden vorliegen,
umsetzt, gegebenenfalls vorhandene Schutzgruppen abspaltet, die so erhaltenen Kaskaden-Polymere - sofern K' für einen Komplexbildner steht - in an sich bekannter Weise mit mindestens einem Metalloxid oder Metallsalz eines Elements der Ordnungszahlen 20-29, 39, 42. 44 oder 57-83 umsetzt und gegebenenfalls anschließend in den so erhaltenen Kaskaden-Polymer-Komplexen noch vorhandene acide Wasserstoffatome ganz oder teilweise durch Kationen von anorganischen und/oder organischen Basen, Aminosäuren oder Aminosäureamiden substituiert und gegebenenfalls noch vorhandene freie terminale Aminogruppen gewünschtenfalls - vor oder nach der Metallkomplexierung - acyliert.

13. Verbindungen der allgemeinen Formel I'A 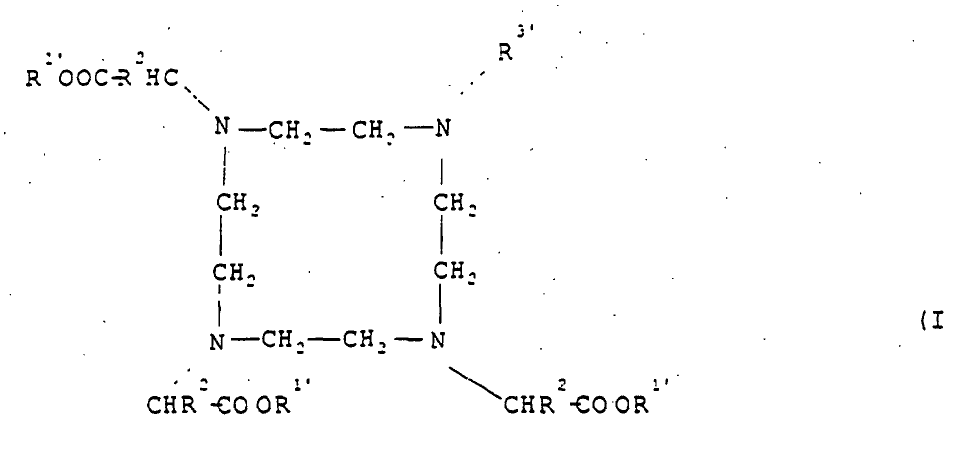 wobei
R1' unabhängig voneinander für ein Wasserstoffatom, ein Metallionenäquivalent der Ordnungszahlen 20-29, 39, 42-44 oder 57-83 oder eine Säureschutzgruppe,
R² für ein Wasserstoffatom, einen Methyl- oder einen Ethylrest, der gegebenenfalls mit 1-2 Hydroxy- oder 1 Carboxygruppe(n) substituiert ist,
R^{3'} für eine 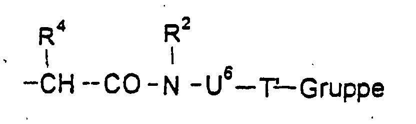
R⁴ für eine geradkettige, verzweigte, gesättigte oder ungesättigte C₁-C₃₀-Alkylkette, die gegebenenfalls durch 1-10 Sauerstoffatome, 1 Phenylen-, 1 Phenylenoxygruppen unterbrochen und/oder gegebenenfalls durch 1-5 Hydroxy-, 1-3 Carboxy-, 1 Phenylgruppe(n) substituiert ist,
U⁶ für eine gegebenenfalls 1-5 Imino-, 1-3 Phenylen-, 1-3 Phenylenoxy-, 1-3 Phenylenimino-, 1-5 Amid-, 1-2 Hydrazid-, 1-5 Carbonyl-, 1-5 Ethylenoxy-, 1 Harnstoff-, 1-Thioharnstoff-, 1-2 Carboxyalkylimino-, 1-2 Estergruppen, 1-10 Sauerstoff-, 1-5 Schwefel- und/oder 1-5 Stickstoff-Atom(e) enthaltende und/oder gegebenenfalls durch 1-5 Hydroxy-, 1-2 Mercapto-, 1-5 Oxo-, 1-5 Thioxo-, 1-3 Carboxy-, 1-5 Carboxyalkyl-, 1-5 Ester- und/oder 1-3 Aminogruppe(n) substituierte geradkettige, verzweigte, gesättigte oder ungesättigte C₁-C₂₀-Alkylengruppe, wobei die gegebenenfalls enthaltenen Phenylengruppen durch 1-2 Carboxy-, 1-2 Sulfon- oder 1-2 Hydroxygruppen substituiert sein können,
T' für eine -C*O-, -COOH-, -N=C=O- oder -N=C=S-Gruppe und
C*O für eine aktivierte Carboxylgruppe
stehen.

14. Verfahren zur Herstellung der pharmazeutischen Mittel gemäß Anspruch 9, **dadurch gekennzeichnet, daß** man den in Wasser oder physiologischer Salzlösung gelösten oder suspendierten Kaskaden-Polymer-Komplex, gegebenenfalls mit den in der Galenik üblichen Zusätzen, in eine für die enterale oder parenterale Applikation geeignete Form bringt.

## Claims

1. Cascade polymer complexes containing
a) complexing ligands of the general formula I
A-{X-[Y-(Z-(W-K_{w})_{z})_{y}]ₓ}ₐ (I),
in which
A stands for a nitrogen-containing cascade nucleus of base multiplicity a,
X and Y, independently of one another, stand for a direct bond or a cascade reproduction unit of reproduction multiplicity x or y,
Z and W, independently of one another, stand for a cascade reproduction unit of reproduction multiplicity z or w,
K stands for the radical of a complexing agent,
a stands for a number from 2 to 12,
x, y, z and w, independently of one another, stand for a number from 1 to 4,
with the proviso that at least two reproduction units are different and that
16 ≤ a·x·y·z·w ≤ 64 holds true for the product of the multiplicities,
b) at least 16 ions of an element of atomic numbers 20 to 29, 39, 42, 44 or 57-83,
c) optionally cations of inorganic and/or organic bases, amino acids or amino acid amides, and
d) optionally acylated terminal amino groups
**characterized by** the following features:
the complexing agent radical K which is bonded to the terminal nitrogen atoms of the last generation of reproduction unit W stands for a radical of the general formulae IA or IB 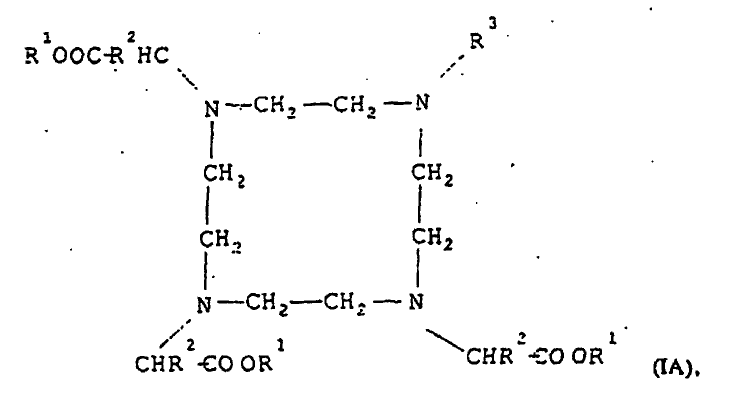 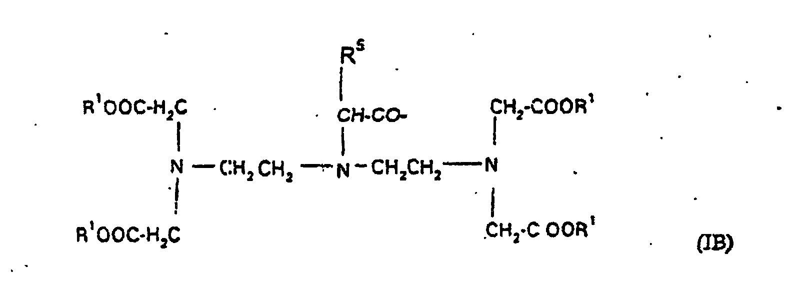 in which
R¹, independently of one another, stand for a hydrogen atom or a metal ion equivalent of atomic numbers 20-29, 39, 42-44 or 57-83,
R² stands for a hydrogen atom, a methyl or an ethyl radical which optionally is substituted by 1-2 hydroxy or 1 carboxy group(s),
R³ stands for a 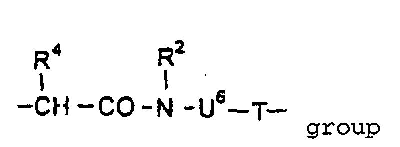
R⁴ stands for a straight-chain, branched, saturated or unsaturated C₁-C₃₀ alkyl chain, which optionally is interrupted by 1-10 oxygen atoms, 1 phenylene group, 1 phenyleneoxy group and/or optionally substituted by 1-5 hydroxy, 1-3 carboxy, 1-phenyl group(s),
R⁵ stands for a hydrogen atom or for R⁴,
U⁶ stands for a straight-chain, branched, saturated or unsaturated C₁-C₂₀ alkylene group optionally containing 1-5 imino, 1-3 phenylene, 1-3 phenyleneoxy, 1-3 phenyleneimino, 1-5 amide, 1-2 hydrazide, 1-5 carbonyl, 1-5 ethyleneoxy, 1 urea, 1 thiourea, 1-2 carboxyalkylimino, 1-2 ester groups, or 1-10 oxygen, 1-5 sulphur and/or 1-5 nitrogen atom(s) and/or optionally substituted by 1-5 hydroxy, 1-2 mercapto, 1-5 oxo, 1-5 thioxo, 1-3 carboxy, 1-5 carboxyalkyl, 1-5 ester and/or 1-3 amino group(s), and the phenylene groups that are optionally contained can be substituted by 1-2 carboxy, 1-2 sulphone or 1-2 hydroxy groups,
T stands for a -CO-α, -NHCO-α or -NHCS-α group, and
α stands for the bonding site to the terminal nitrogen atoms of the last generation of reproduction unit W.

2. Cascade polymer complexes according to Claim 1,
**characterized in that** A means a nitrogen atom, 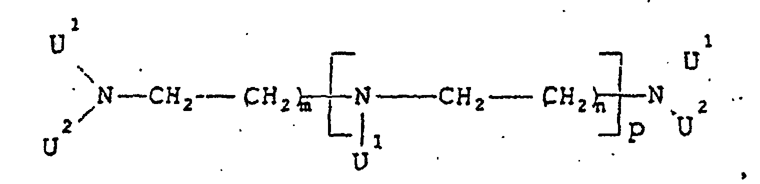 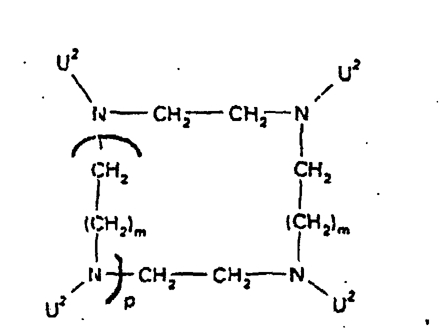 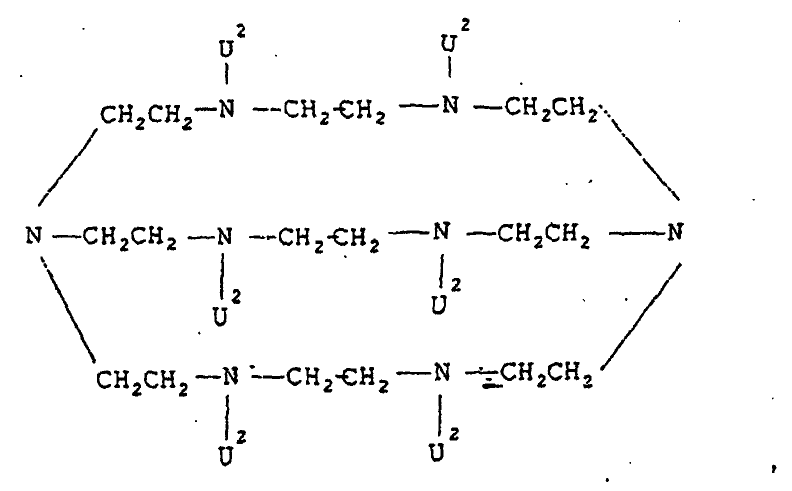 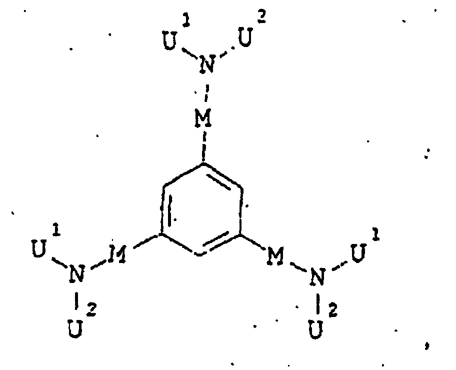 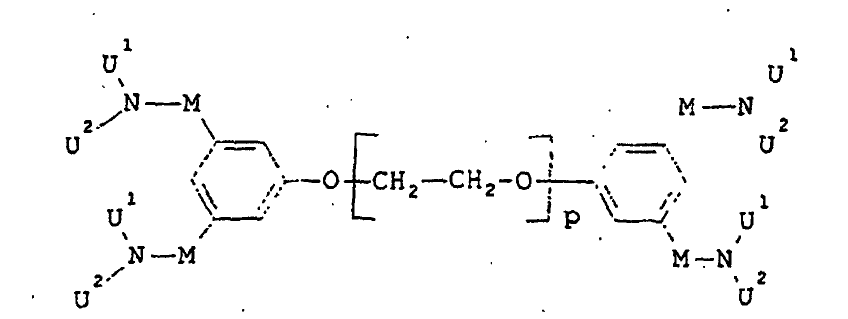 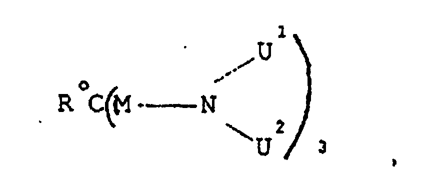 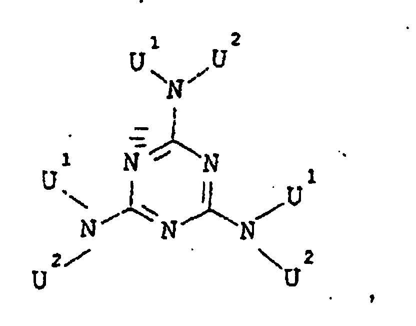 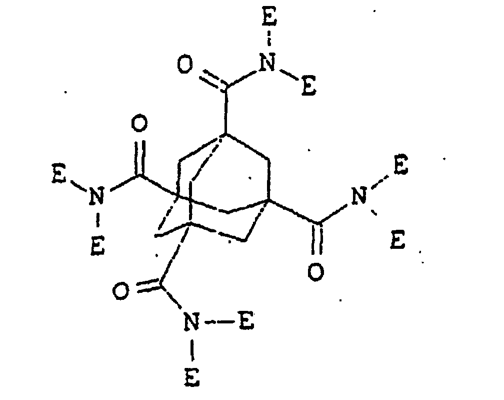 in which
m and n stand for the numbers 1 to 10,
p stands for the numbers 0 to 10,
U¹ stands for Q¹ or E,
U² stands for Q² or E with
E meaning the group 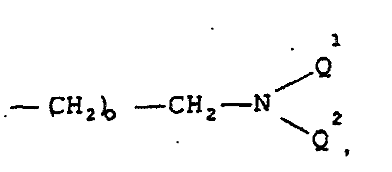 where o stands for the numbers 1 to 6,
Q¹ stands for a hydrogen atom or Q² and
Q² stands for a direct bond,
M stands for a C₁-C₁₀ alkylene chain which optionally is interrupted by 1 to 3 oxygen atoms and/or optionally is substituted by 1 to 2 oxo groups,
R^{o} stands for a branched or unbranched C₁-C₁₀ alkyl radical, a nitro, amino, carboxylic acid group or for 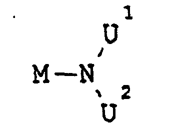 where the number of Q² elements equals the base multiplicity a.

3. Cascade polymer complexes according to Claim 1, **characterized in that** the cascade reproduction units X, Y, Z and W, independently of one another, stand for E,
E, 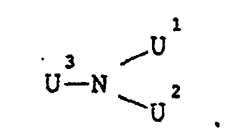 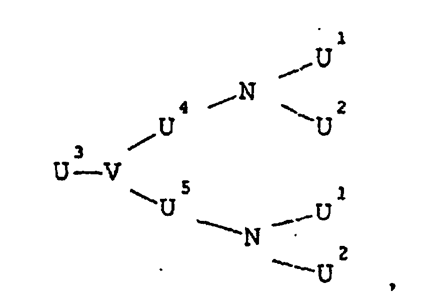 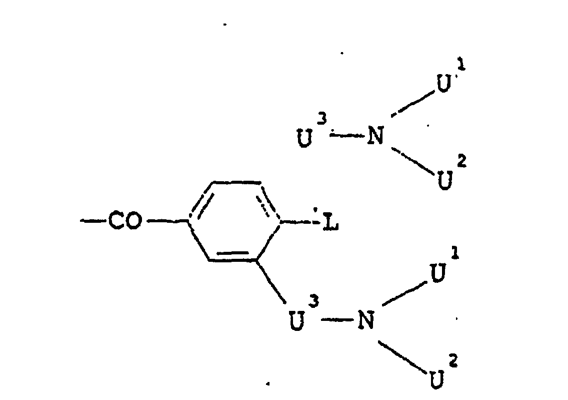 in which
U¹ stands for Q¹ or E,
U² stands for Q² or E with
E meaning the group 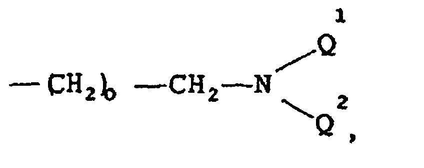 where
o stands for the numbers 1 to 6,
Q¹ stands for a hydrogen atom or Q²,
Q² stands for a direct bond,
U³ stands for a C₁-C₂₀ alkylene chain which optionally is interrupted by 1 to 10 oxygen atoms and/or 1 to 2 -N(CO)_{q}-R²-, 1 to 2 phenylene and/or 1 to 2 phenyleneoxy radicals, and/or optionally is substituted by 1 to 2 oxo, thioxo, carboxy, C₁-C₅ alkylcarboxy, C₁-C₅ alkoxy, hydroxy, C₁-C₅ alkyl groups, where
q stands for the numbers 0 or 1 and
R² stands for a hydrogen atom, a methyl or ethyl radical which optionally is substituted by 1-2 hydroxy or 1 carboxy group(s),
L stands for a hydrogen atom or the group 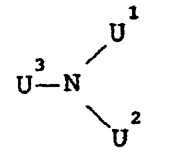
V stands for the methine group 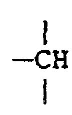 if at the same time U⁴ means a direct bond
or the group M and U⁵ has one of the meanings of U³ or
V stands for the group 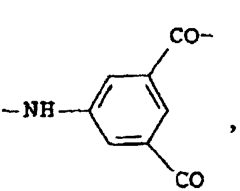 if at the same time U⁴ and U⁵ are identical and mean the direct bond or the group M, where M stands for a C₁-C₁₀ alkylene chain which optionally is interrupted by 1 to 3 oxygen atoms and/or optionally is substituted by 1 to 2 oxo groups.

4. Cascade polymer complexes according to Claim 1, **characterized in that** the C₁-C₂₀ alkylene chain standing for U⁶ contains the groups -CH₂-, -CH₂NHCO-, -NHCOCH₂O-, -NHCOCH₂OC₆H₄-, -N(CH₂CO₂H)-, -NHCOCH₂C₆H₄-, -NHCSNHC₆H₄-, -CH₂OC₆H₄-, -CH₂CH₂O-, and/or is substituted by the groups -COOH, -CH₂COOH.

5. Cascade polymer complexes according to Claim 1, **characterized in that** U⁶ stands for a -CH₂-, -CH₂CH₂-, -CH₂CH₂CH₂-, -C₆H₄-, -C₆H₁₀-, -CH₂C₆H₅-, -CH₂NHCOCH₂CH (CH₂CO₂H)-C₆H₄-, -CH₂NHCOCH₂OCH₂-, -CH₂NHCOCH₂C₆H₄- group.

6. Cascade polymer complexes according to Claim 3, **characterized in that** the radical U³ present in the cascade reproduction units X, Y, Z and W stands for -CO-, -COCH₂OCH₂CO-, -COCH₂-, -CH₂CH₂-, -CONHC₆H₄-, -COCH₂CH₂CO-, -COCH₂-CH₂CH₂CO-, -COCH₂CH₂CH₂CH₂CO-, the radical U⁴ stands for a direct bond, for -CH₂CO-, the radical U⁵ stands for a direct bond, for -(CH₂)₄-, -CH₂CO-, -CH(COOH)-, CH₂OCH₂CH₂-, -CH₂C₆H₄-, CH₂-C₆H₄OCH₂CH₂- the radical E stands for a 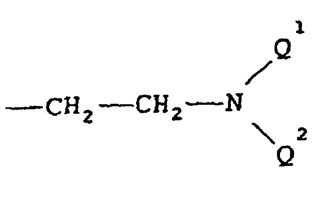 group.

7. Cascade polymer complexes according to Claim 3, **characterized in that** the cascade reproduction units X, Y, Z and W, independently of one another, stand for
-CH₂CH₂NH-; -CH₂CH₂N<; -COCH(NH-)(CH₂)₄NH-; -COCH(N<)(CH₂)₄N<; -COCH₂OCH₂CON(CH₂CH₂NH-)₂; -COCH₂OCH₂CON(CH₂CH₂N<)₂; -COCH₂N(CH₂CH₂NH-)₂; -COCH₂N(CH₂CH₂N<)₂; -COCH₂NH-; -COCH₂N<; -COCH₂CH₂CON(CH₂CH₂NH-)₂; -COCH₂CH₂CON(CH₂CH₂N<)₂; -COCH₂OCH₂CONH-C₆H₄-CH[CH₂CON(CH₂CH₂NH-)₂]₂; -COCH₂OCH₂CONH-C₆H₄-CH[CH₂CON(CH₂CH₂N<)₂]₂; -COCH₂CH₂CO-NH-C₆H₄-CH[CH₂CON(CH₂NH-)₂]₂; -COCH₂CH₂CO-NH-C₆H₄-CH[CH₂CON(CH₂CH₂N<)₂]₂; -CONH-C₆H₄-CH[CH₂CON(CH₂CH₂NH-)₂]₂; -CONH-C₆H₄-CH[CH₂CON(CH₂CH₂N<)₂]₂; -COCH(NH-)CH(COOH)NH-; -COCH(N<)CH(COOH)N<;

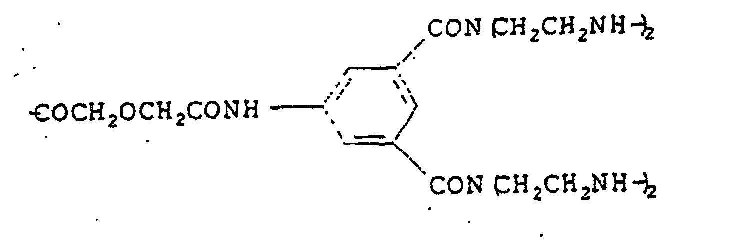

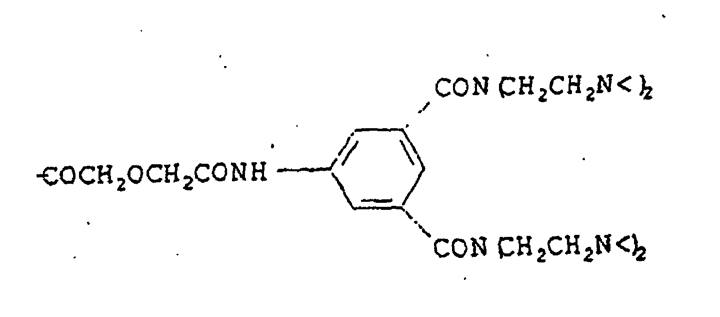

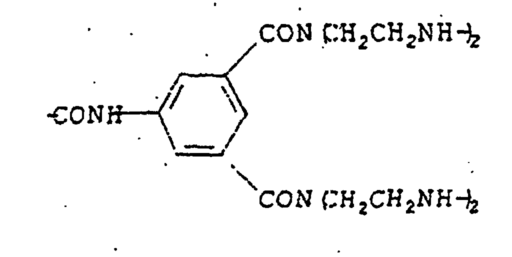

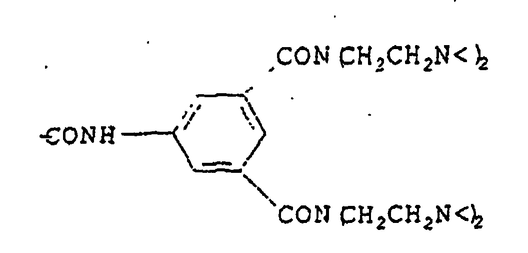

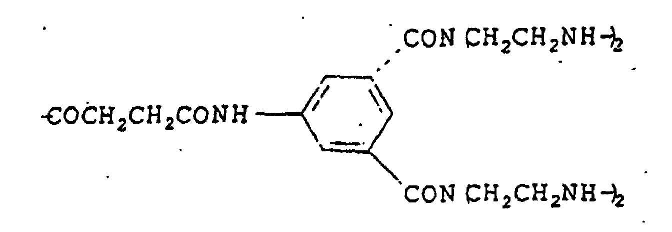

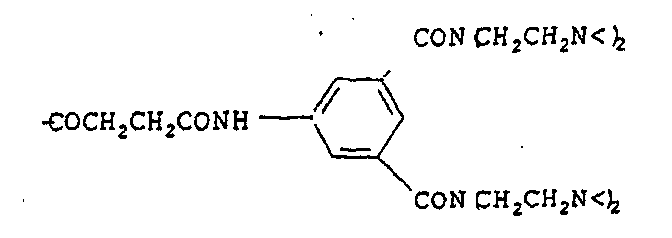

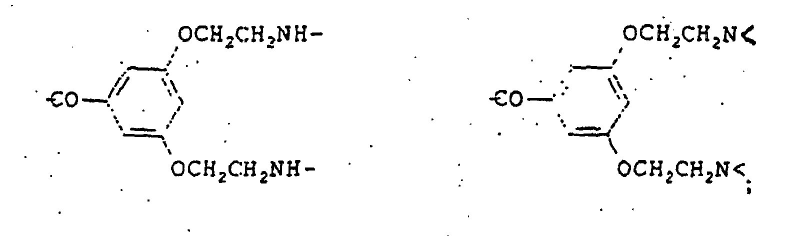

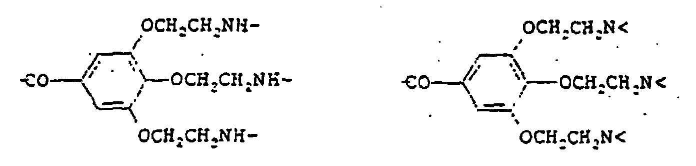

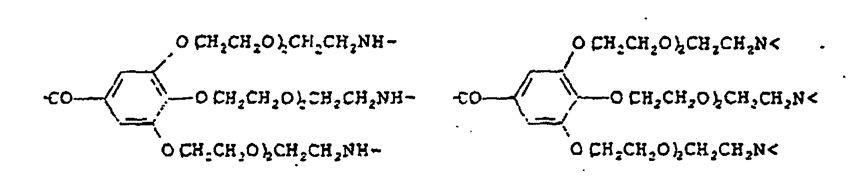

8. Cascade polymer complexes according to Claim 2, **characterized in that**
m stands for the numbers 1-3,
n stands for the numbers 1-3,
o stands for the number 1,
p stands for the numbers 0-3,
M stands for a -CH₂-, -CO- or -CH₂CO- group and
R^{o} stands for a -CH₂NU¹U², CH₃ or NO₂ group.

9. Pharmaceutical compositions containing at least one cascade polymer complex according to Claim 1, optionally with the additions usual in pharmaceutical technology.

10. Use of at least one polymer complex according to Claim 1 for producing compositions for NMR or radiological diagnosis (conventional methods and computed tomography).

11. Use of the cascade polymer complexes according to Claim 1 for producing compositions for differentiating benign and malignant tumours in body regions without a blood-brain barrier.

12. Process for the production of cascade polymer complexes according to Claims 1 to 8, **characterized in that** compounds of the general formula I'
A-{X-[Y-(Z-(W-β_{w})_{z})_{y}]ₓ}a (I'),
in which
A stands for a nitrogen-containing cascade nucleus of base multiplicity a,
X and Y, independently of one another, stand for a direct bond or a cascade reproduction unit of reproduction multiplicity x or y,
Z and W, independently of one another, stand for a cascade reproduction unit of reproduction multiplicity z or w,
a stands for the numbers 2 to 12,
x, y, z and w, independently of one another, stand for the numbers 1 to 4 and
β stands for the bonding site of the terminal NH groups of the last generation of reproduction unit W,
with the proviso that at least two reproduction units are different and that for the product of the multiplicities,
16 ≤ a·x·y·z·w ≤ 64 holds true, are reacted with a complex or complexing agent K' of the general formula I'A or I'B 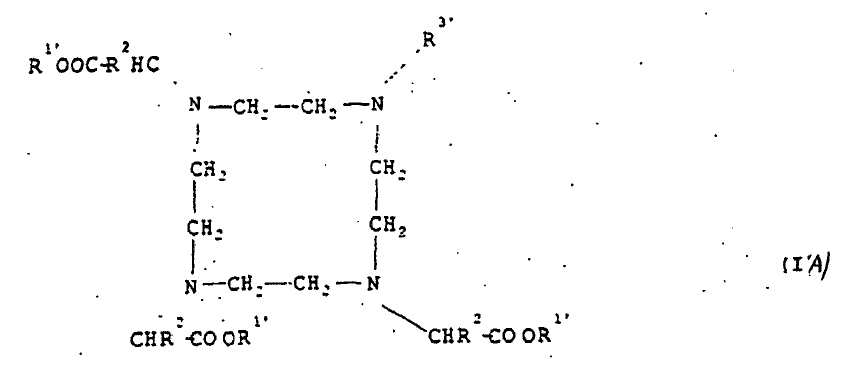 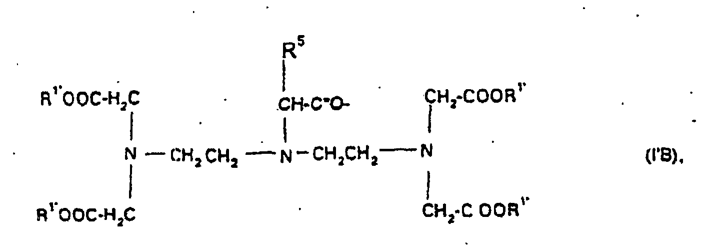 where
R¹', independently of one another, stand for a hydrogen atom, a metal ion equivalent of atomic numbers 20-29, 39, 42-44, or 57-83 or an acid protective group,
R² stands for a hydrogen atom, a methyl or an ethyl radical which optionally is substituted by 1-2 hydroxy or 1 carboxy group(s),
R^{3'} stands for a 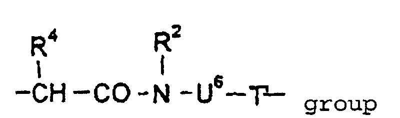
R⁴ stands for a straight-chain, branched, saturated or unsaturated C₁-C₃₀ alkyl chain, which optionally is interrupted by 1-10 oxygen atoms, 1 phenylene group, 1 phenyleneoxy group and/or optionally substituted by 1-5 hydroxy, 1-3 carboxy, 1 phenyl group(s),
R⁵ stands for a hydrogen atom or for R⁴,
U⁶ stands for a straight-chain, branched, saturated or unsaturated C₁-C₂₀ alkylene group optionally containing 1-5 imino, 1-3 phenylene, 1-3 phenyleneoxy, 1-3 phenyleneimino, 1-5 amide, 1-2 hydrazide, 1-5 carbonyl, 1-5 ethyleneoxy, 1 urea, 1 thiourea, 1-2 carboxyalkylimino, 1-2 ester groups; 1-10 oxygen, 1-5 sulphur and/or 1-5 nitrogen atom(s) and/or optionally substituted by 1-5 hydroxy, 1-2 mercapto, 1-5 oxo, 1-5 thioxo, 1-3 carboxy, 1-5 carboxyalkyl, 1-5 ester and/or 1-3 amino group(s), where the phenylene groups that are optionally contained can be substituted by 1-2 carboxy, 1-2 sulphone or 1-2 hydroxy groups,
T' stands for an -C*O, -COOH, -N=C=O or -N=C=S group, and
C*O stands for an activated carboxyl group
with the proviso that - if K' stands for a complex - at least two (in the case of divalent metals) or three (in the case of trivalent metals),, of the substituents R¹ stand for a metal ion equivalent of the abovementioned elements and that if desired other carboxyl groups are present in the form of their salts with inorganic and/or organic bases, amino acids or amino acid amides,
any optionally present protective groups are eliminated, the thus obtained cascade polymers - if K' stands for a complexing agent - are reacted in a manner known per se with at least one metal oxide or metal salt of an element of atomic numbers 20-29, 39, 42, 44, or 57-83 and optionally then acidic hydrogen atoms that are still present in the cascade polymer complexes thus obtained are completely or partially substituted by cations of inorganic and/or organic bases, amino acids or amino acid amides and optionally still present free terminal amino groups are optionally acylated - before or after the metal complexing.

13. Compounds of the general formula I'A 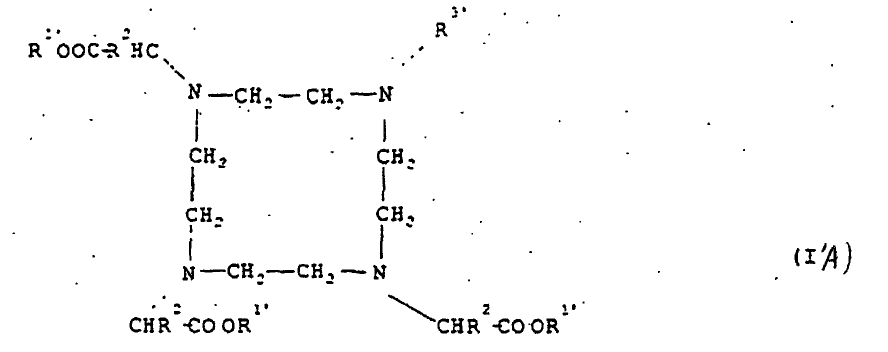 where
R^{1'}, independently of one another, stand for a hydrogen atom, a metal ion equivalent to the atomic numbers 20-29, 39, 42-44 or 57-83 or an acid protective group,
R² stands for a hydrogen atom, a methyl or ethyl radical which optionally is substituted by 1-2 hydroxy or 1 carboxy group(s),
R^{3'} stands for a 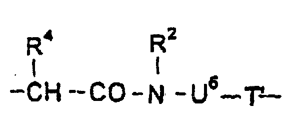 group
R⁴ stands for a straight-chain, branched, saturated or unsaturated C₁-C₃₀ alkyl chain which optionally is interrupted by 1-10 oxygen atoms, 1 phenylene, 1 phenyleneoxy groups and/or optionally is substituted by 1-5 hydroxy, 1-3 carboxy, 1 phenyl group(s),
U⁶ stands for a straight-chain, branched, saturated or unsaturated C₁-C₂₀ alkylene group which optionally contains 1-5 imino, 1-3 phenylene, 1-3 phenyleneoxy, 1-3 phenyleneimino, 1-5 amide, 1-2 hydrazide, 1-5 carbonyl, 1-5 ethyleneoxy, 1 urea, 1 thiourea, 1-2 carboxyalkylimino, 1-2 ester groups, 1-10 oxygen, 1-5 sulphur and/or 1-5 nitrogen atom(s) and/or optionally is substituted by 1-5 hydroxy, 1-2 mercapto, 1-5 oxo, 1-5 thioxo, 1-3 carboxy, 1-5 carboxyalkyl, 1-5 ester and/or 1-3 amino group(s), and the phenylene groups that are optionally contained can be substituted by 1-2 carboxy, 1-2 sulphone or 1-2 hydroxy groups,
T' stands for a -C*O, -COOH, -N=C=O or -N=C=S group and
C*O stands for an activated carboxyl group.

14. Process for the production of the pharmaceutical compositions according to Claim 9, **characterized in that** the cascade polymer complex, dissolved or suspended in water or physiological salt solution, optionally with the additions usual in pharmaceutical technology, is brought into a form suitable for enteral or parenteral administration.

## Revendications

1. Complexes polymère en cascade comprenant
a) des ligands complexants de formule générale I
A-{X-[Y-(Z-<W-K_{w}>_{z})_{y}]ₓ}ₐ (I)
dans laquelle
A représente un noyau en cascade azoté de multiplicité de base a,
X et Y représentent, indépendamment l'un de l'autre, une liaison directe ou un motif de reproduction en cascade de multiplicité de reproduction x ou y repectivement,
Z et W représentent, indépendamment l'un de l'autre, un motif de reproduction en cascade de multiplicité de reproduction z ou w repectivement,
K représente le radical d'un agent complexant,
a représente les chiffres 2 à 12,
x, y, z et w représentent, indépendamment les uns des autres, les chiffres 1 à 4,
à condition qu'au moins deux motifs de reproduction soient différents et que le produit des multiplicités satisfasse à
16 ≤ a · x · y · z · w ≤ 64,
b) au moins 16 ions d'un élément des numéros atomiques 20 à 29, 39, 42, 44 ou 57-83,
c) éventuellement des cations de bases inorganiques et/ou organiques, d'aminoacides ou d'aminoamides ainsi que
d) éventuellement des groupes amino terminaux acylés,
**caractérisés par** les caractéristiques suivantes:
le radical d'agent complexant K lié aux atomes d'azote terminaux de la dernière génération du motif de reproduction W représente un radical de formule générale IA ou IB 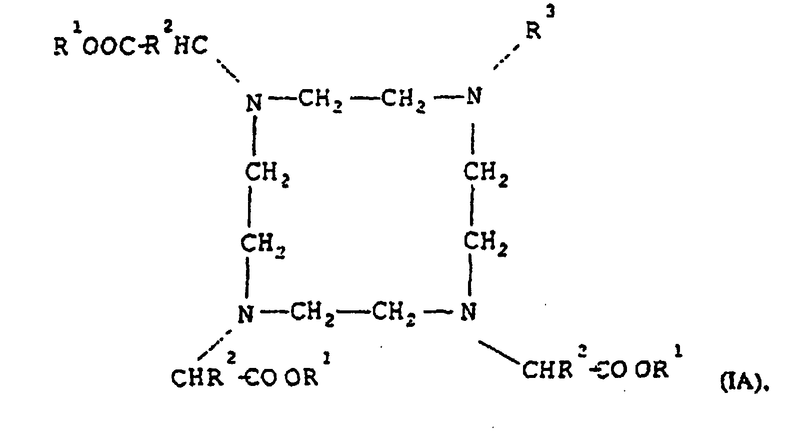 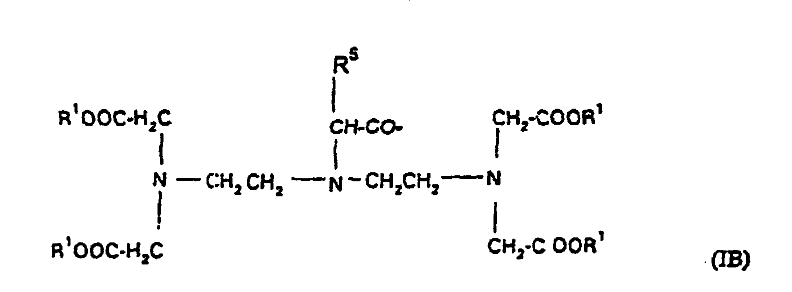 dans lesquelles
R¹ représente indépendamment un atome d'hydrogène ou un équivalent d'ion métallique des numéros atomiques 20-29, 39, 42-44 ou 57-83,
R² représente un atome d'hydrogène, un radical méthyle ou éthyle qui est éventuellement substitué par 1-2 groupe(s) hydroxy ou 1 groupe carboxy,
R³ représente un groupe 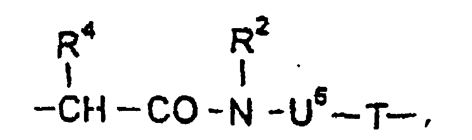
R⁴ représente une chaîne alkyle en C₁-C₃₀ linéaire, ramifiée, saturée ou insaturée qui est éventuellement interrompue par 1-10 atomes d'oxygène, 1 groupe phénylène, 1 groupe phénylène-oxy et/ou éventuellement substituée par 1-5 groupe(s) hydroxy, 1-3 groupe(s) carboxy, 1 groupe phényle,
R⁵ représente un atome d'hydrogène ou R⁴,
U⁶ représente un groupe alkylène en C₁-C₂₀ linéaire, ramifié, saturé ou insaturé, renfermant éventuellement 1-5 groupe(s) imino, 1-3 groupe(s) phénylène, 1-3 groupe(s) phénylène-oxy, 1-3 groupe(s) phénylène-imino, 1-5 groupe(s) amide, 1-2 groupe(s) hydrazide, 1-5 groupe(s) carbonyle, 1-5 groupe(s) éthylène-oxy, 1 groupe urée, 1 groupe thio-urée, 1-2 groupe(s) carboxyalkylimino, 1-2 groupe(s) ester, 1-10 atome(s) d'oxygène, 1-5 atome(s) de soufre et/ou 1-5 atome(s) d'azote et/ou éventuellement substitué par 1-5 groupe(s) hydroxy, 1-2 groupe(s) mercapto, 1-5 groupe(s) oxo, 1-5 groupe(s) thioxo, 1-3 groupe(s) carboxy, 1-5 groupe(s) carboxyalkyle, 1-5 groupe(s) ester et/ou 1-3 groupe(s) amino, les groupes phénylènes éventuellement présents pouvant être substitués par 1-2 groupe(s) carboxy, 1-2 groupe(s) sulfone ou 1-2 groupe(s) hydroxy,
T représente un groupe -CO-α, -NHCO-α ou -NHCS-α et
α représente la position de liaison à l'atome d'azote terminal de la dernière génération du motif de reproduction W.

2. Complexes polymère en cascade selon la revendication 1, **caractérisés en ce que** A représente un atome d'azote, 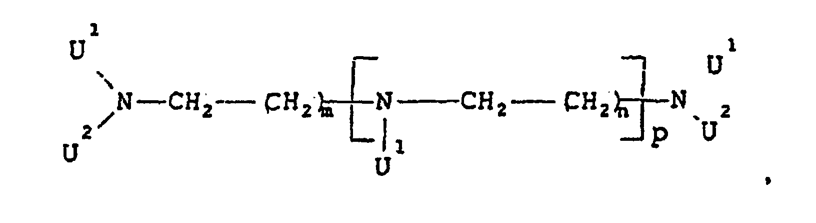 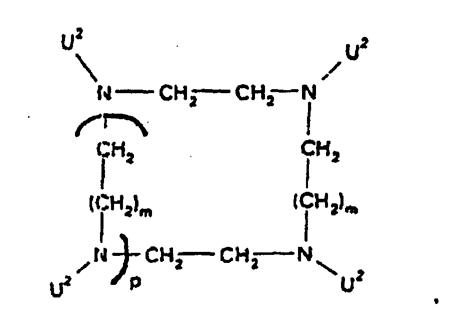 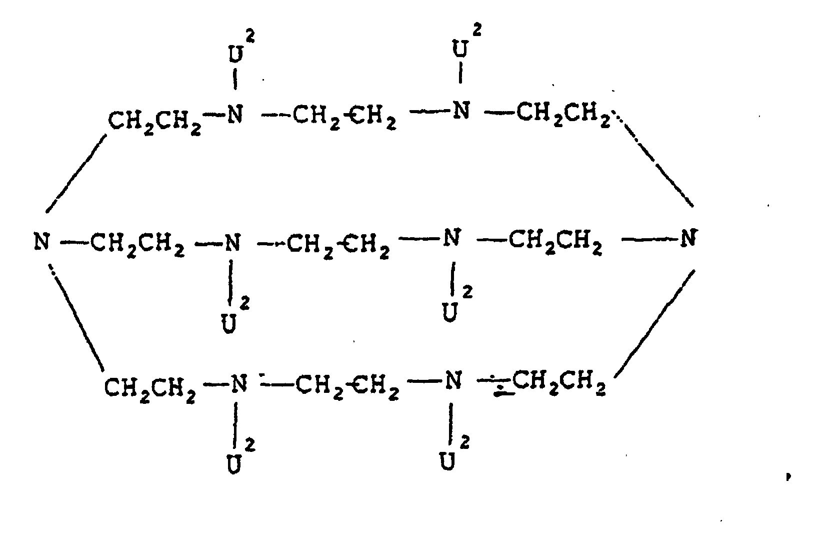 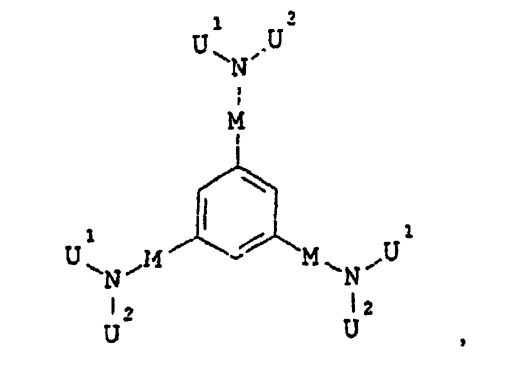 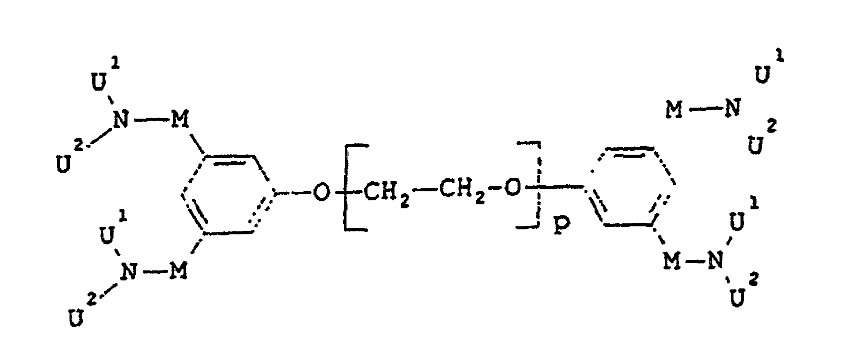 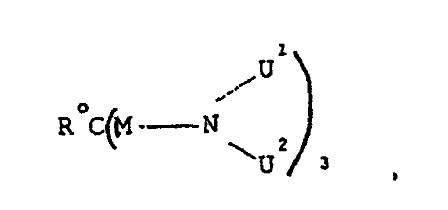 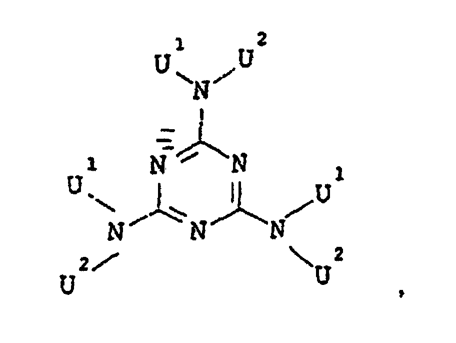 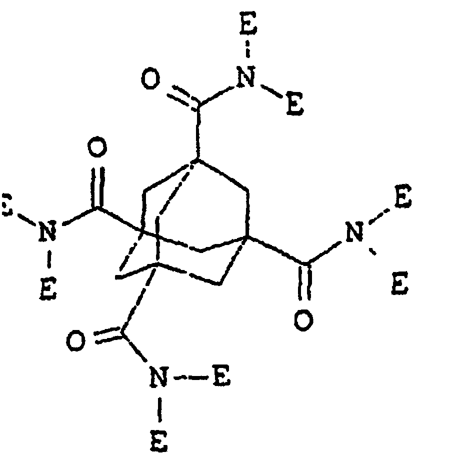 où
m et n représentent les chiffres 1 à 10,
p représente les chiffres 0 à 10,
U¹ représente Q¹ ou E,
U² représente Q² ou E où
E représente le groupe 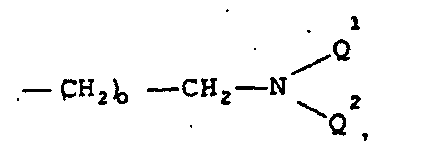 où
o représente les chiffres 1 à 6,
Q¹ représente un atome d'hydrogène ou Q² et
Q² représente une liaison directe,
M représente une chaîne alkylène en C₁-C₁₀ qui est éventuellement interrompue par 1 à 3 atomes d'oxygène et/ou est éventuellement substituée par 1 à 2 groupes oxo,
R⁰ représente un radical alkyle en C₁-C₁₀ ramifié ou non ramifié, un groupe nitro, amino, acide carboxylique ou 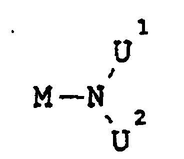 où
le nombre de Q² correspond à la multiplicité de base a.

3. Complexes polymère en cascade selon la revendication 1, **caractérisés en ce que** les motifs de reproduction en cascade X, Y, Z et W représentent, indépendamment les uns des autres,
E, 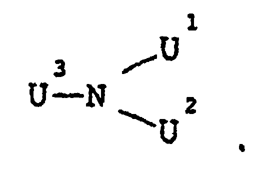 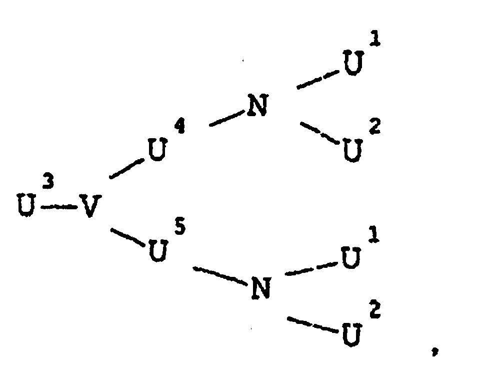 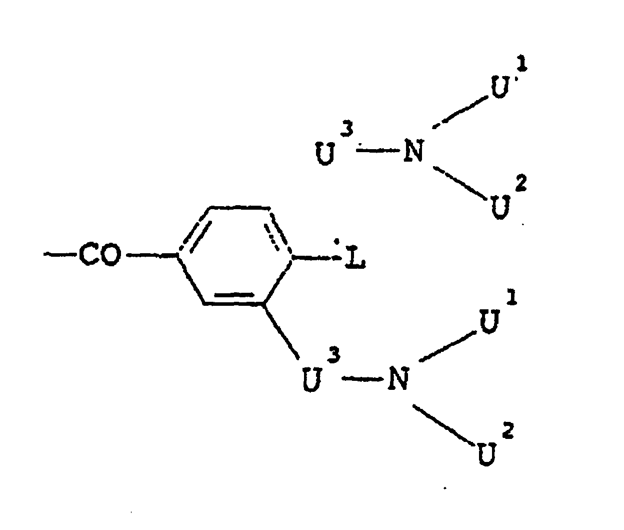 où
U¹ représente Q¹ ou E,
U² représente Q² ou E où
E représente le groupe 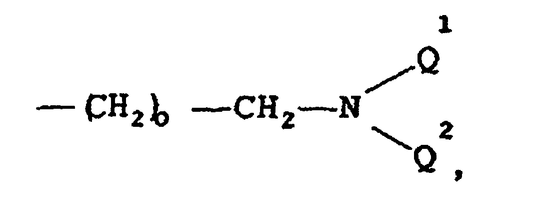 où
o représente les chiffres 1 à 6,
Q¹ représente un atome d'hydrogène ou Q²,
Q² représente une liaison directe,
U³ représente une chaîne alkylène en C₁-C₂₀ qui est éventuellement interrompue par 1 à 10 atomes d'oxygène et/ou 1 à 2 radicaux -N(CO)_{q}-R², 1 à 2 radicaux phénylène et/ou 1 à 2 radicaux phénylène-oxy et/ou est éventuellement substituée par 1 à 2 groupes oxo, thioxo, carboxy, C₁-C₅-alkylcarboxy, alcoxy en C₁-C₅, hydroxy, alkyle en C₁-C₅, où
q représente les chiffres 0 ou 1 et
R² 'représente un atome d'hydrogène, un radical méthyle ou éthyle qui est éventuellement substitué par 1-2 groupe(s) hydroxy ou 1 groupe carboxy,
L représente un atome d'hydrogène ou le groupe 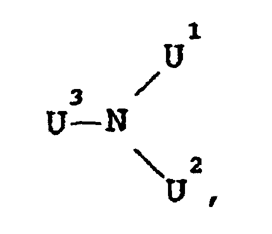
V représente le groupe méthine 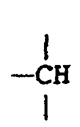 si U⁴ représente simultanément une liaison directe ou le groupe M et U⁵ a l'une des significations de U³, ou
V représente le groupe 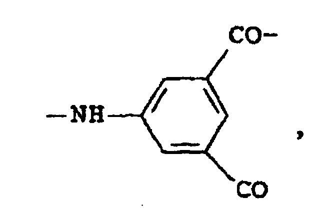 si U⁴ et U⁵ sont simultanément identiques et représentent la liaison directe ou le groupe M,
où M représente une chaîne alkylène en C₁-C₁₀ qui est éventuellement interrompue par 1 à 3 atomes d'oxygène et/ou est éventuellement substituée par 1 à 2 groupes oxo.

4. Complexes polymère en cascade selon la revendication 1, **caractérisés en ce que** la chaîne alkylène en C₁-C₂₀ représentant U⁶ renferme les groupes
-CH₂-, -CH₂-NHCO-, -NHCOCH₂O-, -NHCOCH₂OC₆H₄-, -N(CH₂CO₂H)-, -NHCOCH₂C₆H₄-, -NHCSNHC₆H₄-, -CH₂OC₆H₄-, -CH₂CH₂O-, et/ou est substituée par les groupes -COOH, -CH₂COOH.

5. Complexes polymère en cascade selon la revendication 1, **caractérisés en ce que** U⁶ représente un groupe
-CH₂-, -CH₂CH₂-, -CH₂CH₂CH₂-, -C₆H₄-, -C₆H₁₀-, -CH₂C₆H₅-, -CH₂NHCOCH₂CH(CH₂CO₂H)-C₆H₄-, -CH₂NHCOCH₂OCH₂-, -CH₂NHCOCH₂C₆H₄-.

6. Complexes polymère en cascade selon la revendication 3, **caractérisés en ce que** le radical U³ présent dans les motifs de reproduction en cascade X, Y, Z et W représente
-CO-, -COCH₂OCH₂CO-, -COCH₂-, -CH₂CH₂-, -CONHC₆H₄-, -COCH₂CH₂CO-, -COCH₂-CH₂CH₂CO-, -COCH₂CH₂CH₂CH₂CO-, le radical U⁴ représente une liaison directe, -CH₂CO-,
le radical U⁵ représente une liaison directe,
-(CH₂)₄-, -CH₂CO-, -CH(COOH)-, CH₂OCH₂CH₂-, -CH₂C₆H₄-, CH₂-C₆H₄OCH₂CH₂-,
le radical E représente un groupe

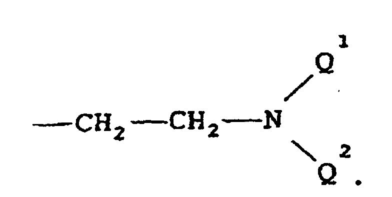

7. Complexes polymère en cascade selon la revendication 3, **caractérisés en ce que** les motifs de reproduction en cascade X, Y, Z et W représentent, indépendamment les uns des autres,
-CH₂CH₂NH-; -CH₂CH₂N<; -COCH(NH-) (CH₂)₄NH-; -COCH(N<) (CH₂)₄N<; -COCH₂OCH₂CON (CH₂CH₂NH-)₂; -COCH₂OCH₂CON (CH₂CH₂N<)₂; -COCH₂N(CH₂CH₂NH-)₂; -COCH₂N (CH₂CH₂N<)₂; -COCH₂NH-; -COCH₂N<; -COCH₂CH₂CON(CH₂CH₂NH-)₂; -COCH₂CH₂CON (CH₂CH₂N<)₂; -COCH₂OCH₂CONH-C₆H₄-CH[CH₂CON(CH₂CH₂NH-)₂]₂; -COCH₂OCH₂CONH-C₆H₄-CH[CH₂CON(CH₂CH₂N<)₂]₂; -COCH₂CH₂CO-NH-C₆H₄-CH[CH₂CON(CH₂CH₂NH-)₂]₂; -COCH₂CH₂CO-NH-C₆H₄-CH[CH₂CON(CH₂CH₂N<)₂]₂; -COCH₂C₆H₄-CH[CH₂CON(CH₂CH₂NH-)₂]₂; -COCH(NH-)CH(COOH)NH-; -COCH(N<)CH(COOH)N<;

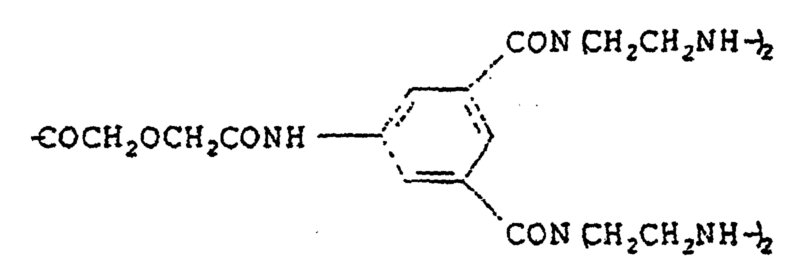

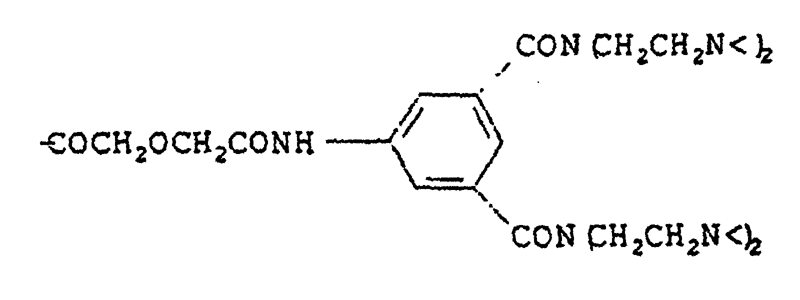

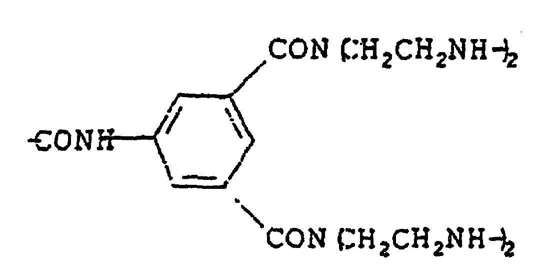

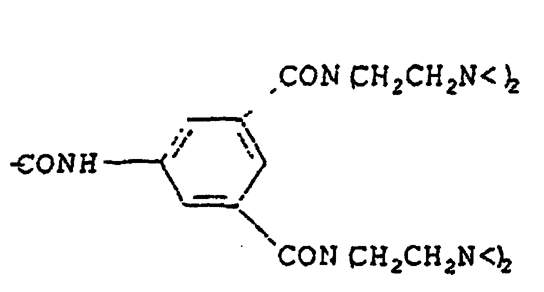

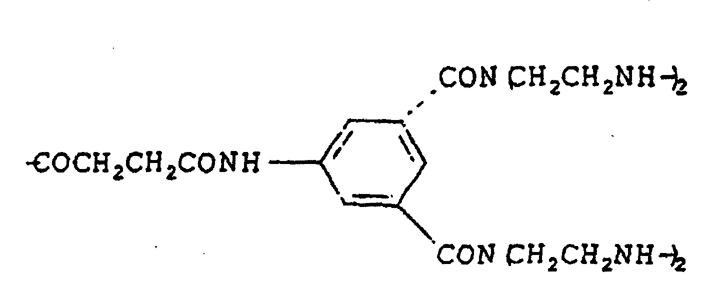

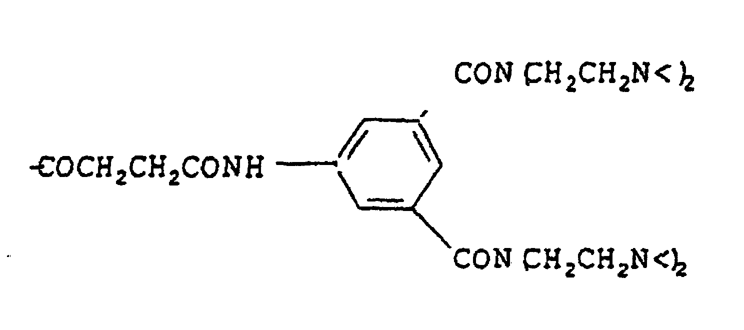

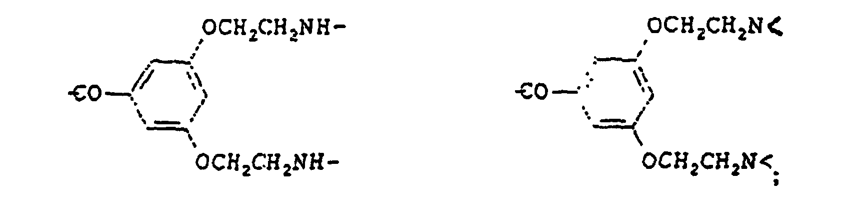

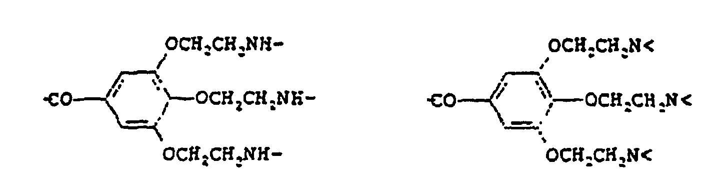

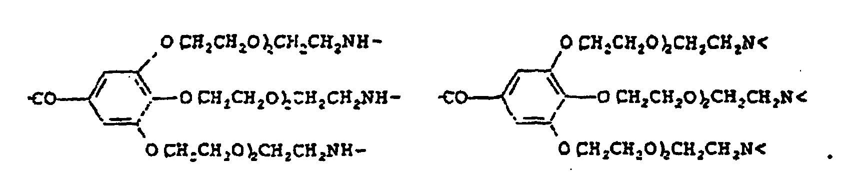

8. Complexes polymère en cascade selon la revendication 2, **caractérisés en ce que**
m représente les chiffres 1-3,
n représente les chiffres 1-3,
o représente le chiffre 1,
P représente les chiffres 0-3,
M représente un groupe -CH₂, -CO ou -CH₂CO et
R^{o} représente un groupe -CH₂NU¹U², CH₃ ou NO₂.

9. Compositions pharmaceutiques comprenant au moins un complexe polymère en cascade selon la revendication 1, éventuellement avec les additifs habituels en galénique.

10. Utilisation d'au moins un complexe polymère selon la revendication 1 pour la préparation d'agents destinés au diagnostic par RMN ou par rayons X (méthodes traditionnelles et tomographie informatique).

11. Utilisation des complexes polymère en cascade selon la revendication 1 pour la préparation d'agents destinés à la différenciation de tumeurs bénignes et malignes dans des régions corporelles sans barrière hémato-encéphalique.

12. Procédé de préparation de complexes polymère en cascade selon les revendications 1 à 8, **caractérisé en ce que** l'on fait réagir des composés de formule générale I'
A-{X-[Y-(Z-<W-β_{w}>_{z})_{y}]ₓ}ₐ (I'),
dans laquelle
A représente un noyau en cascade azoté de multiplicité de base a,
X et Y représentent, indépendamment l'un de l'autre, une liaison directe ou un motif de reproduction en cascade de multiplicité de reproduction x ou y repectivement,
Z et W représentent, indépendamment l'un de l'autre, un motif de reproduction en cascade de multiplicité de reproduction z ou w respectivement,
a représente les chiffres 2 à 12,
x, y, z et w représentent, indépendamment les uns des autres, les chiffres 1 à 4, et
β représente la position de liaison des groupes NH terminaux de la dernière génération du motif de reproduction W,
à condition qu'au moins deux motifs de reproduction soient différents et que le produit des multiplicités satisfasse à
16 ≤ a · x · y · z · w ≤ 64,
avec un complexe ou un agent complexant K' de formule générale I'A ou I'B 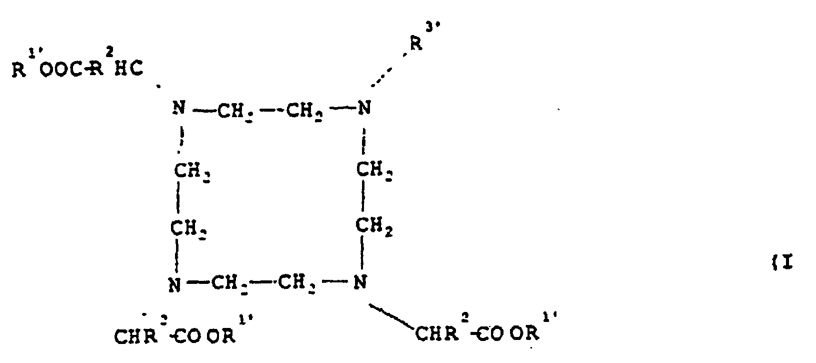 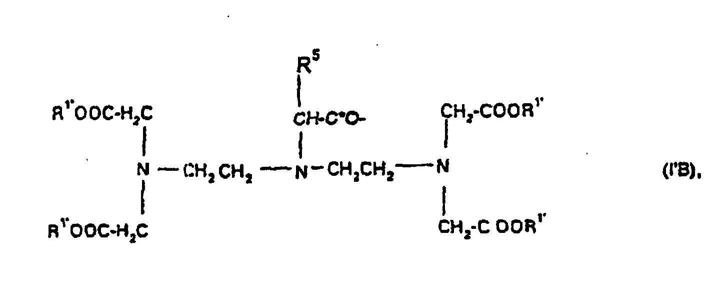 dans lesquelles
R^{1'} représente indépendamment un atome d'hydrogène, un équivalent d'ion métallique des numéros atomiques 20-29, 39, 42-44 ou 57-83 ou un groupe protecteur contre les acides,
R^{2'} représente un atome d'hydrogène, un radical méthyle ou éthyle qui est éventuellement substitué par 1-2 groupe(s) hydroxy ou 1 groupe carboxy,
R^{3'} représente un groupe 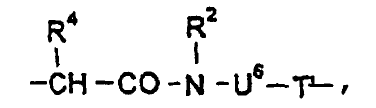
R⁴ représente une chaîne alkyle en C₁-C₃₀ linéaire, ramifiée, saturée ou insaturée qui est éventuellement interrompue par 1-10 atomes d'oxygène, 1 groupe phénylène, 1 groupe phénylène-oxy et/ou éventuellement substituée par 1-5 groupe(s) hydroxy, 1-3 groupe(s) carboxy, 1 groupe phényle,
R⁵ représente un atome d'hydrogène ou R⁴,
U⁶ représente un groupe alkylène en C₁-C₂₀ linéaire, ramifié, saturé ou insaturé, renfermant éventuellement 1-5 groupe(s) imino, 1-3 groupe(s) phénylène, 1-3 groupe(s) phénylène-oxy, 1-3 groupe(s) phénylène-imino, 1-5 groupe(s) amide, 1-2 groupe(s) hydrazide, 1-5 groupe(s) carbonyle, 1-5 groupe(s) éthylène-oxy, 1 groupe urée, 1 groupe thio-urée, 1-2 groupe(s) carboxyalkylimino, 1-2 groupe(s) ester, 1-10 atome(s) d'oxygène, 1-5 atome(s) de soufre et/ou 1-5 atome(s) d'azote et/ou éventuellement substitué par 1-5 groupe(s) hydroxy, 1-2 groupe(s) mercapto, 1-5 groupe(s) oxo, 1-5 groupe(s) thioxo, 1-3 groupe(s) carboxy, 1-5 groupe(s) carboxyalkyle, 1-5 groupe(s) ester et/ou 1-3 groupe(s) amino, les groupes phénylènes éventuellement présents pouvant être substitués par 1-2 groupe(s) carboxy, 1-2 groupe(s) sulfone ou 1-2 groupe(s) hydroxy,
T' représente un groupe -C*O, -COOH, -N=C=O ou -N=C=S et
C*O représente un groupe carboxy activé,
à condition que - dans la mesure où K' représente un complexe - au moins deux (dans le cas de métaux bivalents) ou trois (dans le cas de métaux trivalents) des substituants R¹ représentent un équivalent d'ion métallique des éléments susmentionnés et que, si on le souhaite, d'autres groupes carboxy soient présents sous forme de leurs sels avec des bases inorganiques et/ou organiques, des aminoacides ou des aminoamides,
les groupes protecteurs éventuellement présents sont éliminés, les polymères en cascade ainsi obtenus - dans la mesure où K' représente un agent complexant - sont mis à réagir, de manière connue en soi, avec au moins un oxyde métallique ou sel métallique d'un élément des numéros atomiques 20-29, 39, 42, 44 ou 57-83 et ensuite, éventuellement, les atomes d'hydrogène acides encore présents dans les complexes polymère en cascade ainsi obtenus sont substitués, totalement ou partiellement, par des cations de bases inorganiques et/ou organiques, d'aminoacides ou d'aminoamides, et les groupes amino terminaux libres éventuellement encore présents sont acylés, si on le souhaite, avant ou après la complexation métallique.

13. Composés de formule générale I'A 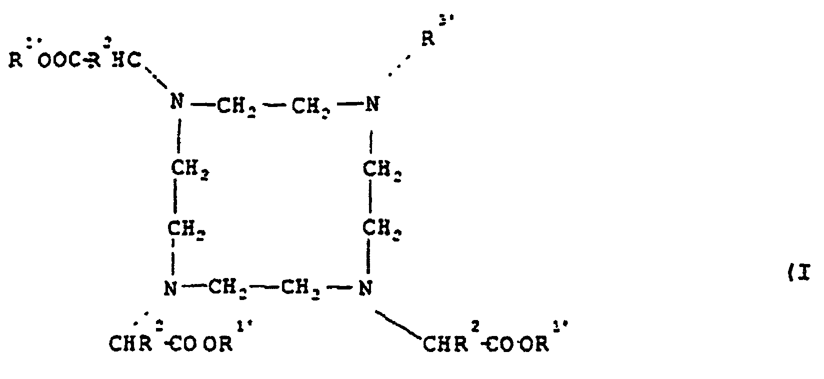 dans laquelle
R^{1'} représente indépendamment un atome d'hydrogène, un équivalent d'ion métallique des numéros atomiques 20-29, 39, 42-44 ou 57-83 ou un groupe protecteur contre les acides,
R² représente un atome d'hydrogène, un radical méthyle ou éthyle qui est éventuellement substitué par 1-2 groupe(s) hydroxy ou 1 groupe carboxy,
R^{3'} représente un groupe 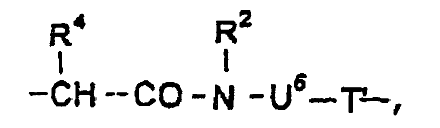
R⁴ représente une chaîne alkyle en C₁-C₃₀ linéaire, ramifiée, saturée ou insaturée qui est éventuellement interrompue par 1-10 atomes d'oxygène, 1 groupe phénylène, 1 groupe phénylène-oxy et/ou éventuellement substituée par 1-5 groupe(s) hydroxy, 1-3 groupe(s) carboxy, 1 groupe phényle,
U⁶ représente un groupe alkylène en C₁-C₂₀ linéaire, ramifié, saturé ou insaturé, renfermant éventuellement 1-5 groupe(s) imino, 1-3 groupe(s) phénylène, 1-3 groupe(s) phénylène-oxy, 1-3 groupe(s) phénylène-imino, 1-5 groupe(s) amide, 1-2 groupe(s) hydrazide, 1-5 groupe(s) carbonyle, 1-5 groupe(s) éthylène-oxy, 1 groupe urée, 1 groupe thio-urée, 1-2 groupe(s) carboxyalkylimino, 1-2 groupe(s) ester, 1-10 atome(s) d'oxygène, 1-5 atome(s) de soufre et/ou 1-5 atome(s) d'azote et/ou éventuellement substitué par 1-5 groupe(s) hydroxy, 1-2 groupe(s) mercapto, 1-5 groupe(s) oxo, 1-5 groupe(s) thioxo, 1-3 groupe(s) carboxy, 1-5 groupe(s) carboxyalkyle, 1-5 groupe(s) ester et/ou 1-3 groupe(s) amino, les groupes phénylènes éventuellement présents pouvant être substitués par 1-2 groupe(s) carboxy, 1-2 groupe(s) sulfone ou 1-2 groupe(s) hydroxy,
T' représente un groupe -C-*O, -COOH, -N=C=O ou -N=C=S et
C*O représente un groupe carboxy activé.

14. Procédé de préparation des compositions pharmaceutiques selon la revendication 9, **caractérisé en ce que** le complexe polymère en cascade dissout ou mis en suspension dans de l'eau ou dans une solution de sel physiologique, éventuellement avec les additifs habituels en galénique, est mis sous une forme appropriée pour l'application par voie entérale ou parentérale.
